# EUROPEAN PATENT SPECIFICATION

(11) **EP 2 864 310 B1**
(45) Date of publication and mention of the grant of the patent: **26.10.2022**
(21) Application number: 13733147.6
(22) Date of filing: 12.06.2013
(51) Int. Cl.: C07D 311/72, C08J 3/00, C08K 5/00

(54) **STABILIZER COMPOSITIONS CONTAINING SUBSTITUTED CHROMAN COMPOUNDS AND METHODS OF USE**
STABILISATORZUSAMMENSETZUNGEN MIT SUBSTITUIERTEN CHROMANVERBINDUNGEN UND ANWENDUNGSVERFAHREN
COMPOSITIONS STABILISATRICES CONTENANT DES COMPOSÉS CHROMANNE SUBSTITUÉS ET MÉTHODES D'UTILISATION ASSOCIÉES

(30) Priority: 13.06.2012 US 201213495109
(43) Date of publication of application: 29.04.2015
(73) Proprietor: Cytec Technology Corp., Wilmington, DE 19801 (US)
(72) Inventor: GUPTA, Ram, Stamford, CT 06905 (US); SAMUELS, Sari-Beth, Woodland Park, NJ 07424 (US); ENG., J., Mon Hei, Wilton, CT 06897 (US); STEELE, Thomas, Milford, CT 06460 (US)
(74) Representative: Carpmaels & Ransford LLP
(86) International application number: PCT/US2013/045318
(87) International publication number: WO 2013/188490

(56) References cited:
- EP-A1- 0 860 467
- WO-A1-00/00540
- WO-A1-97/49758
- WO-A1-99/54394
- WO-A2-2004/024810
- US-A1- 2012 146 257
- US-B1- 6 444 733

## Description

### BACKGROUND OF THE INVENTION

### 1. Field of the Invention

The invention described herein relates to stabilizer compositions for nonliving organic materials. More particularly, the invention relates to stabilizer compositions having certain substituted chroman compounds and, if desired, other additives for stabilizing nonliving organic materials against the action of light, oxygen, and/or heat, thereby improving the color, performance, and/or processing of such materials, including plastics.

### 2. Description of the Related Art

The mechanical, chemical, and/or aesthetic properties of nonliving organic material, e.g., plastics and coating materials, are known to be impaired by the effects of light, oxygen, and heat. For such polymeric materials, this impairment is typically manifested as yellowing or other discoloration, cracking or embrittlement of the material, and/or loss of gloss. Additionally, such polymeric organic materials are subjected to high temperatures and pressures during processing into articles, which can adversely impact the physical properties and/or characteristics, as well as the appearance, of the finished articles made from such materials.

Accordingly, such materials require the addition of various additive systems thereto in order both to be processed and to retain long term stability in order to retain desired service properties. A wide variety of substances are known in the art for use as such additives and stabilizers. In many instances, a mixture of such additives is employed. Known stabilizers include, for example, sterically hindered amines such as Hindered Amine Light Stabilizers (HALS), ultraviolet (UV) light absorbers, and antioxidants.

While some stabilization of organic materials is achieved by using one or more of these classes of compounds with a variety of other additives, the high concentrations required for effective protection often leads to undesirable changes in in the properties of the material being stabilized, as well as to compatibility problems, which include exudation, chalking, formation of coatings, or color changes. Stabilizers such as antioxidants traditionally include sterically hindered phenolics, aromatic amines, organo-phosphites/phosphonites, and/or thioethers. However, appropriate combinations of stabilizers have to be carefully selected based on the desired final properties the end article should have.

For example, the prior art is replete with examples of stabilizer compositions for organic polymers containing 6-hydroxychroman compounds including α-tocopherols, alone or in combination with other additives such as phenolics and/or organophosphites/phosphonites. Aside from its inherent safety (it is edible and generally recognized as safe ("GRAS")) and its suitable physical properties, α-tocopherol is an effective scavenger of oxy radicals. It is also very reactive toward less electrophilic radicals such as alkyl, toward hydroperoxides, excited states of ketones, ozone, peroxide, nitrogen oxides, and other reactive species associated with oxidative damage. However, despite its recognition as an excellent processing stabilizer and color stabilizer, it is inherently viscous and a dark amber oil that is known to lead to discoloring of organic polymers. As noted by Laermer and Zambetti in Alpha-Tocopherol (Vitamin E) - the Natural Antioxidant for Polyolefins, Journal of Plastic Film and Sheeting 1992 8:228-248, 247 a-tocopherol is a suitable stabilizing agent if polymer color is not critical.

Other prior art references detail similar issues with using α-tocopherols in organic polymer materials. U.S. Patent No. 4,806,580 details the unsuitability of using α-tocopherols for stabilizing colorless plastics (alone or in combination with other additives) as they give rise to discoloration. US2012/146257 details stabilizer mixtures of chroman derivatives for rotomolding processes.

U.S. Patent No. 5,807,504 details stabilizer mixtures of chroman derivatives and organic phosphites or phosphonites, but that they are disadvantageous for being unstable upon storage and after incorporation into the material being stabilized.

U.S. Patent No. 6,465,548 details that 6-hydroxychroman compounds have not been widely used as antioxidants for purposes of stabilizing organic polymer materials because its marked coloring of these materials has not been overcome.

Accordingly, stabilizer compositions for use in minimizing the effects of light, oxygen, and/or heat in nonliving organic materials require further improvement. Thus, stabilizer compositions and processes that specifically enhance the color, performance, and/or processing of such materials, while simultaneously permitting higher processing temperatures and/or shorter cooling times, and faster injection speeds for high speed molding or that effectively reduce the molding cycle times would be a useful advance in the field and would find rapid acceptance in the chemical additives and various industrial molding industries.

### SUMMARY OF THE INVENTION

The present invention provides a use of a stabilizer composition comprising a stabilizing amount of vitamin E acetate in an organic polymer material for improving the rheological property of said organic polymer material, wherein the improved rheological property is measured by less variation in the values of melt index of said organic polymer material upon repeat measurements thereof in multiple-pass extrusion testing, as defined in the claims.

As described in detail hereinbelow, the inventors have discovered stabilizer compositions that enhance the characteristics and performance of nonliving organic materials against the deleterious effects of light, oxygen, and/or heat, as well as the use of these compositions for reducing cycle time in various molding processes related to polyolefin articles. These stabilizer compositions surprisingly have multiple effects that lead to improved articles and/or processes, which include: reduction of time required to reach optimal physical and/or mechanical properties; improved viscosity leading to improved flow of resin and quicker mold fill time; and improved color stabilization of material, including lower or no discoloration due to yellowing.

Accordingly, in one aspect the invention provides a use of stabilizer compositions having a stabilizing amount of vitamin E acetate, as defined in the claims.

In certain embodiments, the stabilizer composition can optionally include additional stabilizers chosen from, but not limited to, organic phosphites/phosphonites; hindered phenols; light stabilizers; and other chroman type compounds such as vitamin E or any of its isomers or mixtures of isomers, as well as certain modifiers such as, but not limited to, co-additives, nucleating agents, fillers, reinforcing agents, and polymer additives.

In another aspect, the invention provides a use of a stabilizer composition, as defined in the claims, in processes for stabilizing an organic material subject to degradation and/or discoloration due to effects from light, oxygen, and/or heat, by adding a stabilizing amount of a stabilizer composition having a chroman-based compound, which is vitamin E acetate, as described above and hereinbelow.

In certain embodiments, the organic material can be a polyolefin type polymer or co-polymer, an organic dye, wax, or ink.

Yet a further aspect of the invention provides a use of a stabilizer composition, as defined in the claims, in methods for enhancing the processing stability of an organic material by admixing therein before or during processing a stabilizing amount of the stabilizer composition having a chroman-based compound, which is vitamin E acetate, as described above and hereinbelow.

In another aspect, the invention provides a use of a stabilizer composition, as defined in the claims, in methods for reducing or preventing discoloration of an organic material by admixing therein before or during processing an effective amount of the stabilizer composition having a chroman-based compound, which is vitamin E acetate, as described above and hereinbelow.

In yet a further aspect, the invention provides a use of a stabilizer composition, as defined in the claims, in masterbatch compositions having a stabilizer composition having a chroman-based compound, which is vitamin E acetate, as described above and hereinbelow, and an organic material that is identical to or compatible with the organic material to be stabilized. The compositions and processes described herein are also useful for producing polymeric articles such as by any of the art recognized industrial molding processes including, for example, rotomolding, injection molding, blow molding, reel-to-reel molding, compression molding, micro molding, metal injection molding, etc., as well as for reducing cycle times and/or maintaining broad process windows in rotational molding processes for producing polymeric hollow articles.

Accordingly, in another aspect the invention provides a use of a stabilizer composition, as defined in the claims, in processes for producing a molded article by adding a polymeric organic material and a polymer-stabilizing amount of a stabilizer composition having a chroman-based compound, which is vitamin E acetate, as described above and hereinbelow to a molding device or industrial molding process, and cycling the stabilized polymeric organic material through the device and/or process.

These and other objects, features and advantages of this invention will become apparent from the following detailed description of the various aspects of the invention taken in conjunction with the accompanying Figures and Examples.

### BRIEF DESCRIPTION OF THE DRAWINGS

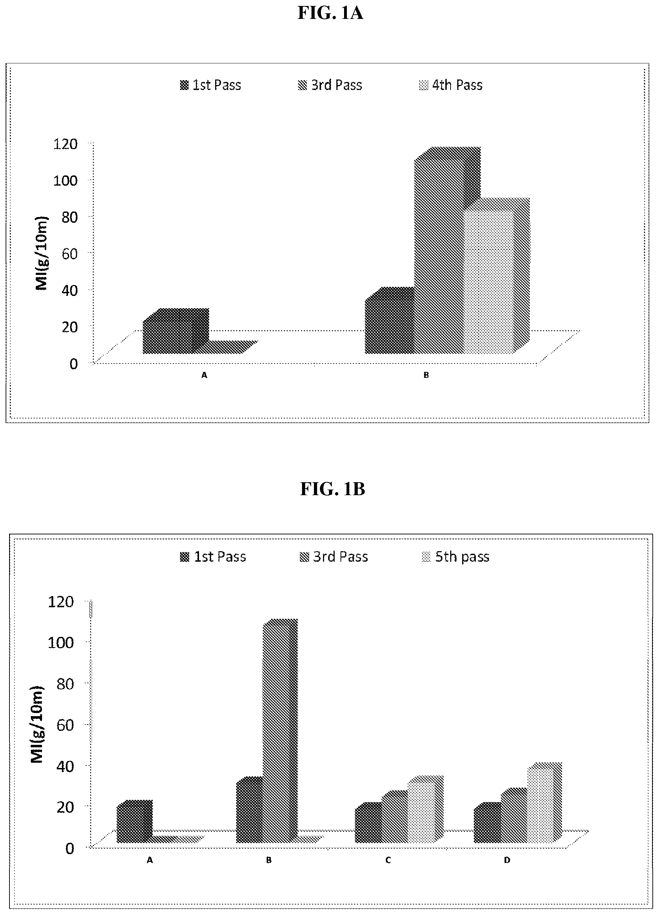

**FIGS. 1A-B** illustrate the Melt-Index results of polypropylene resin formulated with or without various stabilizer compositions, including those according to the present invention, and multi-pass extruded. FIG. 1A - (A): polypropylene resin multi-pass extruded without stabilizer additive; (B): polypropylene resin multi-pass extruded with 0.15 % of a stabilizer composition (vitamin E acetate). FIG. 1B - (A): polypropylene resin multi-pass extruded without stabilizing additive; (B): polypropylene resin multi-pass extruded with 0.15 % of vitamin E acetate; (C): polypropylene resin multi-pass extruded with 0.15 % of vitamin E; (D): polypropylene resin multi-pass extruded with 0.075 % each of vitamin E and vitamin E acetate. Example 1 below provides further information as to the experimental details and results.

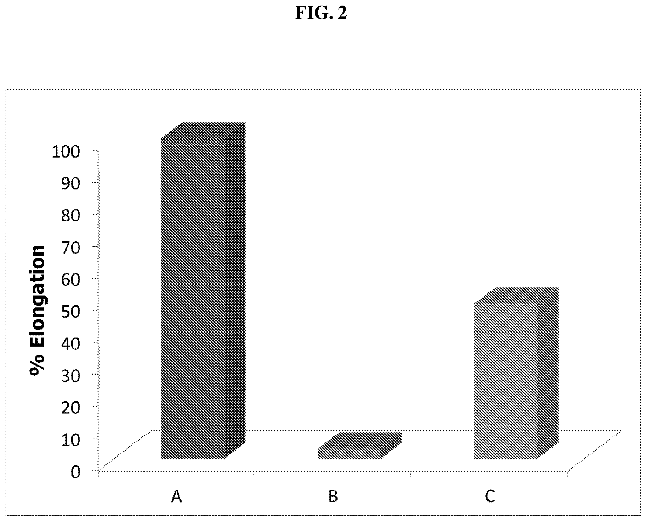

**FIG. 2** illustrates the measurement of mechanical properties (as determined by percentage of retention of strain/elongation at break) of polypropylene resin formulated with (C) or without (B) a stabilizer composition according to the present invention and extruded one time, as compared to unprocessed *(i.e.,* not extruded) polypropylene (A). Further experimental details are provided in Example 2 below.

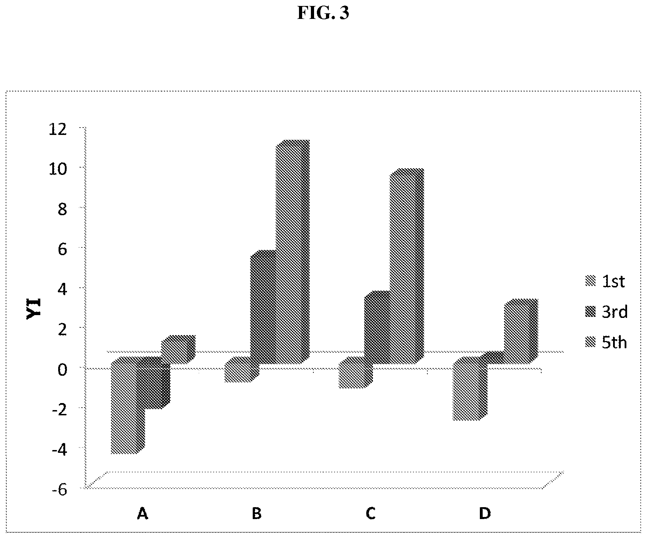

**FIG. 3** illustrates the Yellowness Index of a low density polyethylene resin formulated with or without various stabilizer compositions, including those according to the present invention, and multi-pass extruded. (A): polyethylene resin multi-pass extruded without a stabilizing additive; (B): polyethylene resin multi-pass extruded with IRGANOX^{®} 1010 (phenolic antioxidant); (C): polyethylene resin multi-pass extruded with CYANOX^{®} 1790 (phenolic antioxidant); (D): polyethylene resin multi-pass extruded with vitamin E acetate according to the compositions and methods of the present invention. Further experimental details are provided in Example 3 below.

### DETAILED DESCRIPTION OF CERTAIN EMBODIMENTS OF THE INVENTION

As employed above and throughout the disclosure, the following terms are provided to assist the reader. Unless otherwise defined, all terms of art, notations and other scientific terminology used herein are intended to have the meanings commonly understood by those of skill in the chemical arts. As used herein and in the appended claims, the singular forms include plural referents unless the context clearly dictates otherwise.

Throughout this specification the terms and substituents retain their definitions. A comprehensive list of abbreviations utilized by organic chemists *(i.e.* persons of ordinary skill in the art) appears in the first issue of each volume of the Journal of Organic Chemistry. The list, which is typically presented in a table entitled "Standard List of Abbreviations".

The term "hydrocarbyl" is a generic term encompassing aliphatic, alicyclic and aromatic groups having an all-carbon backbone and consisting of carbon and hydrogen atoms. In certain cases, as defined herein, one or more of the carbon atoms making up the carbon backbone may be replaced or interrupted by a specified atom or group of atoms, such as by one or more heteroatom of N, O, and/or S. Examples of hydrocarbyl groups include alkyl, cycloalkyl, cycloalkenyl, carbocyclic aryl, alkenyl, alkynyl, alkylcycloalkyl, cycloalkylalkyl, cycloalkenylalkyl, and carbocyclic aralkyl, alkaryl, aralkenyl and aralkynyl groups. Such hydrocarbyl groups can also be optionally substituted by one or more substituents as defined herein. Accordingly, the chemical groups or moieties discussed in the specification and claims should be understood to include the substituted or unsubstituted forms. The examples and preferences expressed below also apply to each of the hydrocarbyl substituent groups or hydrocarbyl-containing substituent groups referred to in the various definitions of substituents for compounds of the formulas described herein unless the context indicates otherwise.

Preferred non-aromatic hydrocarbyl groups are saturated groups such as alkyl and cycloalkyl groups. Generally, and by way of example, the hydrocarbyl groups can have up to fifty carbon atoms, unless the context requires otherwise. Hydrocarbyl groups with from 1 to 30 carbon atoms are preferred. Within the sub-set of hydrocarbyl groups having 1 to 30 carbon atoms, particular examples are C₁₋₂₀ hydrocarbyl groups, such as C₁₋₁₂ hydrocarbyl groups (e.g. C₁₋₆ hydrocarbyl groups or C₁₋₄ hydrocarbyl groups), specific examples being any individual value or combination of values selected from C₁ through C₃₀ hydrocarbyl groups.

Alkyl is intended to include linear, branched, or cyclic hydrocarbon structures and combinations thereof. Lower alkyl refers to alkyl groups of from 1 to 6 carbon atoms. Examples of lower alkyl groups include methyl, ethyl, propyl, isopropyl, butyl, s-and t-butyl and the like. Preferred alkyl groups are those of C₃₀ or below.

Alkoxy or alkoxyalkyl refers to groups of from 1 to 20 carbon atoms of a straight, branched, cyclic configuration and combinations thereof attached to the parent structure through an oxygen. Examples include methoxy, ethoxy, propoxy, isopropoxy, cyclopropyloxy, cyclohexyloxy and the like.

Acyl refers to formyl and to groups of 1, 2, 3, 4, 5, 6, 7, 8, 9, 10, 11 and 12 carbon atoms of a straight, branched, cyclic configuration, saturated, unsaturated and aromatic and combinations thereof, attached to the parent structure through a carbonyl functionality. Examples include acetyl, benzoyl, propionyl, isobutyryl, *t*-butoxycarbonyl, benzyloxycarbonyl and the like. Lower-acyl refers to groups containing one to six carbons.

References to "carbocyclic" or "cycloalkyl" groups as used herein shall, unless the context indicates otherwise, include both aromatic and non-aromatic ring systems. Thus, for example, the term includes within its scope aromatic, non-aromatic, unsaturated, partially saturated and fully saturated carbocyclic ring systems. In general, such groups may be monocyclic or bicyclic and may contain, for example, 3 to 12 ring members, more usually 5 to 10 ring members. Examples of monocyclic groups are groups containing 3, 4, 5, 6, 7, and 8 ring members, more usually 3 to 7, and preferably 5 or 6 ring members. Examples of bicyclic groups are those containing 8, 9, 10, 11 and 12 ring members, and more usually 9 or 10 ring members. Examples of non-aromatic carbocycle/cycloalkyl groups include c-propyl, c-butyl, c-pentyl, c-hexyl, and the like. Examples of C₇ to C₁₀ polycyclic hydrocarbons include ring systems such as norbornyl and adamantyl.

Aryl (carbocyclic aryl) refers to a 5- or 6-membered aromatic carbocycle ring containing; a bicyclic 9- or 10-membered aromatic ring system; or a tricyclic 13- or 14-membered aromatic ring system. The aromatic 6- to 14-membered carbocyclic rings include, e.g., substituted or unsubstituted phenyl groups, benzene, naphthalene, indane, tetralin, and fluorene.

Substituted hydrocarbyl, alkyl, aryl, cycloalkyl, alkoxy, etc. refer to the specific substituent wherein up to three H atoms in each residue are replaced with alkyl, halogen, haloalkyl, hydroxy, alkoxy, carboxy, carboalkoxy (also referred to as alkoxycarbonyl), carboxamido (also referred to as alkylaminocarbonyl), cyano, carbonyl, nitro, amino, alkylamino, dialkylamino, mercapto, alkylthio, sulfoxide, sulfone, acylamino, amidino, phenyl, benzyl, halobenzyl, heteroaryl, phenoxy, benzyloxy, heteroaryloxy, benzoyl, halobenzoyl, or loweralkylhydroxy.

The term "halogen" means fluorine, chlorine, bromine or iodine.

As used herein, the term "chroman" or "chroman-based compound" refers to those compounds having a functional chroman group as part of the compound. In certain embodiments the chroman-based compound will be substituted. In other embodiments, the chroman-based compound can include chromanones. Coumarin, tocopherols, and tocotrienols are specific examples of chroman-based compounds.

The terms "cycle time" or "molding cycle" as used herein are given their ordinary meaning as commonly understood by those of skill in the industrial molding arts and refer to the time from one point in the molding cycle to the corresponding point in the next repeated sequence *(i.e.,* the time required to produce a part in a molding operation as measured from a point of one operation to the same point of the first repeat of the operation).

The terms "optimal mechanical property" or "optimal physical property" as used herein refer to molded parts having the most desirable: impact strength, coalescence or scintering of polymer particles, and general appearance such as color.

The term "organic material" or "material to be stabilized" as used herein refers to nonliving organic material including, for example, cosmetic preparations such as ointments and lotions, drug formulations such as pills and suppositories, photographic recording materials, organic dyes, inks, and fibers, as well as synthetic and natural organic polymers, and biopolymers. The synthetic organic polymers are exemplified by synthetic resin such as thermoplastic resin, thermosetting resin and the like. Various such resins are known to those of ordinary skill in the art and are suitable for use with the present invention. The natural organic polymers are exemplified by natural rubbers, proteins, cellulosic derivatives, mineral oils, animal or vegetable oils, wax, fats and oils and the like.

All numbers expressing quantities of ingredients, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

The use of stabilizer compositions according to the invention, as defined in the claims, and suitable for use in stabilizing organic materials subject to degradation and/or discoloration due to the effects from light, oxygen and heat, and in processes for producing articles from organic materials blended therewith, includes wherein the stabilizer composition includes at least one chroman-based compound, which is vitamin E acetate. Chroman-based compound referred to herein are suitably compounds according to Formula V: 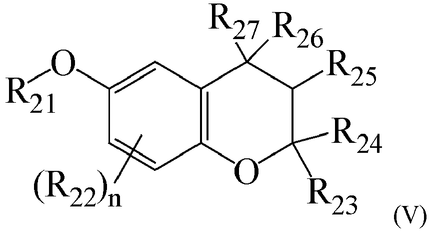 wherein
R₂₁ is chosen from COR₂₈ or Si(R₂₉)₃, wherein R₂₈ is chosen from H or a C₁-C₂₀ hydrocarbyl; and R₂₉ is chosen from a C₁-C₁₂ hydrocarbyl or alkoxy;
R₂₂ is a substituent that can be the same or different at from n = 0 to 3 positions of the aromatic portion of Formula V and is independently chosen from H or a C₁-C₁₂ hydrocarbyl;
R₂₃ is chosen from H or a C₁-C₁₂ hydrocarbyl;
R₂₄ is chosen from H or a C₁-C₂₀ hydrocarbyl; and
each of R₂₅-R₂₇ is independently chosen from a member selected from the group consisting of H; a C₁-C₁₂ hydrocarbyl; and OR₃₀, wherein R₃₀ is chosen from H or a C₁-C₁₂ hydrocarbyl; and
R₂₇ is H, or a bond which together with R₂₆ forms =O.

In certain embodiments, R₂₄ is a C₁-C₁₈ hydrocarbyl.

Vitamin E acetate is a chroman-based compound according to Formula (Va)

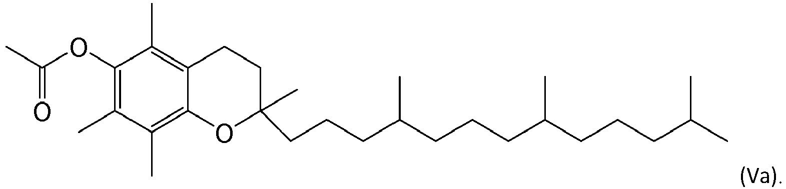

In certain embodiments, the stabilizer composition includes two or more chroman-based compounds according to Formula (V), or a chroman-based compound according to Formula (V) and another chroman compound. In certain embodiments, the other chroman compound is a tocopherol or tocotrienol.

The chroman-based compound, which is vitamin E acetate, can be present from 0.001 to 5.0 % by weight of the total weight of the stabilizer composition, preferably from 0.01 to 2.0 % by weight of the total weight of the stabilizer composition, and more preferably from 0.01 to 1.0 % by weight of the total weight of the stabilizer composition. In certain embodiments, the chroman-based compound, which is vitamin E acetate, is present at 0.05 % by weight of the total weight of the stabilizer composition.

In certain embodiments, the stabilizer composition can further include at least one compound chosen from the group of organic phosphites or phosphonites. In some embodiments the organic phosphite or phosphonite compound includes at least one organic phosphite or phosphonite chosen from a compound according to Formulas 1-7: 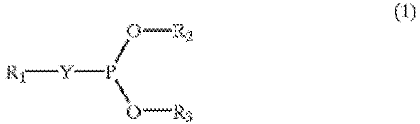 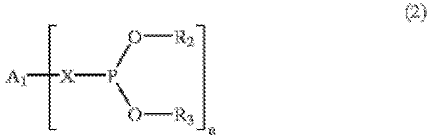 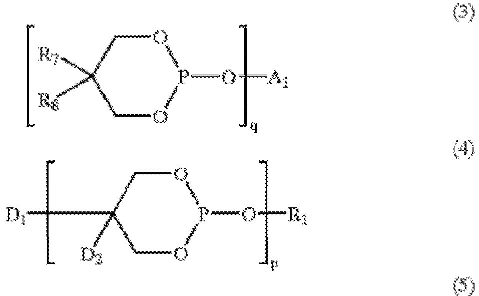 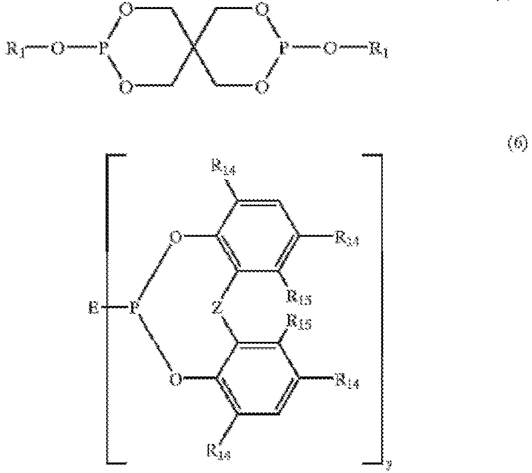 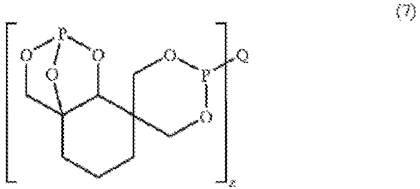 in which the indices are integral and
n is 2, 3 or 4; p is 1 or 2; q is 2 or 3; r is 4 to 12; y is 1, 2 or 3; and z is 1 to 6;
A₁, if n is 2, is C₂-C₁₈ alkylene; C₂-C₁₂ alkylene interrupted by oxygen, sulfur or -NR₄-; a radical of the formula 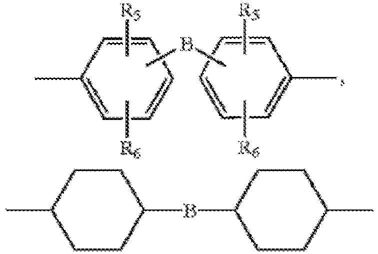
or phenylene;
A₁, if n is 3, is a radical of the formula -CᵣH₂ᵣ₋₁-;
A₁, if n is 4, is 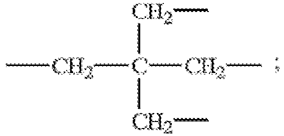
A₂ is as defined for A₁ if n is 2;
B is a direct bond, -CH₂-, -CHR₄-, -CR₁R₄-, sulfur, C₅-C₇ cycloalkylidene, or cyclohexylidene which is substituted by from 1 to 4 C₁-C₄ alkyl radicals in position 3, 4 and/or 5;
D₁, if p is 1, is C₁-C₄ alkyl and, if p is 2, is -CH₂OCH₂-;
D₂, if p is 1, is C₁-C₄ alkyl;
E, if y is 1, is C₁-C₁₈ alkyl, -OR₁ or halogen;
E, if y is 2, is -O-A₂-O-,
E, if y is 3, is a radical of the formula R₄C(CH₂O-)₃ or N(CH₂CH₂O-)₃;
Q is the radical of an at least z-valent alcohol or phenol, this radical being attached via the oxygen atom to the phosphorus atom;
R₁, R₂ and R₃ independently of one another are C₁-C₁₈ alkyl which is unsubstituted or substituted by halogen, -COOR₄, -CN or -CONR₄R₄; C₂-C₁₈ alkyl interrupted by oxygen, sulfur or -NR₄-; C₇-C₉ phenylalkyl; C₅-C₁₂ cycloalkyl, phenyl or naphthyl; naphthyl or phenyl substituted by halogen, 1 to 3 alkyl radicals or alkoxy radicals having a total of 1 to 18 carbon atoms or by C₇-C₉ phenylalkyl; or a radical of the formula 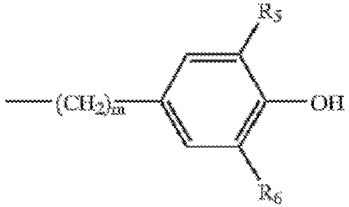
in which m is an integer from the range 3 to 6;
R₄ is hydrogen, C₁-C₈ alkyl, C₅-C₁₂ cycloalkyl or C₇-C₉ phenylalkyl,
R₅ and R₆ independently of one another are hydrogen, C₁-C₈ alkyl or C₅-C₆ cycloalkyl,
R₇ and R₈, if q is 2, independently of one another are C₁-C₄ alkyl or together are a 2,3-dehydropentamethylene radical; and
R₇ and R₈, if q is 3, are methyl;
R₁₄ is hydrogen, C₁-C₉ alkyl or cyclohexyl,
R₁₅ is hydrogen or methyl and, if two or more radicals R₁₄ and R₁₅ are present, these radicals are identical or different,
X and Y are each a direct bond or oxygen,
Z is a direct bond, methylene, -C(R₁₆)₂- or sulfur, and
R₁₆ is C₁-C₈ alkyl;
a trisarylphosphite according to Formula 8: 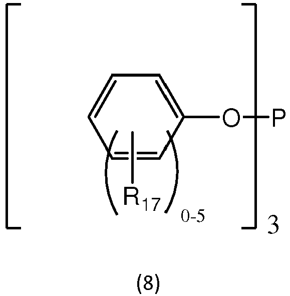 wherein R₁₇ is a substituent that is the same or different at from 0 to 5 positions of the aromatic portion of Formula 8 and is independently chosen from C₁-C₂₀ alkyl, C₃-C₂₀ cycloalkyl, C₄-C₂₀ alkyl cycloalkyl, C₆-C₁₀ aryl, and C₇-C₂₀ alkylaryl; and combinations thereof.

In some embodiments, the following organic phosphites or phosphonites are preferred: triphenyl phosphite; diphenyl alkyl phosphites; phenyl dialkyl phosphites; trilauryl phosphite; trioctadecyl phosphite; distearyl pentaerythritol phosphite; tris(2,4-di-tert-butylphenyl) phosphite; tris(nonylphenyl) phosphite; a compound of formulae (A), (B), (C), (D), (E), (F), (G), (H), (J), (K) and (L): 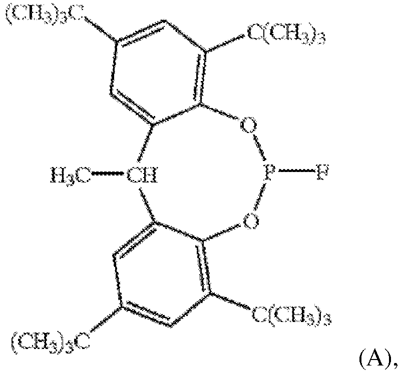 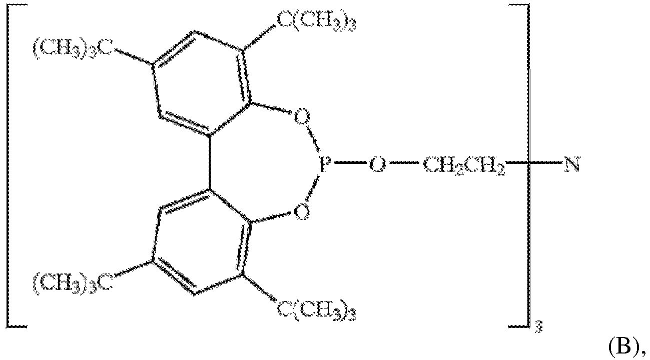 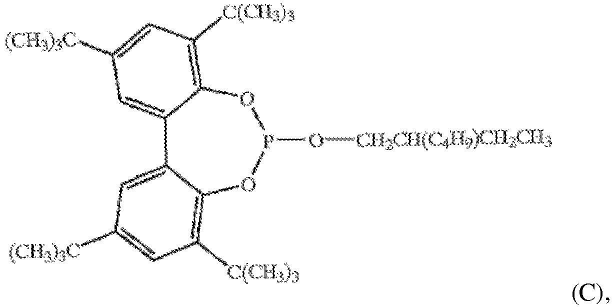 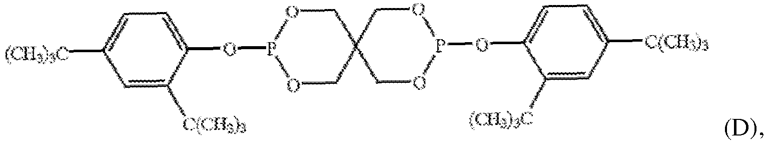 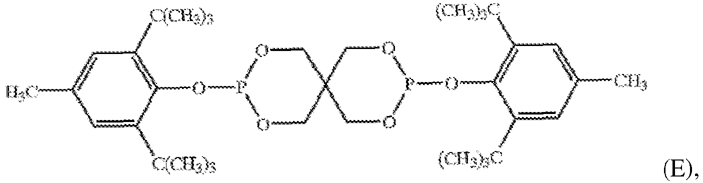 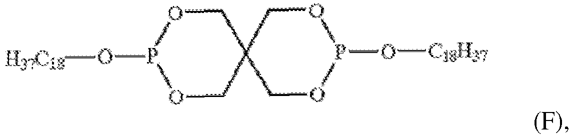 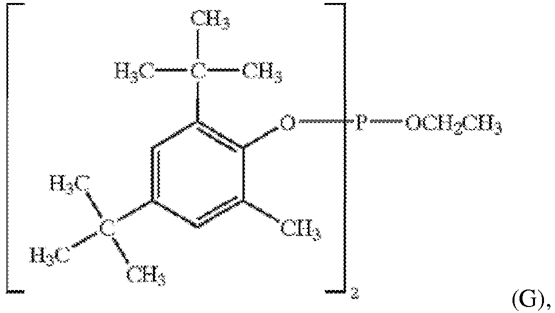 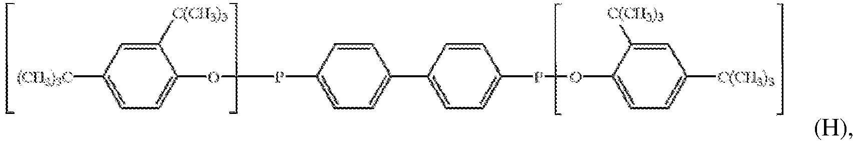 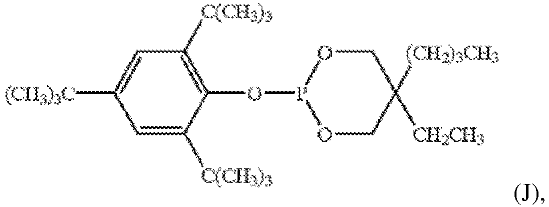 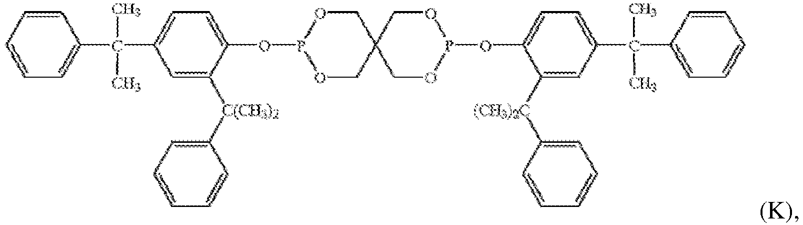 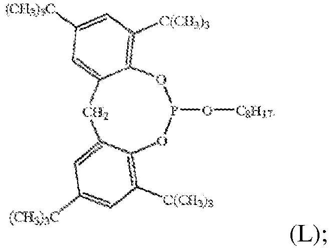 2-butyl-2-ethyl-1,3-propanediol 2,4,6-tri-t-butylphenol phosphite; bis-(2,6-di-t-butyl-4-methlphenyl) pentaerythritol diphosphite; 2-butyl-2-ethyl-1,3-propanediol 2,4-di-cumylphenol phosphite; 2-butyl-2-ethyl-1,3-propanediol 4-methyl-2,6-di-t-butylphenol phosphite; and bis-(2,4,6-tri-t-butyl-phenyl) pentaerythritol diphosphite.

The following organic phosphites and phosphonites are particularly suitable for use in the rotomolding processes described herein: tris(2,4-di-tert-butylphenyl)phosphite (IRGAFOS^{®} 168); Bis(2,4-dicumylphenyl)pentaerythritol diphosphite (DOVERPHOS^{®} S9228); and tetrakis(2,4-di-tert-butylphenyl)4,4'-biphenylene-diphosphonite (IRGAFOS^{®} P-EPQ).

The organic phosphites or phosphonites can be present in an amount from 0.01 % to 10 % by weight based on the total weight of the organic material to be stabilized. Preferably, the amount of organic phosphite or phosphonite is available from 0.05 to 5 %, and more preferably from 0.1 to 3 % by weight based on the total weight of the organic material to be stabilized.

In certain embodiments, the stabilizer composition can further include at least one hindered phenol compound. Suitable hindered phenols for use with the rotomolding processes described herein include, but are not limited to, those having a molecular fragment according to one or more of Formula (IVa), (IVb), or (IVc): 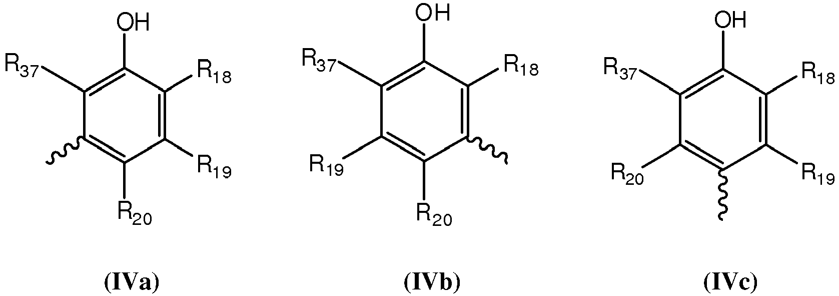 wherein " 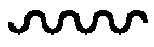 " indicates the point of attachment (via a carbon bond) of the molecular fragment to a parent compound, and wherein R₁₈ is chosen from hydrogen and a C₁₋₄ hydrocarbyl; R₁₉ and R₂₀ are the same or different and are independently chosen from hydrogen and a C₁-C₂₀ hydrocarbyl; and R₃₇ is chosen from a C₁-C₁₂ hydrocarbyl. In some embodiments, R₁₈ and R₃₇ are independently chosen from methyl and t-butyl.

The following compounds exemplify some hindered phenols that are suitable for use in the compositions and processes of the invention: (1,3,5-Tris(4-t-butyl-3-hydroxy-2,6-dimethylbenzyl)-1,3,5-triazine-2,4,6-(1H,3H,5H)-trione; 1,3,5-tris(3,5-di-tert-butyl-4-hydroxybenzyl)-1,3,5-triazine-2,4,6(1H,3H,5H)-trione (IRGANOX^{®} 3114); 1,1,3-Tris(2'-methyl-4'-hydroxy-5'-*t*-butylphenyl)butane; Triethylene glycol bis[3-(3-*t*-butyl-4-hydroxy-5-methylphenyl)propionate]; 4,4'-Thiobis(2-*t*-butyl-5-methylphenol); 2,2'-Thiodiethylene bis[3-(3-*t*-butyl-4-hydroxyl-5-methylphenyl)propionate]; Octadecyl 3-(3'-*t*-butyl-4'-hydroxy-5'-methylphenyl)propionate; Tetrakismethylene(3-*t*-butyl-4-hydroxy-5-methylhydrocinnamate)methane; *N*,*N*'-Hexamethylene bis[3-(3-*t*-butyl-4-hydroxy-5-methylphenyl)propionamide]; Di(4-tertiarybutyl-3-hydroxy-2,6-dimethyl benzyl) thiodipropionate; and octadecyl 3,5-di-(tert)-butyl-4-hydroxyhydrocinnamate.

Other phenols also suitable for use with processes and compositions of the invention are known to those of skill in the art and include, for example:
2,6-di-tert-butyl-4-methylphenol; 2-tert-butyl-4,6-dimethylphenol; 2,6-di-tert-butyl-4-ethylphenol; 2,6-di-tert-butyl-4-n-butylphenol; 2,6-di-tert-butyl-4 isobutylphenol; 2,6-dicyclopentyl-4-methylphenol; 2-(α-methylcyclohexyl)-4,6 dimethylphenol; 2,6-dioctadecyl-4-methylphenol; 2,4,6,-tricyclohexyphenol; and 2,6-di-tert-butyl-4-methoxymethylphenol;
2,2'-methylene-bis-(6-tert-butyl-4-methylphenol) (CYANOX^{®} 2246); 2,2'-methylene-bis-(6-tert-butyl-4-ethylphenol) (CYANOX^{®} 425); 2,2'-methylene-bis-(4-methyl-6-(α-methylcyclohexyl)phenol); 2,2'-methylene-bis-(4-methyl-6-cyclohexylphenol); 2,2'-methylene-bis-(6-nonyl-4-methylphenol); 2,2'-methylene-bis-(6-nonyl-4methylphenol); 2,2'-methylene-bis-(6-(α-methylbenzyl)-4-nonylphenol); 2,2'-methylene-bis-(6-(α, α-dimethylbenzyl)-4-nonyl-phenol); 2,2'-methylene-bis-(4,6-di-tert-butylphenol); 2,2'-ethylidene-bis-(6-tert-butyl-4-isobutylphenol); 4,4'methylene-bis-(2,6-di-tert-butylphenol); 4,4'-methylene-bis-(6-tert-butyl-2-methylphenol); 1,1-bis-(5-tert-butyl-4-hydroxy-2-methylphenol)butane 2,6-di-(3-tert-butyl-5-methyl-2-hydroxybenzyl)-4-methylphenol; 1,1,3-tris-(5-tert-butyl-4-hydroxy-2-methylphenyl)butane; 1,1-bis-(5-tert-butyl-4-hydroxy2-methylphenyl)-3-dodecyl-mercaptobutane; ethyleneglycol-bis-(3,3,-bis-(3 '-tert-butyl-4'-hydroxyphenyl)-butyrate)-di-(3-tert-butyl-4-hydroxy-5-methylpenyl)-dicyclopentadiene; di-(2-(3'-tert-butyl-2'hydroxy-5'methylbenzyl)-6-tert-butyl-4-methylpheny- l)terephthalate; and other phenolics such as monoacrylate esters of bisphenols such as ethylidiene bis-2,4-di-t-butylphenol monoacrylate ester;
Hydroquinones such as 2,6-di-tert-butyl-4-methoxyphenol; 2,5-di-tert-butylhydroquinone; 2,5-di-tert-amyl-hydroquinone; and 2,6-diphenyl-4-octadecyloxyphenol; and

Thiodiphenyl ethers such as 2,2'-thio-bis-(6-tert-butyl-4-methylphenol); 2,2'-thio-bis-(4-octylphenol); 4,4'thio-bis-(6-tert-butyl-3-methylphenol); and 4,4'-thio-bis-(6-tert-butyl-2-methylphenol).

The stabilizer compositions may further include one or more co-stabilizers and/or additives that include, but are not limited to: hindered amine light stabilizers, hindered hydroxyl benzoates, nickel phenolates, ultraviolet light stabilizers, antioxidants, and combinations thereof in an amount effective to stabilize the organic material against the degradative effects of visible and/or ultraviolet light radiation.

Suitable hindered amine light stabilizers for use with the processes and stabilizer compositions include, for example, compounds having a molecular fragment according to 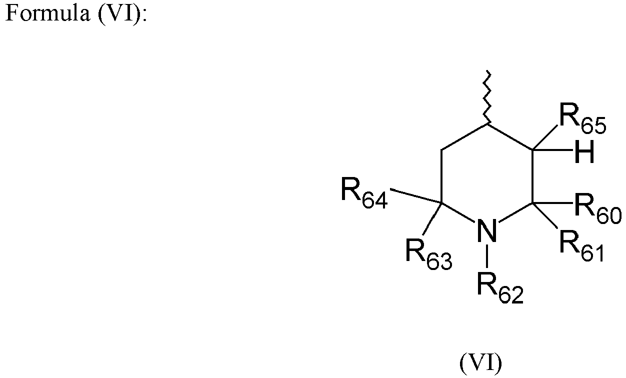 wherein
R₆₂ is chosen from a member selected from the group consisting of hydrogen; OH; C₁-C₂₀ hydrocarbyl; -CH₂CN; C₁-C₁₂ acyl; and C₁-C₁₈ alkoxy;
R₆₅ is chosen from a member selected from the group consisting of hydrogen; and C₁-C₈ hydrocarbyl; and
each of R₆₀, R₆₁, R₆₃, and R₆₄ is independently chosen from a C₁-C₂₀ hydrocarbyl, or R₆₀ and R₆₁ and/or R₆₃ and R₆₄ taken together with the carbon to which they are attached form a C₅-C₁₀ cycloalkyl;
   or 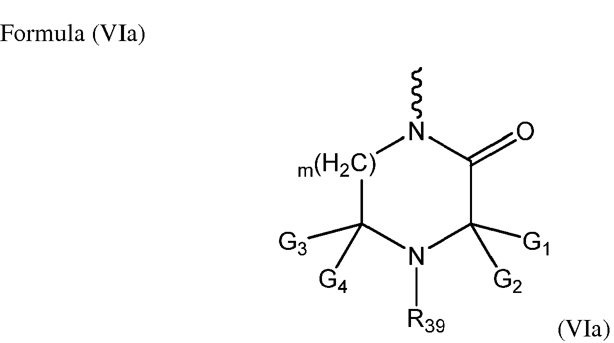 wherein
   m is an integer from 1 to 2;
   R₃₉ is chosen from: hydrogen; OH; C₁-C₂₀ hydrocarbyl; -CH₂CN; C₁-C₁₂ acyl; and C₁-C₁₈ alkoxy; and
   each of G₁-G₄ is independently chosen from C₁-C₂₀ hydrocarbyl.
   Hindered amine light stabilizers particularly suitable for use with the present invention include, but are not limited to, bis(2,2,6,6-tetramethylpiperidin-4-yl) sebacate; bis(2,2,6,6-tetramethylpiperidin-4-yl)succinate; bis(1,2,2,6,6-pentamethylpiperidin-4-yl)sebacate; bis(1-octyloxy-2,2,6,6-tetramethylpiperidin-4-yl)sebacate; bis(1,2,2,6,6-pentamethylpiperidin-4-yl) n-butyl 3,5-di-tert-butyl-4-hydroxybenzylmalonate; a condensate of 1-(2-hydroxyethyl)-2,2,6,6-tetramethyl-4-hydroxypiperidine and succinic acid; 2,2,6,6-tetramethylpiperidin-4-yl stearate; 2,2,6,6-tetramethylpiperidin-4-yl dodecanate; 1,2,2,6,6-pentamethylpiperidin-4-yl stearate; 1,2,2,6,6-pentamethylpiperidin-4-yl dodecanate; a condensate of N,N'-bis(2,2,6,6-tetramethylpiperidin-4-yl)hexamethylenediamine and 4-tert-octylamino-2,6-dichloro-1,3,5-triazine; tris(2,2,6,6-tetramethylpiperidin-4-yl) nitrilotriacetate; tetrakis(2,2,6,6-tetramethylpiperidin-4-yl)- 1,2,3,4-butanetetracarboxylate; 4-benzoyl-2,2,6,6-tetramethylpiperidine; 4-stearyloxy-2,2,6,6-tetramethylpiperidine; bis(1,2,2,6,6-pentamethylpiperidyl)-2-n-butyl-2-(2-hydroxy-3,5-di-tert-butylbenzyl)malonate; 3-n-octyl-7,7,9,9-tetramethyl-1,3,8-triazaspiro[4.5]decan-2,4-dione; bis(1-octyloxy-2,2,6,6-tetramethylpiperidyl)sebacate; bis(1-octyloxy-2,2,6,6-tetramethylpiperidyl)succinate; a condensate of N,N'-bis(2,2,6,6-tetramethylpiperidin-4-yl)hexamethylenediamine and 4-morpholino-2,6-dichloro-1,3,5-triazine; a condensate of 2-chloro-4,6-bis(4-n-butylamino-2,2,6,6-tetramethylpiperidyl)-1,3,5-triazine and 1,2-bis(3-aminopropylamino)ethane; a condensate of 2-chloro-4,6-bis(4-n-butylamino-1,2,2,6,6-pentamethylpiperidyl)-1,3,5-triazine and 1,2-bis-(3- aminopropylamino)ethane; 8-acetyl-3-dodecyl-7,7,9,9-tetramethyl-1,3,8-triazaspiro[4.5]decane-2,4-dione; 3-dodecyl-1-(2,2,6,6-tetramethylpiperidin-4-yl)pyrrolidin-2,5-dione; 3-dodecyl-1-(1-ethanoyl-2,2,6,6-tetramethylpiperidin-4-yl)pyrrolidin-2,5-dione; 3-dodecyl-1-(1,2,2,6,6-pentamethylpiperidin-4-yl)pyrrolidine-2,5-dione; a mixture of 4-hexadecyloxy- and 4-stearyloxy-2,2,6,6-tetramethylpiperidine; a condensate of N,N'-bis(2,2,6,6-tetramethylpiperidin-4-yl)hexamethylenediamine and 4-cyclohexylamino-2,6-dichloro-1,3,5-triazine; a condensate of 1,2-bis(3-aminopropylamino)ethane, 2,4,6-trichloro-1,3,5-triazine and 4-butylamino-2,2,6,6-tetramethylpiperidine; 2-undecyl-7,7,9,9-tetramethyl-1-oxa-3,8-diaza-4-oxospiro[4.5]decane; oxo-piperanzinyl-triazines; a reaction product of 7,7,9,9-tetramethyl-2-cycloundecyl-1-oxa-3,8-diaza-4-oxospiro[4.5]decane and epichlorohydrin;
   N-alkoxy hindered amine light stabilizers including, but not limited to, tetrakis(2,2,6,6-tetramethyl-4-piperidyl) butane-1,2,3,4-tetracarboxylate (MARK^{®} LA-57;); 1,2,3,4-butanetetracarboxylic acid, tetrakis(1,2,2,6,6-pentamethyl-4-piperidinyl)ester (MARK^{®} LA-52); 1,2,3,4-butanetetracarboxylic acid, 1,2,2,6,6-pentamethyl-4-piperdinyl tridecyl ester (MARK^{®} LA-62); 1,2,3,4-butanetetracarboxylic acid, 2,2,6,6-tetramethyl-4-piperidinyl tridecyl ester (MARK^{®} LA-67); 1,2,3,4-butanetetracarboxylic acid, polymer with 2,2,6,6-tetramethyl-2,4,8,10-tetraoxaspiro[5.5]-undecane-3,9-diethanol,1,2,2,6,6-pentamethyl-4-piperdinyl ester (MARK^{®} LA-63); 1,2,3,4-butanetetracarboxylic acid, polymer with 2,2,6,6-tetramethyl-2,4,8,10-tetraoxaspiro[5.5]-undecane-3,9-diethanol, 2,2,6,6-tetramethyl-4-piperdinyl ester (MARK^{®} LA-68); bis(1-undecanoxy-2,2,6,6-tetramethylpiperidin-4-yl)carbonate (MARK^{®} LA-81; aka STAB^{®} LA-81 available from Adeka Palmarole, Saint-Louis, France); TINUVIN^{®} 123; TINUVIN^{®} NOR 371; TINUVIN ^{®} XT-850/XT-855; FLAMESTAB^{®} NOR 116; and those disclosed in EP 0 889 085;
   hydroxyl-substituted N-alkoxy HALS including, but not limited to, those disclosed in U.S. Patent No. 6,271,377 such as 1-(2-hydroxy-2-methylpropoxy)-2,2,6,6-tetramethyl-4-piperdinol; 1-(2-hydroxy-2-methylpropoxy)-4-octadecanoyloxy-2,2,6,6-tetramethylpiperidine; 1-(4-octadecanoyloxy-2,2,6,6-tetramethylpiperidin-1-yloxy)-2-octadecanoyloxy-2-methylpropane; 1-(2-hydroxyethyl)-2,2,6,6-tetramethyl-4-piperdinol; a reaction product of 1-(2-hydroxyethyl)-2,2,6,6-tetramethyl-4-piperdinol and dimethylsuccinate;
   any of the tetramethylpiperidyl groups disclosed in WO 2007/104689 including, but not limited to, 2,2,4,4-tetramethyl-7-oxa-3,20-diazadispiro[5.1.11.2]heneicosan-21-one (HOSTAVIN^{®} N20); the ester of 2,2,6,6-tetramethyl-4-piperidinol with higher fatty acids (CYASORB^{®} 3853); 3-dodecyl-1-(2,2,6,6-tetramethyl-4-piperidyl)pyrrolidine-2,5-dione (SANDUVOR^{®} 3055); and their wax reaction products such as HALS NOW (LS X-N-O-W1);
   piperizinone compounds and derivatives thereof disclosed in U.S. Patent Nos. 6,843,939; 7,109,259; 4,240,961; 4,480,092; 4,629,752; 4,639,479; 5,013,836; 5,310,771; and WO 88/08863 including, but not limited to, 1H-Pyrrole-2,5-dione, 1-octadecyl-, polymer with (1-methylethenyl)benzene and 1-(2,2,6,6-tetramethyl-4-piperidinyl)-1H-pyrrole-2,5-dione; piperazinone, 1,1',1"-[1,3,5-triazine-2,4,6-triyltris[(cyclohexylimino)-2,1-ethanediyl]]tris[3,3,5,5-tetramethyl-; piperazinone, 1,1',1"-[1,3,5-triazine-2,4,6-triyltris[(cyclohexylimino)-2,1-ethanediyl]]tris[3,3,4,5,5-pentamethyl-; the reaction product of 7,7,9,9-tetramethyl-2-cycloundecyl-1-oxa-3,8-diaza-4-oxospiro[4.5]decane and epichlorohydrin; the condensate of N,N'-bis(2,2,6,6-tetramethylpiperidin-4-yl)hexamethylenediamine and 4-cyclohexylamino-2,6-dichloro-1,3,5-triazine; the condensate of 1,2-bis(3-aminopropylamino)ethane, 2,4,6-trichloro-1,3,5-triazine and 4-butylamino-2,2,6,6-tetramethylpiperidine; the condensate of N,N'-bis(2,2,6,6-tetramethylpiperidin-4-yl)hexamethylenediamine and 4-morpholino-2,6-dichloro-1,3,5-triazine; the condensate of 2-chloro-4,6-bis(4-n-butylamino-2,2,6,6-tetramethylpiperidyl)-1,3,5-triazine and 1,2-bis(3-aminopropylamino)ethane; the condensate of 2-chloro-4,6-bis(4-n-butylamino-1,2,2,6,6-pentamethylpiperidyl)-1,3,5-triazine and 1,2-bis-(3-aminopropylamino)ethane; 2-[(2-hydroxyethyl)amino]-4,6-bis[N-(1-cyclohexyloxy-2,2,6,6-tetramethylpiperidin-4-yl)butylamino-1,3,5-triazine; propanedioic acid, [(4-methoxyphenyl)-methylene]-bis-(1,2,2,6,6-pentamethyl-4-piperidinyl) ester; tetrakis(2,2,6,6-tetramethylpiperidin-4-yl)-1,2,3,4-butanetetracarboxylate; benzenepropanoic acid, 3,5-bis(1,1-dimethylethyl)-4-hydroxy-, 1-[2-[3-[3,5-bis(1,1-dimethylethyl)-4-hydroxyphenyl]-1-oxopropoxy]ethyl]-2,2,6,6-tetramethyl-4-piperidinyl ester; N-(1-octyloxy-2,2,6,6-tetramethylpiperidin-4-yl)-N'-dodecyloxalamide; tris(2,2,6,6-tetramethylpiperidin-4-yl) nitrilotriacetate; 1,5-dioxaspiro{5,5}undecane-3,3-dicarboxylic acid, bis(1,2,2,6,6-pentamethyl-4-piperidinyl): 1,5-dioxaspiro{5,5}undecane-3,3-dicarboxylic acid, bis(2,2,6,6-tetramethyl-4-piperidinyl); the condensate of 1-(2-hydroxyethyl)-2,2,6,6-tetramethyl-4-hydroxypiperidine and succinic acid; the condensate of N,N'-bis(2,2,6,6-tetramethylpiperidin-4-yl)hexamethylenediamine and 4-tert-octylamino-2,6-dichloro-1,3,5-triazine; 1,2,3,4-butanetetracarboxylic acid, 1,2,2,6,6-pentamethyl-4-piperidinyl tridecyl ester; tetrakis(2,2,6,6-tetramethylpiperidin-4-yl)-1,2,3,4-butanetetracarboxylate; 1,2,3,4-butanetetracarboxylic acid, 2,2,6,6-tetramethyl-4-piperidinyl tridecyl ester; tetrakis(1,2,2,6,6-pentamethylpiperidin-4-yl)-1,2,3,4-butanetetracarboxylate; mixture of 2,2,4,4-tetramethyl-21-oxo-7-oxa-3.20-diazaspiro(5.1.11.2)-heneicosane-20-propanoic acid-dodecylester and 2,2,4,4-tetramethyl-21-oxo-7-oxa-3.20-diazaspiro(5.1.11.2)-heneicosane-20-propanoic acid-tetradecylester; 1H,4H,5H,8H-2,3a,4a,6,7a,8a-hexaazacyclopenta[def]fluorene-4,8-dione, hexahydro-2,6-bis(2,2,6,6-tetramethyl-4-piperidinyl)-; polymethyl[propyl-3-oxy(2',2',6',6'-tetramethyl-4,4'-piperidinyl)]siloxane; polymethyl[propyl-3-oxy(1',2',2',6',6'-pentamethyl-4,4'-piperidinyl)]siloxane; copolymer of methylmethacrylate with ethyl acrylate and 2,2,6,6-tetramethylpiperidin-4-yl acrylate; copolymer of mixed C₂₀ to C₂₄ alpha-olefins and (2,2,6,6-tetramethylpiperidin-4-yl)succinimide; 1,2,3,4-butanetetracarboxylic acid, polymer with β,β,β',β'-tetramethyl-2,4,8,10-tetraoxaspiro[5.5]undecane-3,9-diethanol, 1,2,2,6,6-pentamethyl-4-piperidinyl ester; 1,2,3,4-butanetetracarboxylic acid, polymer with β,β,β',β'-tetramethyl-2,4,8,10-tetraoxaspiro[5.5]undecane-3,9-diethanol, 2,2,6,6-tetramethyl-4-piperidinyl ester copolymer; 1,3-benzenedicarboxamide, N,N'-bis(2,2,6,6-tetramethyl-4-piperidinyl; 1,1'-(1,10-dioxo-1,10-decanediyl)-bis(hexahydro-2,2,4,4,6-pentamethylpyrimidine; ethane diamide, N-(1-acetyl-2,2,6,6-tetramethylpiperidinyl)-N'-dodecyl; formamide, N,N'-1,6-hexanediylbis[N-(2,2,6,6-tetramethyl-4-piperidinyl); D-glucitol, 1,3:2,4-bis-O-(2,2,6,6-tetramethyl-4-piperidinylidene)-; 2,2,4,4-tetramethyl-7-oxa-3,20-diaza-21-oxo-dispiro[5.1.11.2]heneicosane; propanamide, 2-methyl-N-(2,2,6,6-tetramethyl-4-piperidinyl)-2-[(2,2,6,6-tetramethyl-4-piperidinyl)amino]-; 7-oxa-3,20-diazadispiro[5.1.11.2]heneicosane-20-propanoic acid, 2,2,4,4-tetramethyl-21-oxo-, dodecyl ester; N-(2,2,6,6-tetramethylpiperidin-4-yl)-β-aminopropionic acid dodecyl ester; N-(2,2,6,6-tetramethylpiperidin-4-yl)-N'-aminooxalamide; propanamide, N-(2,2,6,6-tetramethyl-4-piperidinyl)-3-[(2,2,6,6-tetramethyl-4-piperidinyl)amino]-; mixture of 4-hexadecyloxy- and 4-stearyloxy-2,2,6,6-tetramethylpiperidine; 3-dodecyl-1-(1,2,2,6,6-pentamethylpiperidin-4-yl)pyrrolidine-2,5-dione; 3-dodecyl-1-(1-ethanoyl-2,2,6,6-pentamethylpiperidin-4-yl)pyrrolidine-2,5-dione; bis(2,2,6,6-tetramethylpiperidin-4-yl)succinate; bis(1,2,2,6,6-pentamethylpiperidin-4-yl) n-butyl 3,5-di-tert-butyl-4-hydroxybenzylmalonate; tris(2,2,6,6-tetramethylpiperidin-4-yl) nitrilotriacetate; 1,1'-(1,2-ethanediyl)bis(3,3,5,5-tetramethylpiperazinone); 4-benzoyl-2,2,6,6-tetramethylpiperidine; 4-stearyloxy-2,2,6,6-tetramethylpiperidine; bis(1,2,2,6,6-pentamethylpiperidyl)-2-n-butyl-2-(2-hydroxy-3,5-di-tert-butylbenzyl)malonate; 3-n-octyl-7,7,9,9-tetramethyl-1,3,8-triazaspiro[4.5]decan-2,4-dione; bis(1-octyloxy-2,2,6,6-tetramethylpiperidyl)sebacate; bis(1-octyloxy-2,2,6,6-tetramethylpiperidyl)succinate; 8-acetyl-3-dodecyl-7,7,9,9-tetramethyl-1,3,8-triazaspiro[4.5]decane-2,4-dione; 3-dodecyl-1-(2,2,6,6-tetramethylpiperidin-4-yl)pyrrolidin-2,5-dione; 3-dodecyl-1-(1-ethanoyl-2,2,6,6-tetramethylpiperidin-4-yl)pyrrolidin-2,5-dione; 3-dodecyl-1-(1,2,2,6,6-pentamethylpiperidin-4-yl)pyrrolidine-2,5-dione; a mixture of 4-hexadecyloxy- and 4-stearyloxy-2,2,6,6-tetramethylpiperidine; 2-undecyl-7,7,9,9-tetramethyl-1-oxa-3,8-diaza-4-oxospiro[4.5]decane; 1,5-dioxaspiro{5,5}undecane-3,3-dicarboxylic acid, bis(2,2,6,6-tetramethyl-4-piperidinyl) and 1,5-dioxaspiro{5,5}undecane-3,3-dicarboxylic acid, bis(1,2,2,6,6-pentamethyl-4-piperidinyl); N¹-(β-hydroxyethyl)3,3-pentamethylene-5,5-dimethylpiperazin-2-one; N¹-tert-octyl-3,3,5,5-tetramethyl-diazepin-2-one; N¹-tert-octyl-3,3-pentamethylene-5,5-hexamethylene-diazepin-2-one; N¹-tert-octyl-3,3-pentamethylene-5,5-dimethylpiperazin-2-one; trans-1,2-cyclohexane-bis-(N¹-5,5-dimethyl-3,3-pentamethylene-2-piperazinone; trans-1,2-cyclohexane-bis-(N¹-3,3,5,5-dispiropentamethylene-2-piperazinone); N¹-isopropyl-1,4-diazadispiro-(3,3,5,5)pentamethylene-2-piperazinone; N¹-isopropyl-1,4-diazadispiro-3,3-pentamethylene-5,5-tetramethylene-2-piperazinone; N¹-isopropyl-5,5-dimethyl-3,3-pentamethylene-2-piperazinone; trans-1,2-cyclohexane-bis-N¹-(dimethyl-3,3-pentamethylene-2-piperazinone); N¹-octyl-5,5-dimethyl-3,3-pentamethylene-1,4-diazepin-2-one; and N¹-octyl-1,4-diazadispiro-(3,3,5,5)pentamethylene-1,5-diazepin-2-one. Other sterically hindered amines suitable for use with the invention include, for example, any of those disclosed in EP 1 308 084.

The hindered amine component can be present in an amount from 0.01 to 10 % by weight based on the total weight of the organic material to be stabilized. Preferably, the amount of hindered amine is available from 0.05 to 5 %, and more preferably from 0.1 to 3 % by weight based on the total weight of the organic material to be stabilized.

Other light stabilizers suitable for use with the present invention include one or more of the following:
2-(2'-Hydroxyphenyl)benzotriazoles, for example 2-(2'-hydroxy-5'-methylphenyl)-benzotriazole; 2-(3',5'-di-tert-butyl-2'-hydroxyphenyl)benzotriazole; 2-(5'-tert-butyl-2'-hydroxyphenyl)benzotriazole; 2-(2'-hydroxy-5'-(1,1,3,3-tetramethylbutyl)phenyl)benzotriazole; 2-(3',5'-di-tert-butyl-2'-hydroxyphenyl)-5-chloro-benzotriazole; 2-(3'-tert-butyl-2'-hydroxy-5'-methylphenyl)-5-chloro-benzotriazole; 2-(3'-sec-butyl-5'-tert-butyl-2'-hydroxyphenyl)benzotriazole; 2-(2'-hydroxy-4'-octyloxyphenyl)benzotriazole; 2-(3',5'-di-tert-amyl-2'-hydroxyphenyl)benzotriazole; 2-(3',5'-bis-(α,α-dimethylbenzyl)-2'-hydroxyphenyl)benzotriazole; 2-(3'-tert-butyl-2'-hydroxy-5'-(2-octyloxycarbonylethyl)phenyl)-5-chloro-benzotriazole; 2-(3'-tert-butyl-5'-[2-(2-ethylhexyloxy)-carbonylethyl]-2'-hydroxyphenyl)- 5-chloro-benzotriazole; 2-(3'-tert-butyl-2'-hydroxy-5'-(2-methoxycarbonylethyl)phenyl)-5-chloro-benzotriazole; 2-(3'-tert-butyl-2'-hydroxy-5'-(2-methoxycarbonylethyl)phenyl)benzotriazole; 2-(3'-tert-butyl-2'-hydroxy-5'-(2-octyloxycarbonylethyl)phenyl)benzotriazole; 2-(3'-tert-butyl-5'-[2-(2-ethylhexyloxy)carbonyl]-2'-hydroxyphenyl)benzotriazole; 2-(3'-dodecyl-2'-hydroxy-5'-methylphenyl)benzotriazole; 2-(3'-tert-butyl-2'-hydroxy-5'-(2-isooctyloxycarbonylethyl)phenylbenzotriazole; 2,2'-methylene-bis[4-(1,1,3,3-tetramethylbutyl)-6-benzotriazole-2-ylphenol ]; the transesterification product of 2-[3'-tert-butyl-5'-(2-methoxycarbonylethyl)-2'-hydroxyphenyl]-2H-benzotriazole with polyethylene glycol 300; [R-CH₂CH₂-COO-CH₂CH₂]₂ where R=3'-tert-butyl-4'-hydroxy-5'-2H-benzotriazol-2-ylphenyl; 2-[2'-hydroxy-3'-(α,α-dimethylbenzyl)-5'-(1,1,3,3-tetramethylbutyl)-phenyl]benzotriazole; 2-[2'-hydroxy-3'-(1,1,3,3-tetramethylbutyl)-5'-(α,α-dimethylbenzyl)-phenyl]benzotriazole;
2-Hydroxybenzophenones, for example the 4-hydroxy, 4-methoxy, 4-octyloxy, 4-decyloxy, 4-dodecyloxy, 4-benzyloxy, 4,2',4'-trihydroxy and 2'-hydroxy-4,4'-dimethoxy derivatives;
Esters of substituted and unsubstituted benzoic acids, as for example 4-tertbutyl-phenyl salicylate, phenyl salicylate, octylphenyl salicylate, dibenzoyl resorcinol, bis(4-tert-butylbenzoyl)resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate, 2-methyl-4,6-di-tert-butylphenyl 3,5-di-tert-butyl-4-hydroxybenzoate;
Nickel compounds, for example nickel complexes of 2,2'-thio-bis-[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of the monoalkyl esters, e.g. the methyl or ethyl ester, of 4-hydroxy-3,5-di-tert-butylbenzylphosphonic acid, nickel complexes of ketoximes, e.g. of 2-hydroxy-4-methylphenyl undecylketoxime, nickel complexes of 1-phenyl-4-lauroyl-5-hydroxypyrazole, with or without additional ligands; and 2-(2'-hydroxyphenyl)-1,3,5-triazine compounds according to Formula (VII): 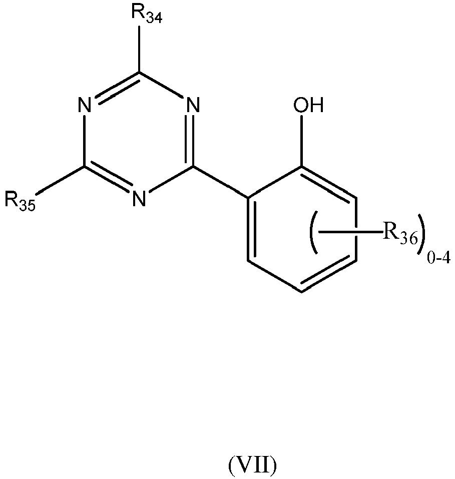
wherein each of R₃₄ and R₃₅ is independently chosen from an optionally substituted C₆-C₁₀ aryl group, C₁-C₁₀ hydrocarbyl-substituted amino, C₁-C₁₀ acyl and C₁-C₁₀ alkoxyl; and wherein R₃₆ is a substituent that is the same or different at from 0 to 4 positions of the phenoxy portion of Formula VII and is independently chosen from hydroxyl, C₁-C₁₂ hydrocarbyl, C₁-C₁₂ alkoxyl, C₁-C₁₂ alkoxyester, and C₁-C₁₂ acyl. Such 2-(2-Hydroxyphenyl)-1,3,5-triazines include, but are not limited to, 4,6-bis-(2,4-dimethylphenyl)-2-(2-hydroxy-4-octyloxyphenyl)-s-triazine (CYASORB^{®} 1164 available from Cytec Industries Inc.); 4,6-bis-(2,4-dimethylphenyl)-2-(2,4-- dihydroxyphenyl)-s-triazine; 2,4-bis(2,4-dihydroxyphenyl)-6-(4-chlorophenyl)-s-triazine; 2,4-bis[2-hydroxy-4-(2-hydroxy-ethoxy)phenyl]-6-(4-chlorophenyl)-s-triazine; 2,4-bis[2-hydroxy-4-(2-hydroxy-4-(2-hydroxy-ethoxy)phenyl]-6-(2,4-dimethylphenyl)-s-triazine; 2,4-bis[2-hydroxy-4-(2-hydroxyethoxy)phenyl]-6-(4-bromophenyl)-s-triazine; 2,4-bis[2-hydroxy-4-(2-acetoxyethoxy)phenyl]-6-(4-chlorophenyl)-s-triazine; 2,4-bis(2,4-dihydroxyphenyl)-6-(2,4-dimethylphenyl)-s-triazine; 2,4-bis(4-biphenylyl)-6-[2-hydroxy-4-[(octyloxycarbonyl)ethylideneoxy]phenyl]-s-triazine; 2,4-bis(4-biphenylyl)-6-[2-hydroxy-4-(2-ethylhexyloxy)phenyl]-s-triazine; 2-phenyl-4-[2-hydroxy-4-(3-sec-butyloxy-2-hydroxypropyloxy)phenyl]-6-[2-hydroxy-4-(3-sec-amyloxy-2-hydroxypropyloxy)phenyl]-s-triazine; 2,4-bis(2,4-dimethylphenyl)-6-[2-hydroxy-4(- 3-benzyloxy-2-hydroxypropyloxy)phenyl]-s-triazine; 2,4-bis(2-hydroxy-4-n-butyloxyphenyl)-6-(2,4-di-n-butyloxyphenyl)-s-triazine; 2,4-bis(2,4-dimethylphenyl)-6-[2-hydroxy-4-(3-nonyloxy-2-hydroxypropylox- y)-5-α-cumylphenyl]-s-triazine; methylenebis-{2,4-bis(2,4-dimethylphenyl)-6-[2-hydroxy-4-(3-butyloxy-2-hydroxypropoxy)phenyl]-s-triazine}; methylene bridged dimer mixture bridged in the 3:5', 5:5' and 3:3' positions in a 5:4:1 ratio; 2,4,6-tris(2-hydroxy-4-isooctyloxycarbonyliso-propylideneoxy-phenyl)-s-triazine; 2,4-bis(2,4-dimethylphenyl)-6-(2-hydroxy-4-hexyloxy-5-α-cumylphenyl)-s-triazine; 2-(2,4,6-trimethylphenyl)-4,6-bis[2-hydroxy-4-(3-butyloxy-2-hydroxypropyloxy)phenyl]-s-triazine; 2,4,6-tris[2-hydroxy-4-(3-sec-butyloxy-2-hydroxypropyloxy)-phenyl]-s-triazine; mixture of 4,6-bis-(2,4-dimethylphenyl)-2-(2-hydroxy-4-(3-dodecyloxy-2-hydroxypropoxy)phenyl)-s-triazine and 4,6-bis-(2,4-dimethylphenyl)-2-(2-hydroxy-4-(3-tridecyloxy-2-hydroxypropoxy)phenyl)-s-triazine (TINUVIN^{®} 400 available from BASF Corp.); 4,6-bis-(2,4-dimethylphenyl)-2-(2-hydroxy-4(3-(2-ethylhexyloxy)-2-hydroxypropoxy)-phenyl)-s-triazine; 4,6-diphenyl-2-(4-hexyloxy-2-hydroxyphenyl)-s-triazine; 2-(4,6-Diphenyl-1,3,5-triazin-2-yl)-5-[2-(2-ethylhexanoyloxy)ethoxy]phenol (ADK STAB^{®} LA-46 available from Adeka Palmarole, Saint-Louis, France); 2,4,6-tris(2-hydroxy-4-octyloxyphenyl)-1,3,5-triazine; propanoic acid, 2,2',2"-[1,3,5-triazine-2,4,6-triyltris[(3-hydroxy-4, 1-phenylene)oxy]]tris-1,1',1" -trioctyl ester (TINUVIN^{®} 477 available from BASF Corp.); propanoic acid, 2-[4-[4,6-bis([1,1'-biphenyl]-4-yl)-1,3,5-triazin-2yl]-3-hydroxyphenoxyl]-isooctyl ester (TINUVIN^{®} 479 available from BASF Corp.); and combinations thereof. Other triazine compounds suitable for use with the present invention include those described in EP 1 308 084 (such as formula IId), and in U.S. Patent Application No. 13/144861 (Publication No. 2011/0272648).

In certain embodiments, the stabilizer compositions include a blend of at least one hindered amine light stabilizer and at least one ultraviolet light absorber.

Antioxidants suitable for use with the stabilizer compositions include any of those antioxidants conventionally known in the art. Particularly suitable antioxidants include any of those listed in U.S. Patent No. 6,444,733. In certain embodiments, the stabilizer compositions can further include a tocopherol compound (e.g., α-tocopherol, β-tocopherol, γ-tocopherol, δ-tocopherol, and mixtures thereof), and/or a tocotrienol compound *(e.g.,* α, β, γ, δ-tocotrienol, and mixtures thereof) Further embodiments of the stabilizer compositions include at least one compound chosen from:
a hydroxylamine compound according to Formula VIII: 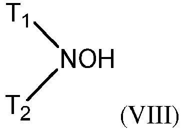 wherein
T₁ is chosen from C₁-C₃₆ hydrocarbyl, C₅-C₁₂ cycloalkyl, and C₇-C₉ aralkyl, optionally substituted; and
T₂ is chosen from hydrogen or T₁; and
a tertiary amine oxide compound according to Formula IX: 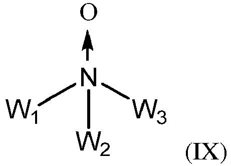 wherein
W₁ and W₂ are each independently chosen from a C₆-C₃₆ hydrocarbyl chosen from a straight or branched chain C₆-C₃₆ alkyl, C₆-C₁₂ aryl, C₇-C₃₆ aralkyl, C₇-C₃₆ alkaryl, C₅-C₃₆ cycloalkyl, C₆-C₃₆ alkcycloalkyl; and C₆-C₃₆ cycloalkylalkyl;
W₃ is chosen from a C₁-C₃₆ hydrocarbyl chosen from a straight or branched chain C₁-C₃₆ alkyl, C₆-C₁₂ aryl, C₇-C₃₆ aralkyl, C₇-C₃₆ alkaryl, C₅-C₃₆ cycloalkyl, C₆-C₃₆ alkcycloalkyl; and C₆-C₃₆ cycloalkylalkyl; with the proviso that at least one of W₁, W₂ and W₃ contains a β carbon-hydrogen bond; and wherein said alkyl, aralkyl, alkaryl, cycloalkyl, alkcycloalkyl and cycloalkylalkyl groups may be interuppted by one to sixteen -O-, -S-, -SO-, -SO₂ -, -COO-, -OCO-, -CO-, -NW₄-, -CONW₄- and -NW₄ CO- groups, or wherein said alkyl, aralkyl, alkaryl, cycloalkyl, alkcycloalkyl and cycloalkylallyl groups may be substituted by one to sixteen groups selected from -OW₄, -SW₄, -COOW₄, -OCOW₄, -COW₄, -N(W₄)₂, -CON(W₄)₂, -NW₄COW₄ and 5- and 6-membered rings containing the -C(CH₃)(CH₂Rₓ)NL(CH₂Rₓ)(CH₃)C- group or wherein said alkyl, aralkyl, alkaryl, cycloalkyl, alkcycloalkyl and cycloalkylalkyl groups are both interuppted and substituted by the groups mentioned above; and wherein
W₄ is chosen from hydrogen or C₁-C₈ alkyl;
Rₓ is chosen from hydrogen or methyl; and
L is chosen from a C₁-C₃₀ alkyl, a --C(O)R moiety wherein R is a C₁-C₃₀ straight or branched chain alkyl group, or a --OR moiety wherein R is a C₁-C₃₀ straight or branched chain alkyl group; and wherein said aryl groups may be substituted by one to three halogen, C₁-C₈ alkyl, C₁-C₈ alkoxy or combinations thereof.

In particular embodiments, preference is given to N,N-dihydrocarbylhydroxylamine compounds according to Formula VIII wherein T₁ and T₂ are independently chosen from benzyl, ethyl, octyl, lauryl, dodecyl, tetradecyl, hexadecyl, heptadecyl and octadecyl; or wherein T₁ and T are each the alkyl mixture found in hydrogenated tallow amine.

In certain embodiments, hydroxylamine compounds according to Formula VIII are chosen from: N,N-dibenzylhydroxylamine; N,N-diethylhydroxylamine; N,N-dioctylhydroxylamine; N,N-dilaurylhydroxylamine; N,N-didodecylhydroxylamine; N,N-ditetradecylhydroxylaamine; N,N-dihexadecylhydroxylamine; N,N-dioctadecylhydroxylamine; N-hexadecyl-N-tetradecylhydroxylamine; N-hexadecyl-N-heptadecylhydroxylamine; N-hexadecyl-N-octadecylhydroxylamine; N-heptadecyl-N-octadecylhydroxylamine; N,N-di(hydrogenated tallow)hydroxylamine; and N,N-di(alkyl)hydroxylamine produced by the direct oxidation of N,N-di(hydrogenated tallow)amine.

In certain embodiments, preference is given to those structures of Formula IX where W₁ and W₂ are independently benzyl or substituted benzyl. It is also possible for each of W₁, W₂, and W₃ to be the same residue. In other embodiments, W₁ and W₂ can be alkyl groups of 8 to 26 carbon atoms, more preferably alkyl groups of 10 to 26 carbon atoms. W₃ can be an alkyl group of 1 to 22 carbon atoms, more preferably methyl or substituted methyl. Other preferred amine oxides include those wherein W₁, W₂, and W₃ are the same alkyl groups of 6 to 36 carbon atoms. Preferably, all of the aforementioned residues for W₁, W₂, and W₃ are saturated hydrocarbon residues or saturated hydrocarbon residues containing at least one of the aforementioned -O-, -S-, -SO-, -CO₂-, -CO-, or -CON- moieties. Those skilled in the art will be able to envision other useful residues for each of W₁, W₂, and W₃ without detracting from the present invention.

The saturated amine oxides may also include poly(amine oxides). By poly(amine oxide) is meant tertiary amine oxides containing at least two tertiary amine oxides per molecule. Illustrative poly(amine oxides), also called "poly(tertiary amine oxides)", include, but are not limited to, the tertiary amine oxide analogues of aliphatic and alicyclic diamines such as, for example, 1,4-diaminobutane; 1,6-diaminohexane; 1,10-diaminodecane; and 1,4-diaminocyclohexane, and aromatic based diamines such as, for example, diamino anthraquinones and diaminoanisoles.

Suitable amine oxides for use with the invention also include tertiary amine oxides derived from oligomers and polymers of the aforementioned diamines. Useful amine oxides also include amine oxides attached to polymers, for example, polyolefins, polyacrylates, polyesters, polyamides, polystyrenes, and the like. When the amine oxide is attached to a polymer, the average number of amine oxides per polymer can vary widely as not all polymer chains need to contain an amine oxide. All of the aforementioned amine oxides may optionally contain at least one -O-, -S-, -SO-, CO₂-, -CO-, or -CONW₄-moiety. In a preferred embodiment, each tertiary amine oxide of the polymeric tertiary amine oxide contains a C₁ residue.

The groups W1, W2 and W3 of Formula IX may be attached to a molecule containing a hindered amine. Hindered amines are known in the art and the amine oxide of the present invention may be attached to the hindered amine in any manner and structural position of the hindered amine. Useful hindered amines when part of an amine oxide compound include those of the general Formulas X and XI: 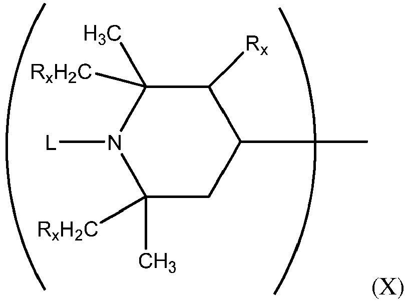 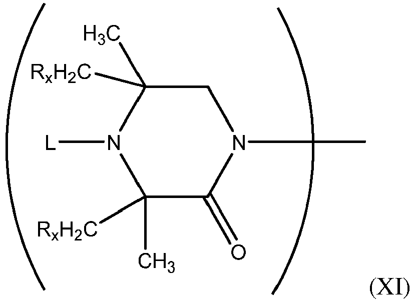 wherein L and Rₓ are defined as described above.

Also included are amine oxides containing more than one hindered amine and more than one saturated amine oxide per molecule. The hindered amine may be attached to a poly(tertiary amine oxide) or attached to a polymeric substrate, as discussed above.

The hydroxyl amine derivatives and/or amine oxide derivatives can be used in amounts, in total, of about 0.0005% to about 5%, in particular from about 0.001% to about 2%, typically from about 0.01% to about 2% by weight, based on the weight of the organic material to be stabilized.

In other embodiments, the stabilizer compositions include further optional additives that can include at least one compound chosen from co-additives; nucleating agents; fillers; reinforcing agents; and combinations thereof.

Examples of such additives include, but are not limited to:
Basic co-additives, for example, melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example calcium stearate, zinc stearate, magnesium behenate, magnesium stearate, sodium ricinoleate and potassium palmitate, antimony pyrocatecholate or zinc pyrocatecholate;
Nucleating agents, for example, inorganic substances such as talcum, metal oxides such as titanium dioxide or magnesium oxide, phosphates, carbonates or sulfates of, preferably, alkaline earth metals; organic compounds such as mono- or polycarboxylic acids and the salts thereof, e.g. 4-tert-butylbenzoic acid, adipic acid, diphenylacetic acid, sodium succinate or sodium benzoate; polymeric compounds such as ionic copolymers (ionomers);
Fillers and reinforcing agents, for example, calcium carbonate, silicates, glass fibres, glass bulbs, asbestos, talc, kaolin, mica, barium sulfate, metal oxides and hydroxides (e.g., aluminium hydroxide or magnesium hydroxide, carbon black, graphite, wood flour and flours or fibers of other natural products, synthetic fibers; impact modifiers;
Benzofuranones and indolinones, for example those disclosed in U.S. Pat. Nos. 4,325,863; 4,338,244; 5,175,312; 5,216,052; 5,252,643; 5,369,159; 5,488,117; 5,356,966; 5,367,008; 5,428,162; 5,428,177; 5,516,920; DE-A-4316611; DE-A-4316622; DE-A-4316876; EP-A-0589839 or EP-A-0591102 or 3-[4-(2-acetoxyethoxy)phenyl]-5,7-di-tert-butyl-benzofuran-2-one, 5,7-di-tert-butyl-3-[4-(2-stearoyloxyethoxy)phenyl]benzofuran-2-one, 3,3'-bis[5,7-di-tert-butyl-3-(4-[2-hydroxyethoxy]phenyl)benzofuran-2-one], 5,7-di-tert-butyl-3-(4-ethoxyphenyl)benzofuran-2-one, 3-(4-acetoxy-3,5-dimethylphenyl)-5,7-di-tert-butyl-benzofuran-2-one, 3-(3,5-dimethyl-4-pivaloyloxyphenyl)-5,7-di-tert-butyl-benzofuran-2-one, 3-(3,4-dimethylphenyl)-5,7-di-tert-butyl-benzofuran-2-one, 3-(2,3-dimethylphenyl)-5,7-di-tert-butyl-benzofuran-2-one;
Metal deactivators, for example N,N'-diphenyloxamide, N-salicylal-N'-salicyloyl hydrazine, N,N'-bis(salicyloyl)hydrazine, N,N'-bis(3,5-di-tert-butyl-4-hydroxyphenylpropionyl) hydrazine, 3-salicyloylamino-1,2,4-triazole, bis(benzylidene)oxalyl dihydrazide, oxanilide, isophthaloyl dihydrazide, sebacoyl bisphenylhydrazide, N,N'-diacetyladipoyl dihydrazide, N,N'-bis(salicyloyl)oxalyl dihydrazide, N,N'-bis(salicyloyl)thiopropionyl dihydrazide;
Nitrones, for example, N-benzyl-alpha-phenyl-nitrone, N-ethyl-alpha-methyl-nitrone, N-octyl-alpha-heptyl-nitrone, N-lauryl-alpha-undecyl-nitrone, N-tetradecyl-alpha-tridcyl-nitrone, N-hexadecyl-alpha-pentadecyl-nitrone, N-octadecyl-alpha-heptadecyl-nitrone, N-hexadecyl-alpha-heptadecyl-nitrone, N-ocatadecyl-alpha-pentadecyl-nitrone, N-heptadecyl-alpha-heptadecyl-nitrone, N-octadecyl-alpha-hexadecyl-nitrone, nitrone derived from N,N-di(hydrogenated tallow)hydroxylamine;
Thiosynergists, for example, dilauryl thiodipropionate or distearyl thiodipropionate; and

Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakis(β-dodecylmercapto)propionate.

Other additives suitable for use with the present invention that would not markedly impair the properties of the organic material to be stabilized are known to those of ordinary skill in the art and can include, for example, plasticisers, lubricants, emulsifiers, pigments, rheology additives, catalysts, flow-control agents, optical brighteners, flameproofing agents, antistatic agents, clarifying agents and blowing agents.

In certain embodiments, the stabilizer composition is present from 0.001 to 65.0 % by weight based on the total weight of the organic material composition to be stabilized and based on the number and type of stabilizing additives being added and/or the characteristics of the material to be stabilized. In some embodiments, the stabilizer composition is present from 0.01 to 50 % by weight of the total weight of the organic material, and preferably from 0.05 to 25 % by weight of the total, or from 0.1 to 10 % by weight of the total. Those of ordinary skill in the art will be able to readily determine the amount and type of stabilizing additive(s) that should be added based on preparations as known and/or described in the literature, or through no more than routine experimentation.

The stabilizer compositions can be readily blended with the organic material to be stabilized by any suitable method known to those of skill in the art. In certain embodiments, the components of the stabilizer compositions are mixed with the material to be stabilized by at least one technique chosen from extruding, pelletizing, grinding, and molding. In other embodiments, mixing can be performed by at least one of melting, dissolution in a solvent, direct mixing, and dry mixing.

The incorporation of components for the stabilizer composition and optional further additives into the organic material to be stabilized is carried out by any suitable method known to those of skill in the art, for example before or after molding or also by applying the dissolved or dispersed stabilizer mixture to the organic material to be stabilized, with or without subsequent evaporation of the solvent. The stabilizer components and optional further additives can also be added to the organic material to be stabilized in the form of a masterbatch.

Components of the stabilizer composition and optional further additives can also be added before or during, for example, polymerization or before crosslinking. They can also be incorporated into the organic material to be stabilized in pure form (i.e., neat and directly to the resin) or encapsulated in waxes, oils or polymers. Various additives can also be preblended (i.e., mixed together) for simple addition to the organic material to be stabilized. Components of the stabilizer composition and optional further additives can also be sprayed onto the organic material to be stabilized. They are able to dilute other additives (for example the conventional additives indicated above) or their melts so that they can be sprayed also together with these additives onto the materials to be stabilized. In the case of spherically polymerized polymers it may, for example, be advantageous to apply components of the stabilizer composition optionally together with other additives, by spraying.

Reference has been made to use of the stabilizer compositions for stabilizing an organic material. Accordingly, another aspect of the present invention provides a use of a stabilizer composition, as defined in the claims, in: (i) processes for stabilizing an organic material subject to degradation and/or discoloration due to effects from light, oxygen, and/or heat; (ii) processes for enhancing the processing stability of an organic material; and (iii) processes for reducing or preventing discoloration of an organic material. These processes are each achieved by adding before, during, or after processing a stabilizing amount of a stabilizer composition as described throughout the specification and claims to the organic material to be stabilized. In certain embodiments, a masterbatch composition containing the stabilizer composition will be added to the organic material to be stabilized.

Various nonliving organic materials suitable for stabilizing include, but are not limited to, polyolefins, polyesters, polyethers, polyketones, polyamides, natural and synthetic rubbers, polyurethanes, polystyrenes, high-impact polystyrenes, polyacrylates, polymethacrylates, polyacetals, polyacrylonitriles, polybutadienes, polystyrenes, acrylonitrile-butadiene-styrene, styrene acrylonitrile, acrylate styrene acrylonitrile, cellulosic acetate butyrate, cellulosic polymers, polyimides, polyamideimides, polyetherimides, polyphenylsulfides, polyphenyloxidepolysulfones, polyethersulfones, polyvinylchlorides, polycarbonates, polyketones, aliphatic polyketones, thermoplastic olefins, aminoresin cross-linked polyacrylates and polyesters, polyisocyanate cross-linked polyesters and polyacrylates, phenol/formaldehyde, urea/formaldehyde and melamine/formaldehyde resins, drying and non-drying alkyd resins, alkyd resins, polyester resins, acrylate resins cross-linked with melamine resins, urea resins, isocyanates, isocyanurates, carbamates, epoxy resins, cross-linked epoxy resins derived from aliphatic, cycloaliphatic, heterocyclic and aromatic glycidyl compounds, which are cross-linked with anhydrides or amines, polysiloxanes, Michael addition polymers, amines, blocked amines with activated unsaturated and methylene compounds, ketimines with activated unsaturated and methylene compounds, polyketimines in combination with unsaturated acrylic polyacetoacetate resins, polyketimines in combination with unsaturated acrylic resins, radiation curable compositions, epoxymelamine resins, organic dyes, cosmetic products, cellulose-based paper formulations, photographic film paper, fibers, waxes, and inks.

In certain embodiments, the nonliving organic material to be stabilized is a polyolefin. Examples of polyolefins suitable for such use with the stabilizer composition include at least the following:
(A) Polymers of monoolefins, for example polypropylene, polyisobutylene, polybut-1-ene, and poly-4-methylpent-1-ene, polymers of diolefins such as polyisoprene or polybutadiene, as well as polymers of cycloolefins, for instance of cyclopentene or norbornene, polyethylene (which optionally can be crosslinked), for example high density polyethylene (HDPE), high density and high molecular weight polyethylene (HDPE-HMW), high density and ultrahigh molecular weight polyethylene (HDPE-UHMW), medium density polyethylene (MDPE), low density polyethylene (LDPE), linear low density polyethylene (LLDPE), (VLDPE) and (ULDPE);
(B) Polyolefins, i.e. the polymers of monoolefins exemplified in (A), preferably polyethylene and polypropylene, can be prepared by different, and especially by the following, methods:
   i) radical polymerisation (normally under high pressure and at elevated temperature); or
   ii) catalytic polymerisation using a catalyst that normally contains one or more than one metal of groups IVb, Vb, VIb or VIII of the Periodic Table. These metals usually have one or more than one ligand, typically oxides, halides, alcoholates, esters, ethers, amines, alkyls, alkenyls and/or aryls that may be either p- or s-coordinated. These metal complexes may be in the free form or fixed on substrates, typically on activated magnesium chloride, titanium(III) chloride, alumina or silicon oxide. These catalysts may be soluble or insoluble in the polymerisation medium. The catalysts can be used by themselves in the polymerisation or further activators may be used, typically metal alkyls, metal hydrides, metal alkyl halides, metal alkyl oxides or metal alkyloxanes, said metals being elements of groups la, IIa and/or IIIa of the Periodic Table. The activators may be modified conveniently with further ester, ether, amine or silyl ether groups. These catalyst systems are usually termed Phillips, Standard Oil Indiana, Ziegler (-Natta), TNZ (DuPont), metallocene or single site catalysts (SSC);
(C) Mixtures of the polymers mentioned under (A), for example mixtures of polypropylene with polyisobutylene, polypropylene with polyethylene (for example PP/HDPE, PP/LDPE) and mixtures of different types of polyethylene (for example LDPE/HDPE); and
(D) Copolymers of monoolefins and diolefins with each other or with other vinyl monomers, for example ethylene/propylene copolymers, linear low density polyethylene (LLDPE) and mixtures thereof with low density polyethylene (LDPE), propylene/but-1-ene copolymers, propylene/isobutylene copolymers, ethylene/but-1-ene copolymers, ethylene/hexene copolymers, ethylene/methylpentene copolymers, ethylene/heptene copolymers, ethylene/octene copolymers, propylene/butadiene copolymers, isobutylene/isoprene copolymers, ethylene/alkyl acrylate copolymers, ethylene/alkyl methacrylate copolymers, ethylene/vinyl acetate copolymers and their copolymers with carbon monoxide or ethylene/acrylic acid copolymers and their salts (ionomers) as well as terpolymers of ethylene with propylene and a diene such as hexadiene, dicyclopentadiene or ethylidene-norbornene; and mixtures of such copolymers with one another and with polymers mentioned in (A) above, for example polypropylene/ethylene-propylene copolymers, LDPE/ethylene-vinyl acetate copolymers (EVA), LDPE/ethylene-acrylic acid copolymers (EAA), LLDPE/EVA, LLDPE/EAA and alternating or random polyalkylene/carbon monoxide copolymers and mixtures thereof with other polymers, for example polyamides.

The stabilizer compositions are also contemplated for use in various industrial molding processes to produce stabilized molded articles. Accordingly, in another aspect the invention provides a use of a stabilizer composition, as defined in the claims, in processes for producing a molded article by adding a polymeric organic material and a polymer-stabilizing amount of a stabilizer composition as described and claimed herein to an industrial molding apparatus or device, or otherwise within an industrial molding process, and cycling the stabilized polymeric material through the apparatus/device and thereby the molding process.

Those of skill in the art will appreciate that the compositions and processes are suitable for use with, and readily adapted to, any industrial molding process including, but not limited to, injection molding, rotomolding, blow molding, reel-to-reel molding, metal injection molding, compression molding, transfer molding, dip molding, gas assist molding, insert injection molding, micro molding, reaction injection molding, two shot injection molding, as well as any variations or combinations thereof.

In certain embodiments of the processes described herein, the stabilizer composition is present at from 0.001 % to 65.0 % by weight of the total weight of the organic material to be stabilized; preferably at from 0.01 % to 25 %; and more preferably at from 0.01 % to 10 % by weight of the total weight of the organic material to be stabilized.

It is also contemplated that the components of the stabilizer compositions and/or an organic material to be stabilized described herein may be contained in a kit. The kit may include single or multiple components of at least one stabilizer composition, at least one material to be stabilized (e.g., a polymer composition such as a polyolefin), and at least one further optional additive, each packaged or formulated individually, or single or multiple components of at least one stabilizer composition, at least one material to be stabilized, and at least one further optional additive packaged or formulated in combination. Thus, one or more components of a stabilizer composition can be present in first container, and the kit can optionally include one or more components of the stabilizer composition and/or material to be stabilized in a second or further container. The container or containers are placed within a package, and the package can optionally include administration or mixing instructions in the form of a label or website address on the package, or in the form of an insert included in the packaging of the kit. A kit can include additional components or other means for administering or mixing the components as well as solvents or other means for formulation.

### Examples

The following examples are provided to assist one skilled in the art to further understand certain embodiments of the present invention. These examples are intended for illustration purposes and are not to be construed as limiting the scope of the various embodiments of the present invention.

As a fluidity scale, melt index (hereinafter also referred to as MI) is industrially used predominantly for anticipating processability of an organic polymer material or for indication of standard and quality control. MI shows a flow rate in weight (unit: g) for 10 min when a high molecular weight polymer melted at a given temperature is extruded from a circular die having standard length and diameter by applying a certain load, and is used as an index of melt viscosity. Of the high molecular weight polymers, polypropylene having a lower MI value has better stability during processing, and that having a greater MI value has poor stability during processing. Polymer showing less variation in the values upon repeat measurements of MI is considered to have greater MI retention effect and to be superior in the stability during processing.

When an additive is kneaded into an organic polymer material (such as by multi-pass extrusion), the yellowness index (hereinafter also referred to as YI) is also widely used as a scale for evaluating discoloration of the polymer material. Yellowness index (YI) is measured by colorimeter, wherein a greater value means greater discoloration or color development and a smaller value means less coloring during processing, and therefore, superiority.

The superior advantages and unexpected properties provided by the stabilizer compositions and processes according to the present invention are now revealed by the following Examples taken in conjunction with the Figures.

### Example 1

The rheological property of an organic polymer composition stabilized with a stabilizer composition according to the present invention is tested by multiple-pass extrusion.

FIG. 1A: A stabilizer composition in the form of vitamin E acetate (0.15 %) together with zinc stearate (0.05 %) is dry blended with Profax 6301 polypropylene homopolymer (available from LyondellBasell Industries) (B), and compared to the polypropylene without any stabilizer additive composition (A). A Killion single screw extruder is set with a 60 mesh screen, 280 °C melt temperature, and a screw speed of 100 RPM. The blends are extruded 5 times (extruded and re-extruded 4 more times). Samples are collected after each pass and the melt-index determined. Samples from each pass are collected and the melt flow index is tested according to ASTM D1238-10 on a Dynisco melt indexer.

As indicated by the corresponding Figure 1A, the results show that the polypropylene sample containing no stabilizer composition (A) could only be processed two times, whereas the polypropylene material blended with 0.15 % vitamin E acetate was extruded 4 times.

FIG. 1B: Polypropylene samples are prepared as described above and multi-pass extruded. (A): no stabilizing additive; (B): 0.15 % vitamin E acetate; (C): 0.15 % vitamin E; (D): 0.075 % vitamin E and 0.075 % vitamin E acetate. All samples containing stabilizing additive are also blended with 0.05 % zinc stearate. As indicated by Figure 1B, sample D shows better than expected performance based on the comparison samples A, B, or C.

### Example 2

The mechanical properties of the organic materials (as determined by % elongation at break) are measured to see how the organic materials are affected by the processing. Two formulations are dry blended and compounded as in Example 1 with either no stabilizer additive (B) or vitamin E acetate at 0.15 % together with 0.05 % zinc stearate (C). The compounded material is extruded and then collected and compression molded into 1/16" thick sheets. A sample of unprocessed polypropylene (A) is also compression molded in a 1/16" sheet. Tensile samples are cut into dog-bone shaped specimens of type 5 (ASTM D638) using a punch press. Samples are tested on an MTS tensile tester at 2"/min crosshead speed. The median value of the five samples is used for all analyses. Using the unprocessed polypropylene as the baseline control, the percentage elongation retention of the polypropylene sample with and without a stabilizer composition is calculated.

As indicated by corresponding Figure 2, sample (C) shows significant improvement in elongation retention compared to sample (B), as compared to unprocessed polypropylene resin (A).

### Example 3

The Yellowness Index of organic polymer compositions with or without stabilizer compositions is measured for evaluating discoloration. Samples are prepared by dry blending stabilizing additives of the prior art or those according to the present invention at equal concentrations with low density polyethylene. Compounding is performed using a Killion single screw extruder set with a 60 mesh screen, 250 °C melt temperature, and a screw speed of 100 RPM. The samples are re-extruded 4 additional times (total of five). Samples of the compounded material are collected at each pass. Yellowness Index (ASTM E313) is determined using a Greta Macbeth Color i7, spectrophotometer. (A): no stabilizing additive; (B) 0.1 % IRGANOX ^{®} 1010 phenolic antioxidant (available from BASF); (C): 0.1 % CYANOX^{®} 1790 phenolic antioxidant (available from Cytec Industries Inc.); (D) 0.1 % vitamin E acetate. All samples containing stabilizing additive also contain 0.05 % zinc stearate.

As indicated by corresponding Figure 3, the Yellowness Index shows that samples (B) and (C) are more yellow *(i.e.,* impart more color to the polyethylene) than (D), and that the delta for samples (B) and (C) significantly increases at the 3^{rd} and 5^{th} passes. The Yellowness Index for control sample (A) (no stabilizing additive) remains low because there is no stabilizing additive imparting color. Those of skill in the art would further appreciate and expect that the Yellowness Index for a polyethylene sample stabilized with only vitamin E would be even greater than samples (B) and (C), as it is known in the art that vitamin E badly discolors polymer substrates and that phenolic antioxidants such as IRGANOX^{®} 1010 and CYANOX^{®} 1790 reduce the undesired discoloring imparted by using vitamin E as a stabilizing additive.

Accordingly, the stabilizer compositions and processes according to the present invention provide unexpected and superior advantages to those stabilizer systems currently known and used. Taken together, these experiments show that organic materials stabilized with stabilizer systems have improved mechanical and rheological properties, and that the stabilizer compositions do not impart unfavorable color characteristics to the material to which it is added.

Various patent and/or scientific literature references have been referred to throughout this application. In view of the above description and the examples, one of ordinary skill in the art will be able to practice the disclosure as claimed without undue experimentation.

Although the foregoing description has shown, described, and pointed out the fundamental novel features of the present teachings, it will be understood that various omissions, substitutions, and changes in the form of the detail of the apparatus as illustrated, as well as the uses thereof, may be made by those skilled in the art, without departing from the scope of the present teachings. Consequently, the scope of the present teachings should not be limited to the foregoing discussion, but should be defined by the appended claims.

## Claims

1. Use of a stabilizer composition comprising a stabilizing amount of vitamin E acetate in an organic polymer material for improving the rheological property of said organic polymer material, wherein the improved rheological property is measured by less variation in the values of melt index of said organic polymer material upon repeat measurements thereof in multiple-pass extrusion testing.

2. A use according to claim 1 wherein said stabilizer composition is added before or during processing of said organic material.

3. A use according to claim 1 or 2 wherein said use is for the purpose of improving the rheological property of said organic polymer material in a process for producing a molded article, wherein said stabilizer composition is added before or during processing of said organic material.

4. A use according to claim 3 wherein said process for producing a molded article is selected from injection molding, rotomolding, blow molding, reel-to-reel molding, metal injection molding, compression molding, transfer molding, dip molding, gas assist molding, insert injection molding, micro molding, reaction injection molding, and two shot injection molding;
for example wherein said process for producing a molded article is a rotomolding process.

5. A use according to any one of claims 1 to 4, wherein the organic material is selected from the group consisting of polyolefins, polyesters, polyethers, polyketones, polyamides, natural and synthetic rubbers, polyurethanes, polystyrenes, polyacrylates, polymethacrylates, polyacetals, polyacrylonitriles, polybutadienes, acrylonitrile-butadiene-styrene, styrene acrylonitrile, acrylate styrene acrylonitrile, cellulosic acetate butyrate, cellulosic polymers, polyimides, polyamideimides, polyetherimides, polyphenylsulfides, polyphenyloxidepolysulfones, polyethersulfones, polyvinylchlorides, polycarbonates, aliphatic polyketones, thermoplastic olefins, aminoresin cross-linked polyacrylates and polyesters, polyisocyanate cross-linked polyesters and polyacrylates, phenol/formaldehyde, urea/formaldehyde and melamine/formaldehyde resins, alkyd resins, polyester resins, acrylate resins cross-linked with melamine resins, urea resins, isocyanates, isocyanurates, carbamates, epoxy resins, cross-linked epoxy resins derived from aliphatic, cycloaliphatic, heterocyclic and aromatic glycidyl compounds, which are cross-linked with anhydrides or amines, polysiloxanes, Michael addition polymers, amines, blocked amines with activated unsaturated and methylene compounds, ketimines with activated unsaturated and methylene compounds, polyketimines in combination with unsaturated acrylic polyacetoacetate resins, polyketimines in combination with unsaturated acrylic resins, radiation curable compositions, epoxymelamine resins, organic dyes, cosmetic products, cellulose-based paper formulations, photographic film paper, fibers, waxes, and inks;
for example wherein the organic material is a polyolefin polymer is selected from the group consisting of i) polymers of monoolefins chosen from polypropylene, polyisobutylene, polybut-1-ene, poly-4-methylpent-1-ene; ii) diolefins chosen from polyisoprene or polybutadiene; iii) polymers of cycloolefins chosen from cyclopentene, and norbornene; iv) polyethylene chosen from optionally crosslinked polyethylene, high density polyethylene (HDPE), high density and high molecular weight polyethylene (HDPE-HMW), high density and ultrahigh molecular weight polyethylene (HDPE-UHMW), medium density polyethylene (MDPE), low density polyethylene (LDPE), linear low density polyethylene (LLDPE), very low density polyethylene (VLDPE), and ultralow density polyethylene (ULDPE); v) copolymers thereof; and vi) mixtures thereof.

6. A use according to any one of claims 1 to 5, wherein the stabilizer composition further comprises at least one organic phosphite or phosphonite selected from the group consisting of a compound according to Formulas 1-7: 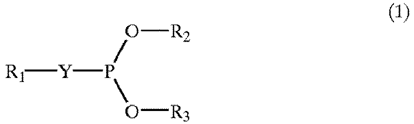 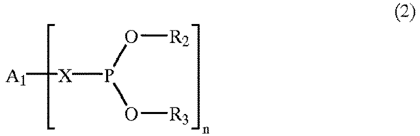 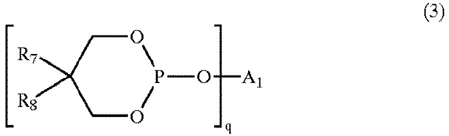 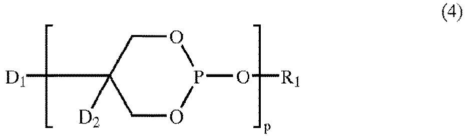 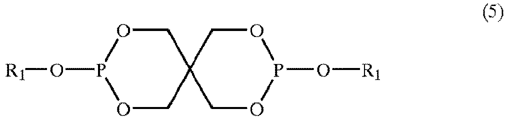 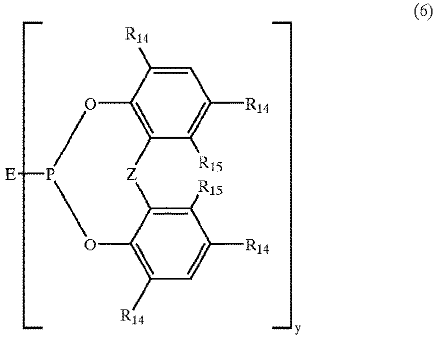 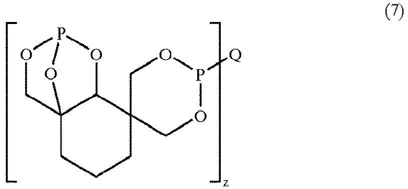
in which the indices are integral and
n is 2, 3 or 4; p is 1 or 2; q is 2 or 3; r is 4 to 12; y is 1, 2 or 3; and z is 1 to 6;
A₁, if n is 2, is C₂-C₁₈ alkylene; C₂-C₁₂ alkylene interrupted by oxygen, sulfur or -NR₄-; a radical of the formula 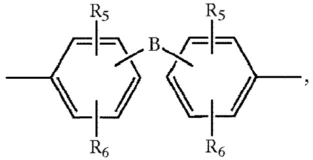 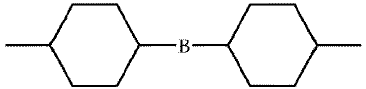
or phenylene;
A₁, if n is 3, is a radical of the formula -CᵣH₂ᵣ₋₁-;
A₁, if n is 4, is 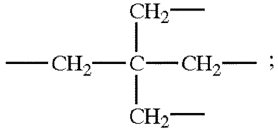
A₂ is as defined for A₁ if n is 2;
B is a direct bond, -CH₂-, -CHR₄-, -CR₁R₄-, sulfur, C₅-C₇ cycloalkylidene, or
cyclohexylidene which is substituted by from 1 to 4 C₁-C₄ alkyl radicals in position 3, 4 and/or 5;
D₁, if p is 1, is C₁-C₄ alkyl and, if p is 2, is -CH₂OCH₂-;
D₂, if p is 1, is C₁-C₄ alkyl;
E, if y is 1, is C₁-C₁₈ alkyl, -OR₁ or halogen;
E, if y is 2, is -O-A₂-O-,
E, if y is 3, is a radical of the formula R₄C(CH₂O-)₃ or N(CH₂CH₂O-)₃;
Q is the radical of an at least z-valent alcohol or phenol, this radical being attached via the oxygen atom to the phosphorus atom;
R₁, R₂ and R₃ independently of one another are C₁-C₁₈ alkyl which is unsubstituted or
substituted by halogen, -COOR₄, -CN or -CONR₄R₄; C₂-C₁₈ alkyl interrupted by oxygen, sulfur or -NR₄-; C₇-C₉ phenylalkyl; C₅-C₁₂ cycloalkyl, phenyl or naphthyl;
naphthyl or phenyl substituted by halogen, 1 to 3 alkyl radicals or alkoxy radicals having
a total of 1 to 18 carbon atoms or by C₇-C₉ phenylalkyl; or a radical of the formula 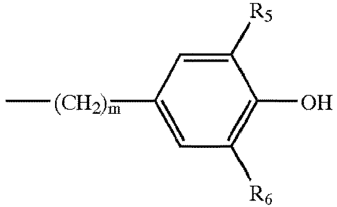
in which m is an integer from the range 3 to 6;
R₄ is hydrogen, C₁-C₈ alkyl, C₅-C₁₂ cycloalkyl or C₇-C₉ phenylalkyl,
R₅ and R₆ independently of one another are hydrogen, C₁-C₈ alkyl or C₅-C₆ cycloalkyl,
R₇ and R₈, if q is 2, independently of one another are C₁-C₄ alkyl or together are a 2,3-dehydropentamethylene radical; and
R₇ and R₈, if q is 3, are methyl;
R₁₄ is hydrogen, C₁-C₉ alkyl or cyclohexyl,
R₁₅ is hydrogen or methyl and, if two or more radicals R₁₄ and R₁₅ are present, these radicals are identical or different,
X and Y are each a direct bond or oxygen,
Z is a direct bond, methylene, -C(R₁₆)₂- or sulfur, and
R₁₆ is C₁-C₈ alkyl;
a trisarylphosphite according to Formula 8: 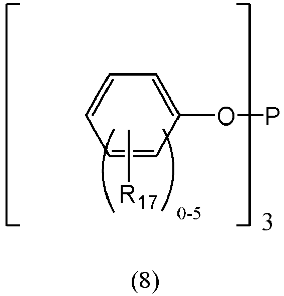 wherein R₁₇ is a substituent that is the same or different at from 0 to 5 positions of the aromatic portion of Formula 8 and is independently chosen from a member selected from the group consisting of C₁-C₂₀ alkyl, C₃-C₂₀ cycloalkyl, C₄-C₂₀ alkyl cycloalkyl, C₆-C₁₀ aryl, and C₇-C₂₀ alkylaryl; and combinations thereof;
for example wherein the organic phosphite or phosphonite is selected from the group consisting of triphenyl phosphite; diphenyl alkyl phosphites; phenyl dialkyl phosphites; trilauryl phosphite; trioctadecyl phosphite; distearyl pentaerythritol phosphite; tris(2,4-di-tert-butylphenyl) phosphite; tris(nonylphenyl) phosphite; a compound of formulae (A), (B), (C), (D), (E), (F), (G), (H), (J), (K) and (L): 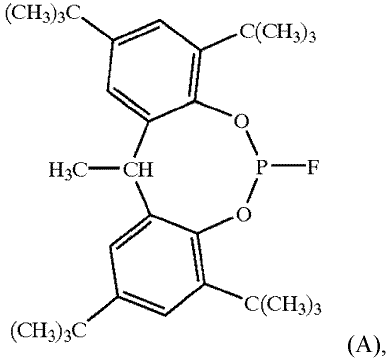 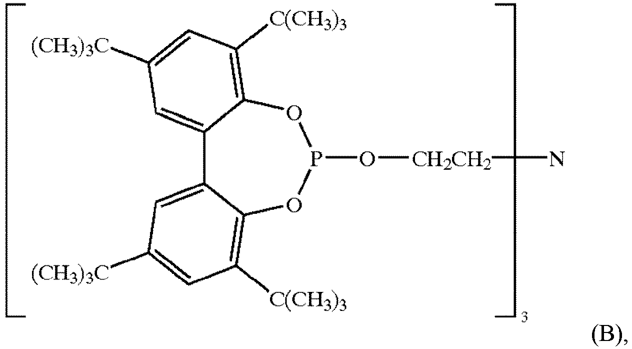 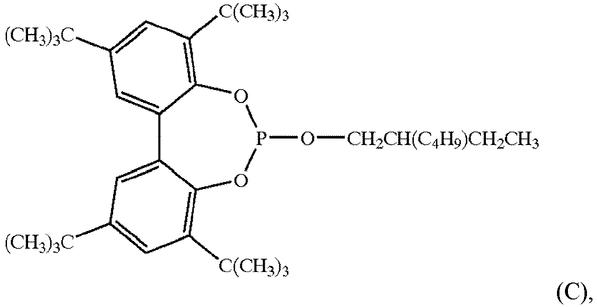 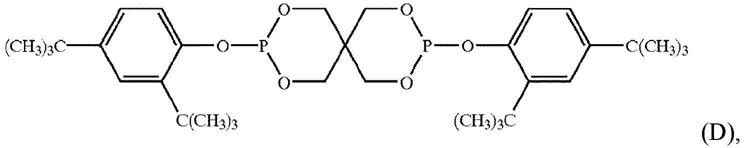 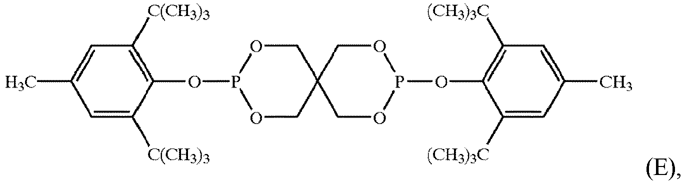 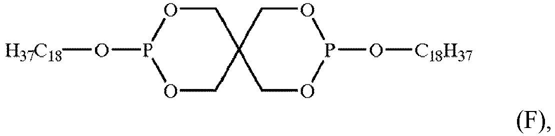 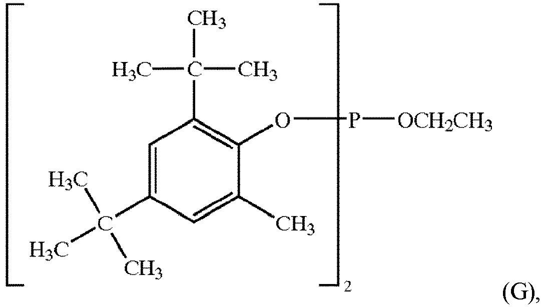 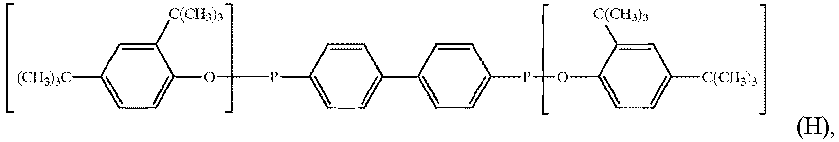 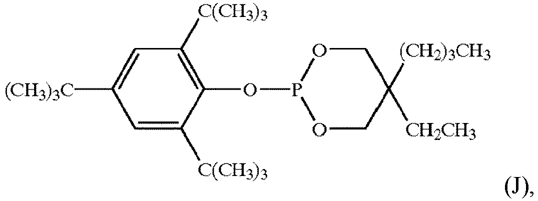 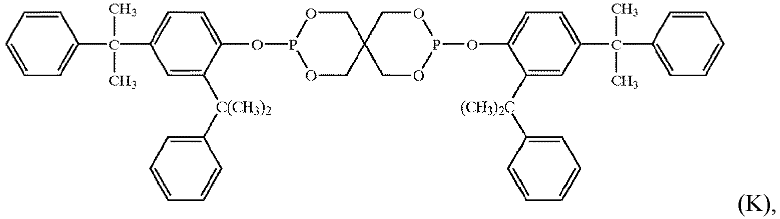 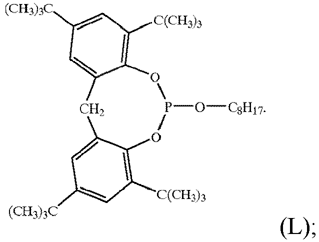 and 2-butyl-2-ethyl-1,3-propanediol 2,4,6-tri-t-butylphenol phosphite, bis-(2,6-di-t-butyl-4-methlphenyl) pentaerythritol diphosphite, 2-butyl-2-ethyl-1,3-propanediol 2,4-dicumylphenol phosphite, 2-butyl-2-ethyl-1,3-propanediol 4-methyl-2,6-di-t-butylphenol phosphite, and bis-(2,4,6-tri-t-butyl-phenyl) pentaerythritol diphosphite.

7. A use according to any one of the preceding claims, wherein the stabilizer composition further comprises at least one hindered phenol compound having a molecular fragment according to one or more of Formula (IVa), (IVb), or (IVc): 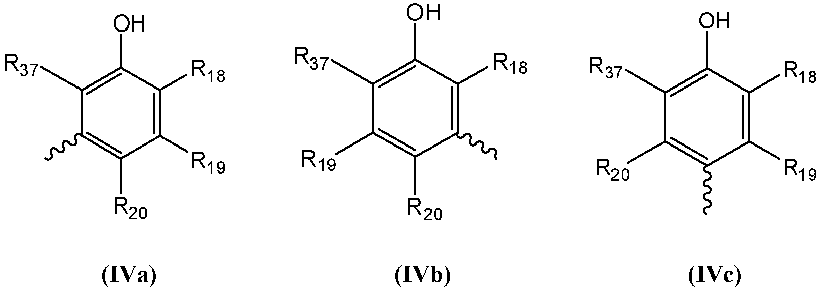 wherein
R₁₈ is chosen from hydrogen or a C₁₋₄ hydrocarbyl;
each of R₁₉ and R₂₀ is independently chosen from hydrogen or a C₁-C₂₀ hydrocarbyl; and
R₃₇ is chosen from a C₁-C₁₂ hydrocarbyl.

8. A use according to claim 7, wherein the at least one hindered phenol compound is selected from the group consisting of (1,3,5-Tris(4-t-butyl-3-hydroxy-2,6-dimethylbenzyl)-1,3,5-triazine-2,4,6-(1H,3H,5H)-trione; 1,1,3-Tris(2'-methyl-4'-hydroxy-5'-*t*-butylphenyl)butane; Triethylene glycol bis[3-(3-*t*-butyl-4-hydroxy-5-methylphenyl)propionate]; 4,4'-Thiobis(2-*t*-butyl-5-methylphenol); 2,2'-Thiodiethylene bis[3-(3-*t*-butyl-4-hydroxyl-5-methylphenyl)propionate]; Octadecyl 3-(3'-*t*-butyl-4'-hydroxy-5'-methylphenyl)propionate; Tetrakismethylene(3-*t*-butyl-4-hydroxy-5-methylhydrocinnamate)methane; *N*,*N*'-Hexamethylene bis[3-(3-*t*-butyl-4-hydroxy-5-methylphenyl)propionamide]; Di(4-tertiarybutyl-3-hydroxy-2,6-dimethyl benzyl) thiodipropionate; and octadecyl 3,5-di-(tert)-butyl-4-hydroxyhydrocinnamate.

9. A use according to any one of the proceeding claims,
wherein the stabilizer composition further comprises at least one compound selected from the group consisting of α-tocopherol, β-tocopherol, γ-tocopherol, δ-tocopherol, related tocotrienols, and mixtures thereof; and/or
wherein the vitamin E acetate is present from 0.001 % to 5.0 % by weight of the total weight of the stabilizer composition; or from 0.01 % to 1.0 % by weight of the total weight of the stabilizer composition.

10. A use according to any one of the preceding claims, wherein the stabilizer composition further comprises an effective amount of a light stabilizer selected from the group consisting of hindered amine light stabilizers, hindered hydroxyl benzoates, nickel phenolates, ultraviolet light stabilizers, and combinations thereof

11. A use according to claim 10, wherein the light stabilizer is a hindered amine light stabilizer compound comprising a molecular fragment according to Formula (VI): 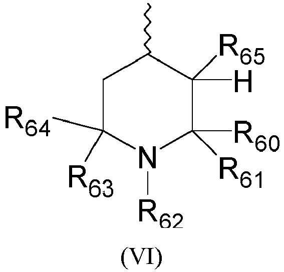 wherein
R₆₂ is chosen from a member selected from the group consisting of hydrogen; OH; C₁-C₂₀ hydrocarbyl; -CH₂CN; C₁-C₁₂ acyl; and C₁-C₁₈ alkoxy;
R₆₅ is chosen from a member selected from the group consisting of hydrogen; and C₁-C₈ hydrocarbyl; and
each of R₆₀, R₆₁, R₆₃, and R₆₄ is independently chosen from a C₁-C₂₀ hydrocarbyl, or R₆₀ and R₆₁ and/or R₆₃ and R₆₄ taken together with the carbon to which they are attached form a C₅-C₁₀ cycloalkyl;
or Formula (VIa) 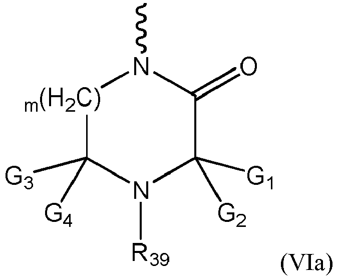 wherein
m is an integer from 1 to 2;
R₃₉ is chosen from a member selected from the group consisting of hydrogen; OH; C₁-C₂₀ hydrocarbyl; -CH₂CN; C₁-C₁₂ acyl; and C₁-C₁₈ alkoxy; and
each of G₁-G₄ is independently chosen from a C₁-C₂₀ hydrocarbyl; and/or
wherein the hindered amine light stabilizer is selected from the group consisting of bis(2,2,6,6-tetramethylpiperidin-4-yl) sebacate; bis(2,2,6,6-tetramethylpiperidin-4-yl)succinate; bis(1,2,2,6,6-pentamethylpiperidin-4-yl)sebacate; bis(1-octyloxy-2,2,6,6-tetramethylpiperidin-4-yl)sebacate; bis(1,2,2,6,6-pentamethylpiperidin-4-yl) n-butyl 3,5-di-tert-butyl-4-hydroxybenzylmalonate; a condensate of 1-(2-hydroxyethyl)-2,2,6,6-tetramethyl-4-hydroxypiperidine and succinic acid; 2,2,6,6-tetramethylpiperidin-4-yl stearate; 2,2,6,6-tetramethylpiperidin-4-yl dodecanate; 1,2,2,6,6-pentamethylpiperidin-4-yl stearate; 1,2,2,6,6-pentamethylpiperidin-4-yl dodecanate; a condensate of N,N'-bis(2,2,6,6-tetramethylpiperidin-4-yl)hexamethylenediamine and 4-tert-octylamino-2,6-dichloro-1,3,5-triazine; tris(2,2,6,6-tetramethylpiperidin-4-yl) nitrilotriacetate; tetrakis(2,2,6,6-tetramethylpiperidin-4-yl)- 1,2,3,4-butanetetracarboxylate; 4-benzoyl-2,2,6,6-tetramethylpiperidine; 4-stearyloxy-2,2,6,6-tetramethylpiperidine; bis(1,2,2,6,6-pentamethylpiperidyl)-2-n-butyl-2-(2-hydroxy-3,5-di-tert-butylbenzyl)malonate; 3-n-octyl-7,7,9,9-tetramethyl-1,3,8-triazaspiro[4.5]decan-2,4-dione; bis(1-octyloxy-2,2,6,6-tetramethylpiperidyl)sebacate; bis(1-octyloxy-2,2,6,6-tetramethylpiperidyl)succinate; a condensate of N,N'-bis(2,2,6,6-tetramethylpiperidin-4-yl)hexamethylenediamine and 4-morpholino-2,6-dichloro-1,3,5-triazine; a condensate of 2-chloro-4,6-bis(4-n-butylamino-2,2,6,6-tetramethylpiperidyl)-1,3,5-triazine and 1,2-bis(3-aminopropylamino)ethane; a condensate of 2-chloro-4,6-bis(4-n-butylamino-1,2,2,6,6-pentamethylpiperidyl)-1,3,5-triazine and 1,2-bis-(3- aminopropylamino)ethane; 8-acetyl-3-dodecyl-7,7,9,9-tetramethyl-1,3,8-triazaspiro[4.5]decane-2,4-dione; 3-dodecyl-1-(2,2,6,6-tetramethylpiperidin-4-yl)pyrrolidin-2,5-dione; 3-dodecyl-1-(1 -ethanoyl-2,2,6,6-tetramethylpiperidin-4-yl)pyrrolidin-2,5-dione; 3-dodecyl-1-(1,2,2,6,6-pentamethylpiperidin-4-yl)pyrrolidine-2,5-dione; a mixture of 4-hexadecyloxy- and 4-stearyloxy-2,2,6,6-tetramethylpiperidine; a condensate of N,N'-bis(2,2,6,6-tetramethylpiperidin-4-yl)hexamethylenediamine and 4-cyclohexylamino-2,6-dichloro-1,3,5-triazine; a condensate of 1,2-bis(3-aminopropylamino)ethane, 2,4,6-trichloro-1,3,5-triazine and 4-butylamino-2,2,6,6-tetramethylpiperidine; 2-undecyl-7,7,9,9-tetramethyl-1-oxa-3,8-diaza-4-oxospiro[4.5]decane; oxo-piperanzinyl-triazines; a reaction product of 7,7,9,9-tetramethyl-2-cycloundecyl-1-oxa-3,8-diaza-4-oxospiro[4.5]decane and epichlorohydrin; tetrakis(2,2,6,6-tetramethyl-4-piperidyl) butane-1,2,3,4-tetracarboxylate; 1,2,3,4-butanetetracarboxylic acid, tetrakis(1,2,2,6,6-pentamethyl-4-piperidinyl)ester; 1,2,3,4-butanetetracarboxylic acid, 1,2,2,6,6-pentamethyl-4-piperdinyl tridecyl ester; 1,2,3,4-butanetetracarboxylic acid, 2,2,6,6-tetramethyl-4-piperidinyl tridecyl ester; 1,2,3,4-butanetetracarboxylic acid, polymer with 2,2,6,6-tetramethyl-2,4,8,10-tetraoxaspiro[5.5]-undecane-3,9-diethanol,1,2,2,6,6-pentamethyl-4-piperdinyl ester; 1,2,3,4-butanetetracarboxylic acid, polymer with 2,2,6,6-tetramethyl-2,4,8,10-tetraoxaspiro[5.5]-undecane-3,9-diethanol, 2,2,6,6-tetramethyl-4-piperdinyl ester; bis(1-undecanoxy-2,2,6,6-tetramethylpiperidin-4-yl)carbonate; 1-(2-hydroxy-2-methylpropoxy)-2,2,6,6-tetramethyl-4-piperdinol; 1-(2-hydroxy-2-methylpropoxy)-4-octadecanoyloxy-2,2,6,6-tetramethylpiperidine; 1-(4-octadecanoyloxy-2,2,6,6-tetramethylpiperidin-1-yloxy)-2-octadecanoyloxy-2-methylpropane; 1-(2-hydroxyethyl)-2,2,6,6-tetramethyl-4-piperdinol; a reaction product of 1-(2-hydroxyethyl)-2,2,6,6-tetramethyl-4-piperdinol and dimethylsuccinate; 2,2,4,4-tetramethyl-7-oxa-3,20-diazadispiro[5.1.11.2]heneicosan-21-one; the ester of 2,2,6,6-tetramethyl-4-piperidinol with higher fatty acids; 3-dodecyl-1-(2,2,6,6-tetramethyl-4-piperidyl)pyrrolidine-2,5-dione; 1H-Pyrrole-2,5-dione, 1-octadecyl-, polymer with (1-methylethenyl)benzene and 1-(2,2,6,6-tetramethyl-4-piperidinyl)-1H-pyrrole-2,5-dione; piperazinone, 1,1',1"-[1,3,5-triazine-2,4,6-triyltris[(cyclohexylimino)-2,1-ethanediyl]]tris[3,3,5,5-tetramethyl-; piperazinone, 1,1',1"-[1,3,5-triazine-2,4,6-triyltris[(cyclohexylimino)-2,1-ethanediyl]]tris[3,3,4,5,5-pentamethyl-; the reaction product of 7,7,9,9-tetramethyl-2-cycloundecyl-1-oxa-3,8-diaza-4-oxospiro[4.5]decane and epichlorohydrin; the condensate of N,N'-bis(2,2,6,6-tetramethylpiperidin-4-yl)hexamethylenediamine and 4-cyclohexylamino-2,6-dichloro-1,3,5-triazine; the condensate of 1,2-bis(3-aminopropylamino)ethane, 2,4,6-trichloro-1,3,5-triazine and 4-butylamino-2,2,6,6-tetramethylpiperidine; the condensate of N,N'-bis(2,2,6,6-tetramethylpiperidin-4-yl)hexamethylenediamine and 4-morpholino-2,6-dichloro-1,3,5-triazine; the condensate of 2-chloro-4,6-bis(4-n-butylamino-2,2,6,6-tetramethylpiperidyl)-1,3,5-triazine and 1,2-bis(3-aminopropylamino)ethane; the condensate of 2-chloro-4,6-bis(4-n-butylamino-1,2,2,6,6-pentamethylpiperidyl)-1,3,5-triazine and 1,2-bis-(3-aminopropylamino)ethane; 2-[(2-hydroxyethyl)amino]-4,6-bis[N-(1-cyclohexyloxy-2,2,6,6-tetramethylpiperidin-4-yl)butylamino-1,3,5-triazine; propanedioic acid, [(4-methoxyphenyl)-methylene]-bis-(1,2,2,6,6-pentamethyl-4-piperidinyl) ester; tetrakis(2,2,6,6-tetramethylpiperidin-4-yl)-1,2,3,4-butanetetracarboxylate; benzenepropanoic acid, 3,5-bis(1,1-dimethylethyl)-4-hydroxy-, 1-[2-[3-[3,5-bis(1,1-dimethylethyl)-4-hydroxyphenyl]-1-oxopropoxy]ethyl]-2,2,6,6-tetramethyl-4-piperidinyl ester; N-(1-octyloxy-2,2,6,6-tetramethylpiperidin-4-yl)-N'-dodecyloxalamide; tris(2,2,6,6-tetramethylpiperidin-4-yl) nitrilotriacetate; 1,5-dioxaspiro{5,5}undecane-3,3-dicarboxylic acid, bis(1,2,2,6,6-pentamethyl-4-piperidinyl): 1,5-dioxaspiro{5,5}undecane-3,3-dicarboxylic acid, bis(2,2,6,6-tetramethyl-4-piperidinyl); the condensate of 1-(2-hydroxyethyl)-2,2,6,6-tetramethyl-4-hydroxypiperidine and succinic acid; the condensate of N,N'-bis(2,2,6,6-tetramethylpiperidin-4-yl)hexamethylenediamine and 4-tert-octylamino-2,6-dichloro-1,3,5-triazine; 1,2,3,4-butanetetracarboxylic acid, 1,2,2,6,6-pentamethyl-4-piperidinyl tridecyl ester; tetrakis(2,2,6,6-tetramethylpiperidin-4-yl)-1,2,3,4-butanetetracarboxylate; 1,2,3,4-butanetetracarboxylic acid, 2,2,6,6-tetramethyl-4-piperidinyl tridecyl ester; tetrakis(1,2,2,6,6-pentamethylpiperidin-4-yl)-1,2,3,4-butanetetracarboxylate; mixture of 2,2,4,4-tetramethyl-21-oxo-7-oxa-3.20-diazaspiro(5.1.11.2)-heneicosane-20-propanoic acid-dodecylester and 2,2,4,4-tetramethyl-21-oxo-7-oxa-3.20-diazaspiro(5.1.11.2)-heneicosane-20-propanoic acid-tetradecylester; 1H,4H,5H,8H-2,3a,4a,6,7a,8a-hexaazacyclopenta[def]fluorene-4,8-dione, hexahydro-2,6-bis(2,2,6,6-tetramethyl-4-piperidinyl)-; polymethyl[propyl-3-oxy(2',2',6',6'-tetramethyl-4,4'-piperidinyl)]siloxane; polymethyl[propyl-3-oxy(1',2',2',6',6'-pentamethyl-4,4'-piperidinyl)]siloxane; copolymer of methylmethacrylate with ethyl acrylate and 2,2,6,6-tetramethylpiperidin-4-yl acrylate; copolymer of mixed C₂₀ to C₂₄ alpha-olefins and (2,2,6,6-tetramethylpiperidin-4-yl)succinimide; 1,2,3,4-butanetetracarboxylic acid, polymer with β,β,β',β'-tetramethyl-2,4,8,10-tetraoxaspiro[5.5]undecane-3,9-diethanol, 1,2,2,6,6-pentamethyl-4-piperidinyl ester; 1,2,3,4-butanetetracarboxylic acid, polymer with β,β,β',β'-tetramethyl-2,4,8,10-tetraoxaspiro[5.5]undecane-3,9-diethanol, 2,2,6,6-tetramethyl-4-piperidinyl ester copolymer; 1,3-benzenedicarboxamide, N,N'-bis(2,2,6,6-tetramethyl-4-piperidinyl; 1,1'-(1,10-dioxo-1,10-decanediyl)-bis(hexahydro-2,2,4,4,6-pentamethylpyrimidine; ethane diamide, N-(1-acetyl-2,2,6,6-tetramethylpiperidinyl)-N'-dodecyl; formamide, N,N'-1,6-hexanediylbis[N-(2,2,6,6-tetramethyl-4-piperidinyl); D-glucitol, 1,3:2,4-bis-O-(2,2,6,6-tetramethyl-4-piperidinylidene)-; 2,2,4,4-tetramethyl-7-oxa-3,20-diaza-21-oxo-dispiro[5.1.11.2]heneicosane; propanamide, 2-methyl-N-(2,2,6,6-tetramethyl-4-piperidinyl)-2-[(2,2,6,6-tetramethyl-4-piperidinyl)amino]-; 7-oxa-3,20-diazadispiro[5.1.11.2]heneicosane-20-propanoic acid, 2,2,4,4-tetramethyl-21-oxo-, dodecyl ester; N-(2,2,6,6-tetramethylpiperidin-4-yl)-β-aminopropionic acid dodecyl ester; N-(2,2,6,6-tetramethylpiperidin-4-yl)-N'-aminooxalamide; propanamide, N-(2,2,6,6-tetramethyl-4-piperidinyl)-3-[(2,2,6,6-tetramethyl-4-piperidinyl)amino]-; mixture of 4-hexadecyloxy- and 4-stearyloxy-2,2,6,6-tetramethylpiperidine; 3-dodecyl-1-(1,2,2,6,6-pentamethylpiperidin-4-yl)pyrrolidine-2,5-dione; 3-dodecyl-1-(1-ethanoyl-2,2,6,6-pentamethylpiperidin-4-yl)pyrrolidine-2,5-dione; bis(2,2,6,6-tetramethylpiperidin-4-yl)succinate; bis(1,2,2,6,6-pentamethylpiperidin-4-yl) n-butyl 3,5-di-tert-butyl-4-hydroxybenzylmalonate; tris(2,2,6,6-tetramethylpiperidin-4-yl) nitrilotriacetate; 1,1'-(1,2-ethanediyl)bis(3,3,5,5-tetramethylpiperazinone); 4-benzoyl-2,2,6,6-tetramethylpiperidine; 4-stearyloxy-2,2,6,6-tetramethylpiperidine; bis(1,2,2,6,6-pentamethylpiperidyl)-2-n-butyl-2-(2-hydroxy-3,5-di-tert-butylbenzyl)malonate; 3-n-octyl-7,7,9,9-tetramethyl-1,3,8-triazaspiro[4.5]decan-2,4-dione; bis(1-octyloxy-2,2,6,6-tetramethylpiperidyl)sebacate; bis(1-octyloxy-2,2,6,6-tetramethylpiperidyl)succinate; 8-acetyl-3-dodecyl-7,7,9,9-tetramethyl-1,3,8-triazaspiro[4.5]decane-2,4-dione; 3-dodecyl-1-(2,2,6,6-tetramethylpiperidin-4-yl)pyrrolidin-2,5-dione; 3-dodecyl-1-(1-ethanoyl-2,2,6,6-tetramethylpiperidin-4-yl)pyrrolidin-2,5-dione; 3-dodecyl-1-(1,2,2,6,6-pentamethylpiperidin-4-yl)pyrrolidine-2,5-dione; a mixture of 4-hexadecyloxy- and 4-stearyloxy-2,2,6,6-tetramethylpiperidine; 2-undecyl-7,7,9,9-tetramethyl-1-oxa-3,8-diaza-4-oxospiro[4.5]decane; 1,5-dioxaspiro{5,5}undecane-3,3-dicarboxylic acid, bis(2,2,6,6-tetramethyl-4-piperidinyl) and 1,5-dioxaspiro{5,5}undecane-3,3-dicarboxylic acid, bis(1,2,2,6,6-pentamethyl-4-piperidinyl); N¹-(β-hydroxyethyl)3,3-pentamethylene-5,5-dimethylpiperazin-2-one; N¹-tert-octyl-3,3,5,5-tetramethyl-diazepin-2-one; N¹-tert-octyl-3,3-pentamethylene-5,5-hexamethylene-diazepin-2-one; N¹-tert-octyl-3,3-pentamethylene-5,5-dimethylpiperazin-2-one; trans-1,2-cyclohexane-bis-(N¹-5,5-dimethyl-3,3-pentamethylene-2-piperazinone; trans-1,2-cyclohexane-bis-(N¹-3,3,5,5-dispiropentamethylene-2-piperazinone); N¹-isopropyl-1,4-diazadispiro-(3,3,5,5)pentamethylene-2-piperazinone; N¹-isopropyl-1,4-diazadispiro-3,3-pentamethylene-5,5-tetramethylene-2-piperazinone; N¹-isopropyl-5,5-dimethyl-3,3-pentamethylene-2-piperazinone; trans-1,2-cyclohexane-bis-N¹-(dimethyl-3,3-pentamethylene-2-piperazinone); N¹-octyl-5,5-dimethyl-3,3-pentamethylene-1,4-diazepin-2-one; and N¹-octyl-1,4-diazadispiro-(3,3,5,5)pentamethylene-1,5-diazepin-2-one.

12. A use according to claim 10 or 11, wherein the light stabilizer comprises an ultraviolet light absorber selected from the group consisting of a 2-hydroxybenzophenone compound, a 2-(2'-hydroxyphenyl)benzotriazole compound, a 2-(2'-hydroxyphenyl)-1,3,5-triazine compound, and combinations thereof;
for example wherein the ultraviolet light absorber is a 2-(2'-hydroxyphenyl)-1,3,5-triazine compound selected from the group consisting of 4,6-bis-(2,4-dimethylphenyl)-2-(2-hydroxy-4-octyloxyphenyl)-s-triazine; 4,6-bis-(2,4-dimethylphenyl)-2-(2,4-- dihydroxyphenyl)-s-triazine; 2,4-bis(2,4-dihydroxyphenyl)-6-(4-chlorophenyl)-s-triazine; 2,4-bis[2-hydroxy-4-(2-hydroxy-ethoxy)phenyl]-6-(4-chlorophenyl)-s-triazine; 2,4-bis[2-hydroxy-4-(2-hydroxy-4-(2-hydroxy-ethoxy)phenyl]-6-(2,4-dimethylphenyl)-s-triazine; 2,4-bis[2-hydroxy-4-(2-hydroxyethoxy)phenyl]-6-(4-bromophenyl)-s-triazine; 2,4-bis[2-hydroxy-4-(2-acetoxyethoxy)phenyl]-6-(4-chlorophenyl)-s-triazine; 2,4-bis(2,4-dihydroxyphenyl)-6-(2,4-dimethylphenyl)-s-triazine; 2,4-bis(4-biphenylyl)-6-[2-hydroxy-4-[(octyloxycarbonyl)ethylideneoxy]phenyl]-s-triazine; 2,4-bis(4-biphenylyl)-6-[2-hydroxy-4-(2-ethylhexyloxy)phenyl]-s-triazine; 2-phenyl-4-[2-hydroxy-4-(3-sec-butyloxy-2-hydroxypropyloxy)phenyl]-6-[2-hydroxy-4-(3-sec-amyloxy-2-hydroxypropyloxy)phenyl]-s-triazine; 2,4-bis(2,4-dimethylphenyl)-6-[2-hydroxy-4(- 3-benzyloxy-2-hydroxypropyloxy)phenyl]-s-triazine; 2,4-bis(2-hydroxy-4-n-butyloxyphenyl)-6-(2,4-di-n-butyloxyphenyl)-s-triazine; 2,4-bis(2,4-dimethylphenyl)-6-[2-hydroxy-4-(3-nonyloxy-2-hydroxypropylox-y)-5-α-cumylphenyl]-s-triazine; methylenebis-{2,4-bis(2,4-dimethylphenyl)-6-[2-hydroxy-4-(3-butyloxy-2-hydroxypropoxy)phenyl]-s-triazine}; methylene bridged dimer mixture bridged in the 3:5', 5:5' and 3:3' positions in a 5:4:1 ratio; 2,4,6-tris(2-hydroxy-4-isooctyloxycarbonyliso-propylideneoxy-phenyl)-s-triazine; 2,4-bis(2,4-dimethylphenyl)-6-(2-hydroxy-4-hexyloxy-5-α-cumylphenyl)-s-triazine; 2-(2,4,6-trimethylphenyl)-4,6-bis[2-hydroxy-4-(3-butyloxy-2-hydroxypropyloxy)phenyl]-s-triazine; 2,4,6-tris[2-hydroxy-4-(3-sec-butyloxy-2-hydroxypropyloxy)-phenyl]-s-triazine; mixture of 4,6-bis-(2,4-dimethylphenyl)-2-(2-hydroxy-4-(3-dodecyloxy-2-hydroxypropoxy)phenyl)-s-triazine and 4,6-bis-(2,4-dimethylphenyl)-2-(2-hydroxy-4-(3-tridecyloxy-2-hydroxypropoxy)phenyl)-s-triazine; 4,6-bis-(2,4-dimethylphenyl)-2-(2-hydroxy-4(3-(2-ethylhexyloxy)-2-hydroxypropoxy)-phenyl)-s-triazine; 4,6-diphenyl-2-(4-hexyloxy-2-hydroxyphenyl)-s-triazine; and combinations thereof.

13. A use according to any one of the preceding claims, wherein the stabilizer composition further comprises at least one compound selected from the group consisting of:
a hydroxylamine compound according to Formula (VIII): 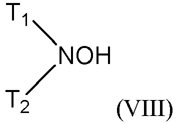 wherein
T₁ is chosen from a member selected from the group consisting of an optionally substituted C₁-C₃₆ hydrocarbyl, an optionally substituted C₅-C₁₂ cycloalkyl, and an optionally substituted C₇-C₉ aralkyl; and
T₂ is chosen from hydrogen or T₁; and
a tertiary amine oxide compound according to Formula (IX): 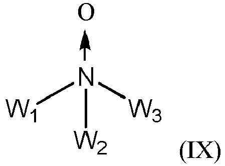 wherein
each of W₁ and W₂ is independently chosen from a C₆-C₃₆ hydrocarbyl chosen from a member selected from the group consisting of a straight or branched chain C₆-C₃₆ alkyl, a C₆-C₁₂ aryl, a C₇-C₃₆ aralkyl, a C₇-C₃₆ alkaryl, a C₅-C₃₆ cycloalkyl, a C₆-C₃₆ alkcycloalkyl; and a C₆-C₃₆ cycloalkylalkyl;
W₃ is chosen from a C₁-C₃₆ hydrocarbyl chosen from a member selected from the group consisting of a straight or branched chain C₁-C₃₆ alkyl, a C₆-C₁₂ aryl, a C₇-C₃₆ aralkyl, a C₇-C₃₆ alkaryl, a C₅-C₃₆ cycloalkyl, a C₆-C₃₆ alkcycloalkyl; and a C₆-C₃₆ cycloalkylalkyl;
with the proviso that at least one of Wi, W₂ and W₃ contains a β carbon-hydrogen bond; and
wherein said alkyl, aralkyl, alkaryl, cycloalkyl, alkcycloalkyl and cycloalkylalkyl groups may be interrupted by one to sixteen groups chosen from a member selected from the group consisting of -O-, -S-, -SO-, -SO₂-, -COO-, -OCO-, -CO-, -NW₄-, -CONW₄and -NW₄ CO-, or wherein said alkyl, aralkyl, alkaryl, cycloalkyl, alkcycloalkyl and cycloalkylallyl groups may be substituted by one to sixteen groups chosen from a member selected from the group consisting of -OW₄, -SW₄, -COOW₄, -OCOW₄, -COW₄, -N(W₄)₂, -CON(W₄)₂, -NW₄COW₄ and 5- and 6-membered rings containing the group -C(CH₃)(CH₂Rₓ)NL(CH₂Rₓ)(CH₃)C-, or wherein said alkyl, aralkyl, alkaryl, cycloalkyl, alkcycloalkyl and cycloalkylalkyl groups are both interrupted and substituted by the groups mentioned above; and
wherein
W₄ is chosen from hydrogen or a C₁-C₈ alkyl;
Rₓ is chosen from hydrogen or methyl; and
L is chosen from a C₁-C₃₀ alkyl; a --C(O)R moiety, or a --OR moiety, wherein R is a C₁-C₃₀ straight or branched chain alkyl group; and
wherein said aryl groups may be substituted by a member selected from the group consisting of one to three halogen groups, a C₁-C₈ alkyl group, a C₁-C₈ alkoxy group, and combinations thereof; for example
wherein the compound according to Formula (VIII) is a N,N-dihydrocarbylhydroxylamine chosen from a member selected from the group consisting of N,N-dibenzylhydroxylamine; N,N-diethylhydroxylamine; N,N-dioctylhydroxylamine; N,N-dilaurylhydroxylamine; N,N-didodecylhydroxylamine; N,N-ditetradecylhydroxylaamine; N,N-dihexadecylhydroxylamine; N,N-dioctadecylhydroxylamine; N-hexadecyl-N-tetradecylhydroxylamine; N-hexadecyl-N-heptadecylhydroxylamine; N-hexadecyl-N-octadecylhydroxylamine; N-heptadecyl-N-octadecylhydroxylamine; and N,N-di(hydrogenated tallow)hydroxylamine.

14. A use according to any one of the preceding claims, wherein the stabilizer composition is present from 0.001 % to 65.0 % by weight of the total weight of the organic material.

15. A use according to claim 14, wherein the stabilizer composition is present from 0.01 % to 25 % by weight of the total weight of the organic material.

## Patentansprüche

1. Verwendung einer Stabilisatorzusammensetzung, die eine stabilisierende Menge an Vitamin E-Acetat in einem organischen Polymermaterial zur Verbesserung der rheologischen Eigenschaft des organischen Polymermaterials umfasst, wobei die verbesserte rheologische Eigenschaft durch weniger Variation der Werte des Schmelzindex des organischen Polymermaterials bei wiederholten Messungen davon bei Extrusionstests mit mehreren Durchgängen gemessen wird.

2. Verwendung nach Anspruch 1, wobei die Stabilisatorzusammensetzung vor oder während der Verarbeitung des organischen Materials zugefügt wird.

3. Verwendung nach Anspruch 1 oder 2, wobei die Verwendung zum Zweck der Verbesserung der rheologischen Eigenschaft des organischen Polymermaterials in einem Verfahren zur Produktion eines Formartikels dient, wobei die Stabilisatorzusammensetzung vor oder während der Verarbeitung des organischen Materials zugefügt wird.

4. Verwendung nach Anspruch 3, wobei das Verfahren zur Produktion eines Formartikels ausgewählt ist aus Spritzgießen, Rotationsformen, Blasformen, Reel-to-Reel-Formen, Metallspritzgießen, Formpressen, Transferpressen, Tauchformen, gasunterstütztem Formen, Einsatzspritzgießen, Mikrospritzguss, Reaktionsspritzgießen und Zweikomponentenspritzgießen;
wobei das Verfahren zum Produzieren eines Formartikels beispielsweise ein Rotationsformungsverfahren ist.

5. Verwendung nach einem der Ansprüche 1 bis 4, wobei das organische Material ausgewählt ist aus der Gruppe bestehend aus Polyolefinen, Polyestern, Polyethern, Polyketonen, Polyamiden, natürlichen und synthetischen Kautschuken, Polyurethanen, Polystyrolen, Polyacrylaten, Polymethacrylaten, Polyacetalen, Polyacrylnitrilen, Polybutadienen, AcrylnitrilButadien-Styrol, Styrol-Acrylnitril, Acrylat-Styrol-Acrylnitril, Celluloseacetatbutyrat, Cellulosepolymeren, Polyimiden, Polyamidimiden, Polyetherimiden, Polyphenylsulfiden, Polyphenyloxidpolysulfonen, Polyethersulfonen, Polyvinylchloriden, Polycarbonaten, aliphatischen Polyketonen, thermoplastischen Olefinen, Aminoharz-vernetzten Polyacrylaten und Polyestern, Polyisocyanat-vernetzten Polyestern und Polyacrylaten, Phenol/Formaldehyd, Harnstoff/Formaldehyd- und Melamin/Formaldehyd-Harzen, Alkydharzen, Polyesterharzen, Acrylatharzen, die vernetzt sind mit Melaminharzen, Harnstoffharzen, Isocyanaten, Isocyanuraten, Carbamaten, Epoxyharzen, vernetzten Epoxyharzen, die abgeleitet sind von aliphatischen, cycloaliphatischen, heterocyclischen und aromatischen Glycidylverbindungen, die vernetzt sind mit Anhydriden oder Aminen, Polysiloxanen, Michael-Additionspolymeren, Aminen, blockierten Aminen mit aktivierten ungesättigten und Methylenverbindungen, Ketiminen mit aktivierten ungesättigten und Methylenverbindungen, Polyketiminen in Kombination mit ungesättigten acrylischen Polyacetoacetatharzen, Polyketiminen in Kombination mit ungesättigten Acrylharzen, strahlungshärtbaren Zusammensetzungen, Epoxymelaminharzen, organischen Farbstoffen, Kosmetikprodukten, Papierformulierungen auf Cellulosebasis, Fotografiefilmpapier, Fasern, Wachsen und Tinten;
wobei das organische Material beispielsweise ein Polyolefinpolymer ist, das ausgewählt ist aus der Gruppe bestehend aus i) Polymeren von Monoolefinen gewählt aus Polypropylen, Polyisobutylen, Polybut-1-en, Poly-4-methylpent-1-en; ii) Diolefinen gewählt aus Polyisopren oder Polybutadien; iii) Polymeren von Cycloolefinen gewählt aus Cyclopenten und Norbornen; iv) Polyethylen gewählt aus gegebenenfalls vernetztem Polyethylen, Polyethylen mit hoher Dichte (HDPE), Polyethylen mit hoher Dichte und hohem Molekulargewicht (HDPE-HMW), Polyethylen mit hoher Dichte und ultrahohem Molekulargewicht (HDPE-UHMW), Polyethylen mit mittlerer Dichte (MDPE), Polyethylen mit niedriger Dichte (LDPE), linearem Polyethylen mit niedriger Dichte (LLDPE), Polyethylen mit sehr niedriger Dichte (VLDPE) und Polyethylen mit ultraniedriger Dichte (ULDPE); v) Copolymeren davon und vi) Mischungen davon.

6. Verwendung nach einem der Ansprüche 1 bis 5, wobei die Stabilisatorzusammensetzung des Weiteren mindestens ein organisches Phosphit oder Phosphonit umfasst, ausgewählt aus der Gruppe bestehend aus einer Verbindung gemäß den Formeln 1 bis 7: 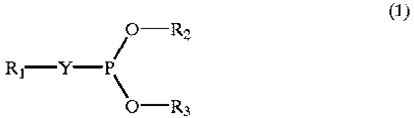 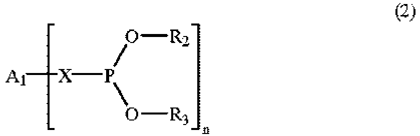 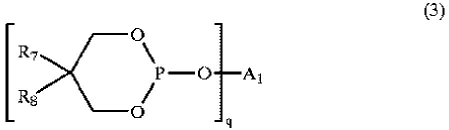 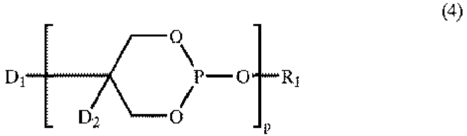 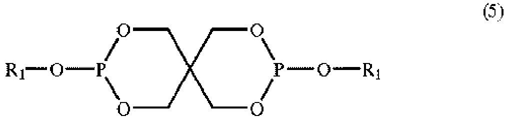 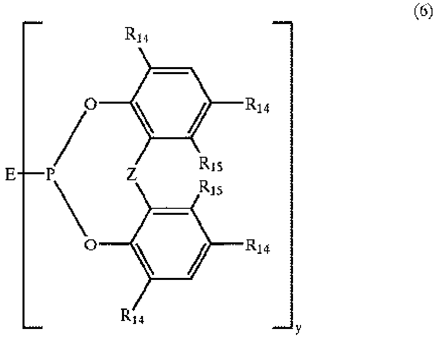 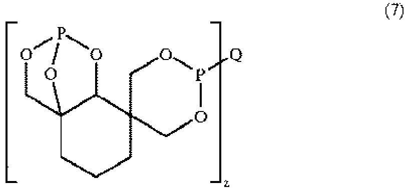 wobei die Indexzahlen ganzzahlig sind, und
n 2, 3 oder 4 ist; p 1 oder 2 ist; q 2 oder 3 ist;
r 4 bis 12 ist; y 1, 2 oder 3 ist; und z 1 bis 6 ist;
A₁, falls n 2 ist, C₂- bis C₁₈-Alkylen; C₂- bis C₁₂-Alkylen, das durch Sauerstoff, Schwefel oder -NR₄unterbrochen ist; ein Rest mit der Formel 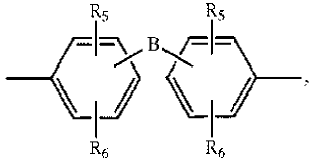 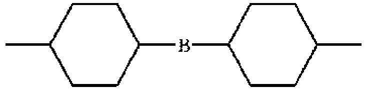 oder Phenylen ist;
A₁, falls n 3 ist, ein Rest mit der Formel -CᵣH₂ᵣ₋₁-ist;
A₁, falls n 4 ist, 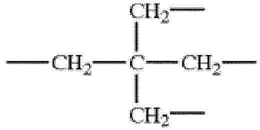 ist;
A₂ wie für A₁ definiert ist, falls n 2 ist;
B eine direkte Bindung, -CH₂-, -CHR₄-, -CR₁R₄-, Schwefel, C₅- bis C₇-Cycloalkyliden oder Cyclohexyliden ist, das mit 1 bis 4 C₁- bis C₄-Alkylresten in Position 3, 4 und/oder 5 substituiert ist;
D₁, falls p 1 ist, C₁- bis C₄-Alkyl ist, und, falls p 2 ist, -CH₂OCH₂- ist;
D₂, falls p 1 ist, C₁- bis C₄-Alkyl ist;
E, falls y 1 ist, C₁- bis C₁₈-Alkyl, -OR₁ oder Halogen ist;
E, falls y 2 ist, -O-A₂-O- ist,
E, falls y 3 ist, ein Rest mit der Formel R₄C(CH₂O-)₃ oder N(CH₂CH₂O-)₃ ist;
Q der Rest eines mindestens z-wertigen Alkohols oder Phenols ist, wobei dieser Rest über das Sauerstoffatom an das Phosphoratom gebunden ist;
R₁, R₂ und R₃ unabhängig voneinander C₁- bis C₁₈-Alkyl, das unsubstituiert oder mit Halogen, -COOR₄, -CN oder -CONR₄R₄ substituiert ist; C₂-bis C₁₈-Alkyl, das durch Sauerstoff, Schwefel oder -NR₄- unterbrochen ist; C₇- bis Cg-Phenylalkyl; C₅-bis C₁₂-Cycloalkyl, Phenyl oder Naphthyl; Naphthyl oder Phenyl, das mit Halogen, 1 bis 3 Alkylresten oder Alkoxyresten mit insgesamt 1 bis 18 Kohlenstoffatomen oder mit C₇- bis Cg-Phenylalkyl substituiert ist; oder ein Rest mit der folgenden Formel sind: 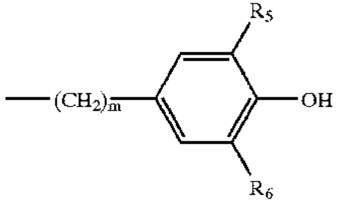
wobei m eine ganze Zahl im Bereich von 3 bis 6 ist;
R₄ Wasserstoff, C₁- bis C₈-Alkyl, C₅- bis C₁₂-Cycloalkyl oder C₇- bis Cg-Phenylalkyl ist,
R₅ und R₆ unabhängig voneinander Wasserstoff, C₁-bis C₈-Alkyl oder C₅- bis C₆-Cycloalkyl sind,
R₇ und R₈, falls q 2 ist, unabhängig voneinander C₁- bis C₄-Alkyl oder zusammen ein 2,3-Dehydropentamethylenrest sind; und
R₇ und R₈, falls q 3 ist, Methyl sind;
R₁₄ Wasserstoff, C₁- bis Cg-Alkyl oder Cyclohexyl ist,
R₁₅ Wasserstoff oder Methyl ist, und, falls zwei oder mehr Reste R₁₄ und R₁₅ vorhanden sind, diese Reste gleich oder verschieden sind,
X und Y jeweils eine direkte Bindung oder Sauerstoff sind,
Z eine direkte Bindung, Methylen, -C(R₁₆)₂- oder Schwefel ist, und
R₁₆ C₁- bis C₈-Alkyl ist;
einem Trisarylphosphit gemäß Formel 8: 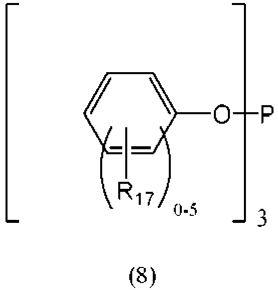
wobei R₁₇ ein Substituent ist, der an 0 bis 5 Positionen des aromatischen Anteils der Formel 8 gleich oder unterschiedlich ist und unabhängig gewählt ist aus einem Element ausgewählt aus der Gruppe bestehend aus C₁- bis C₂₀-Alkyl, C₃- bis C₂₀-Cycloalkyl, C₄- bis C₂₀-Alkylcycloalkyl, C₆- bis C₁₀-Aryl und C₇- bis C₂₀-Alkylaryl; und Kombinationen davon;
wobei beispielsweise das organische Phosphit oder Phosphonit ausgewählt ist aus der Gruppe bestehend aus Triphenylphosphit; Diphenylalkylphosphiten; Phenyldialkylphosphiten; Trilaurylphosphit; Trioctadecylphosphit; Distearylpentaerythritolphosphit; Tris(2,4-di-tert-butylphenyl)phosphit; Tris(nonylphenyl)phosphit; einer Verbindung mit den Formeln (A), (B), (C), (D), (E), (F), (G), (H), (J), (K) und (L): 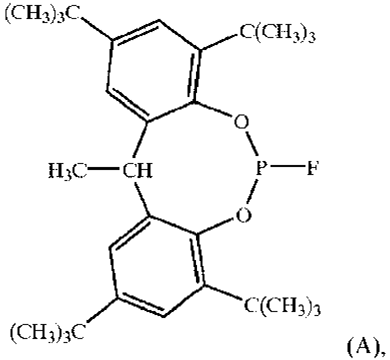 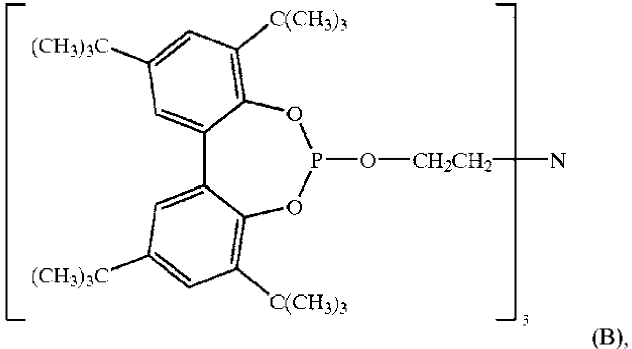 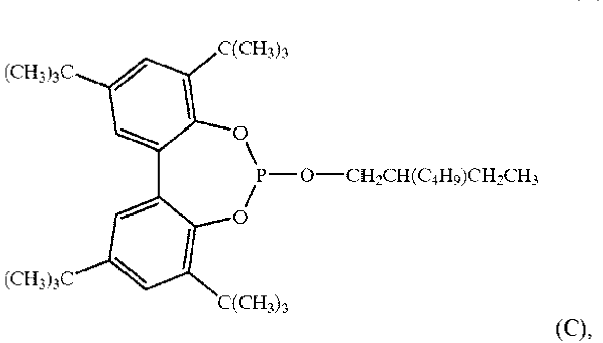 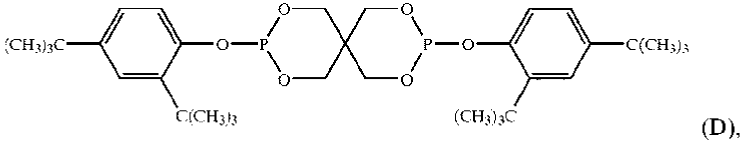 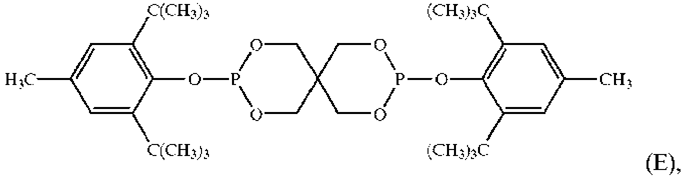 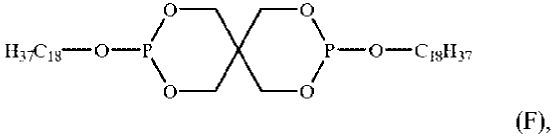 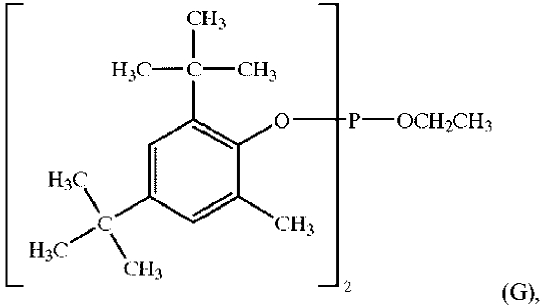 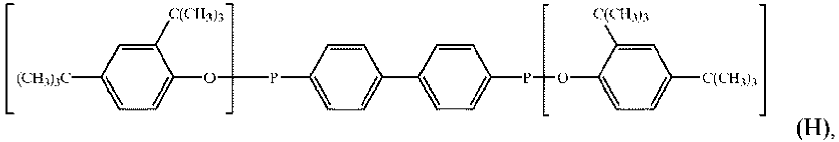 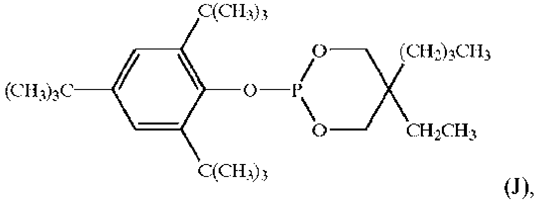 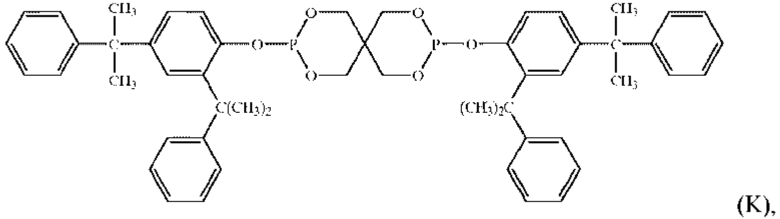 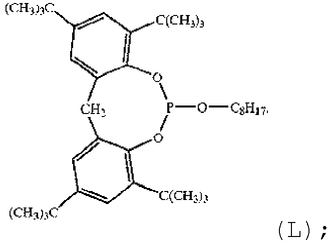 und 2-Butyl-2-ethyl-1,3-propandiol-2,4,6-tri-t-butyl-phenolphosphit, Bis-(2,6-di-t-butyl-4-methylphenyl)pentaerythritoldiphosphit, 2-Butyl-2-ethyl-1,3-propandiol-2,4-dicumylphenolphosphit, 2-Butyl-2-ethyl-1,3-propandiol-4-methyl-2,6-di-t-butylphe-nolphosphit und Bis-(2,4,6-tri-t-butyl-phenyl)pen-taerythritoldiphosphit.

7. Verwendung nach einem der vorhergehenden Ansprüche, wobei die Stabilisatorzusammensetzung des Weiteren mindestens eine gehinderte Phenolverbindung mit einem Molekülfragment gemäß einer oder mehreren der Formel (IVa), (IVb) oder (IVc) umfasst: 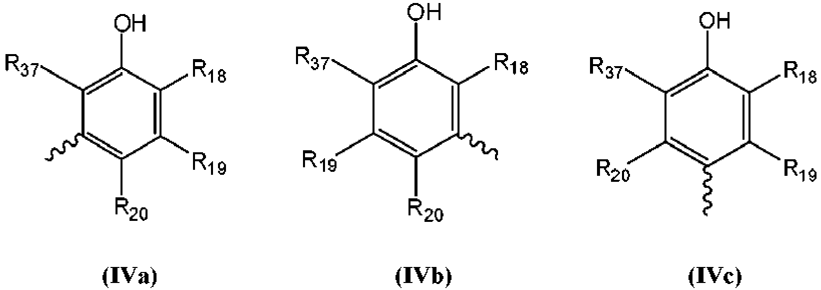 wobei
R₁₈ ausgewählt ist aus Wasserstoff oder einem C₁-bis C₄-Kohlenwasserstoffrest;
jeder von R₁₉ und R₂₀ unabhängig ausgewählt ist aus Wasserstoff oder einem C₁- bis C₂₀-Kohlenwasserstoffrest; und
R₃₇ ausgewählt ist aus einem C₁- bis C₁₂-Kohlenwasserstoffrest.

8. Verwendung nach Anspruch 7, wobei die mindestens eine gehinderte Phenolverbindung ausgewählt ist aus der Gruppe bestehend aus (1,3,5-Tris(4-t-butyl-3-hydroxy-2,6-dimethylbenzyl)-1,3,5-triazin-2,4,6-(1H,3H,5H)-trion; 1,1,3-Tris(2'-methyl-4'-hydroxy-5'-t-butylphenyl)butan; Triethylenglykolbis[3-(3-t-butyl-4-hydroxy-5-methylphenyl)propionat]; 4,4'-Thiobis(2-t-butyl-5-methylphenol); 2,2'-Thiodiethylenbis[3-(3-t-butyl-4-hydroxyl-5-methylphenyl)propionat]; Octadecyl-3-(3'-t-butyl-4'-hydroxy-5'-methylphenyl)propionat; Tetrakismethylen-(3-t-butyl-4-hydroxy-5-methylhydro-cinnamat)methan; N,N'-Hexamethylenbis[3-(3-t-butyl-4-hydroxy-5-methylphenyl)propionamid]; Di(4-tert.-butyl-3-hydroxy-2,6-dimethylbenzyl)thiodipropionat und Octadecyl-3,5-di-(tert)-butyl-4-hydroxyhydrocinnamat.

9. Verwendung nach einem der vorhergehenden Ansprüche,
wobei die Stabilisatorzusammensetzung des Weiteren mindestens eine Verbindung ausgewählt aus der Gruppe bestehend aus α-Tocopherol, β-Tocopherol, γ-Tocopherol, δ-Tocopherol, verwandten Tocotrienolen und Mischungen davon umfasst; und/oder wobei das Vitamin E-Acetat in 0,001 Gew.% bis 5,0 Gew.% des Gesamtgewichts der Stabilisatorzusammensetzung oder von 0,01 Gew.% bis 1,0 Gew.% des Gesamtgewichts der Stabilisatorzusammensetzung vorhanden ist.

10. Verwendung nach einem der vorhergehenden Ansprüche, wobei die Stabilisatorzusammensetzung des Weiteren eine effektive Menge eines Lichtstabilisators ausgewählt aus der Gruppe bestehend aus gehinderten Amin-Lichtstabilisatoren, gehinderten Hydroxylbenzoaten, Nickelphenolaten, Ultraviolettlichtstabilisatoren und Kombinationen davon umfasst.

11. Verwendung nach Anspruch 10, wobei der Lichtstabilisator eine gehinderte Amin-Lichtstabilisatorverbindung ist, die ein Molekülfragment gemäß Formel (VI) umfasst: 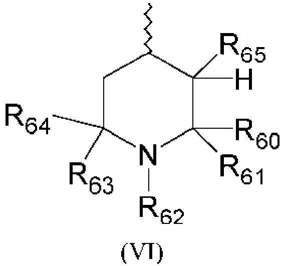 wobei
R₆₂ ausgewählt ist aus einem Element ausgewählt aus der Gruppe bestehend aus Wasserstoff; OH; C₁- bis C₂₀-Kohlenwasserstoffrest; -CH₂CN; C₁- bis C₁₂-Acyl und C₁- bis C₁₈-Alkoxy;
R₆₅ ausgewählt ist aus einem Element ausgewählt aus der Gruppe bestehend aus Wasserstoff und C₁- bis C₈-Kohlenwasserstoffrest; und
jeder von R₆₀, R₆₁, R₆₃ und R₆₄ unabhängig ausgewählt ist aus einem C₁- bis C₂₀-Kohlenwasserstoffrest, oder R₆₀ und R₆₁ und/oder R₆₃ und R₆₄ zusammen mit dem Kohlenstoff, an den sie gebunden sind, ein C₅-bis C₁₀-Cycloalkyl bilden;
oder Formel (VIa) 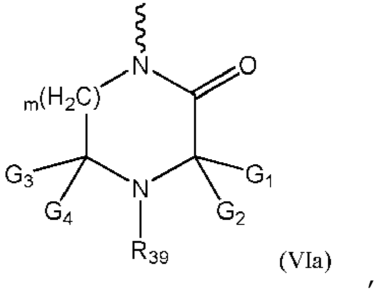 wobei
m eine ganze Zahl von 1 bis 2 ist, und
R₃₉ gewählt ist aus einem Element ausgewählt aus der Gruppe bestehend aus Wasserstoff; OH; C₁- bis C₂₀-Kohlenwasserstoff; -CH₂CN; C₁- bis C₁₂-Acyl und C₁- bis C₁₈-Alkoxy; und
jeder von G₁ bis G₄ unabhängig ausgewählt ist aus einem C₁- bis C₂₀-Kohlenwasserstoffrest; und/oder wobei der gehinderte Amin-Lichtstabilisator ausgewählt ist aus der Gruppe bestehend aus Bis-(2,2,6,6-tetramethylpiperidin-4-yl)sebacat; Bis-(2,2,6,6-tetramethylpiperidin-4-yl)succinat; Bis-(1,2,2,6,6-pentamethylpiperidin-4-yl)sebacat; Bis-(1-octyloxy-2,2,6,6-tetramethylpiperidin-4-yl) sebacat; Bis(1,2,2,6,6-pentamethylpiperidin-4-yl)-n-butyl-3,5-di-tert-butyl-4-hydroxybenzylmalonat; einem Kondensat aus 1-(2-Hydroxyethyl)-2,2,6,6-tetramethyl-4-hydroxypiperidin und Bernsteinsäure; 2,2,6,6-Tetramethylpiperidin-4-ylstearat; 2,2,6,6-Tetramethylpiperidin-4-yldodecanat; 1,2,2,6,6-Pentamethylpiperidin-4-ylstearat; 1,2,2,6,6-Pentamethylpiperidin-4-yldodecanat; einem Kondensat von N,N'-Bis(2,2,6,6-tetramethylpiperidin-4-yl)hexamethylendiamin und 4-tert-Octylamino-2,6-dichlor-1,3,5-triazin; Tris(2,2,6,6-tetramethylpiperidin-4-yl)nitrilotriacetat; Tetrakis(2,2,6,6-tetramethylpiperidin-4-yl)-1,2,3,4-butantetracarboxylat; 4-Benzoyl-2,2,6,6-tetramethylpiperidin; 4-Stearyl-oxy-2,2,6,6-tetramethylpiperidin; Bis(1,2,2,6,6-pentamethylpiperidyl)-2-n-butyl-2-(2-hydroxy-3,5-di-tert-butylbenzyl)malonat; 3-n-Octyl-7,7,9,9-tetramethyl-1,3,8-triazaspiro[4.5]decan-2,4-dion; Bis(1-octyloxy-2,2,6,6-tetramethylpiperidyl) sebacat; Bis(1-octyloxy-2,2,6,6-tetramethylpiperidyl)-succinat; einem Kondensat von N,N'-Bis(2,2,6,6-tetramethylpiperidin-4-yl)hexamethylendiamin und 4-Morpholino-2,6-dichlor-1,3,5-triazin; einem Kondensat von 2-Chlor-4,6-bis(4-n-butylamino-2,2,6,6-tetramethylpiperidyl)-1,3,5-triazin und 1,2-Bis(3-aminopropylamino)ethan; einem Kondensat von 2-Chlor-4,6-bis(4-n-butylamino-1,2,2,6,6-pentame-thylpiperidyl)-1,3,5-triazin und 1,2-Bis-(3-amino-propylamino)ethan; 8-Acetyl-3-dodecyl-7,7,9,9-tetramethyl-1,3,8-triazaspiro[4.5]decan-2,4-dion; 3-Dodecyl-1-(2,2,6,6-tetramethylpiperidin-4-yl)pyrrolidin-2,5-dion; 3-Dodecyl-1-(1-ethanoyl-2,2,6,6-tetramethylpiperidin-4-yl)pyrrolidin-2,5-dion; 3-Dodecyl-1-(1,2,2,6,6-pentamethylpiperidin-4-yl)-pyrrolidin-2,5-dion; einer Mischung von 4-Hexadecyloxy- und 4-Stearyloxy-2,2,6,6-tetramethylpiperidin; einem Kondensat von N,N'-Bis(2,2,6,6-tetramethylpiperidin-4-yl)hexamethylendiamin und 4-Cyc-lohexylamino-2,6-dichlor-1,3,5-triazin; einem Kondensat von 1,2-Bis(3-aminopropylamino)ethan, 2,4,6-Trichlor-1,3,5-triazin und 4-Butylamino-2,2,6,6-tetramethylpiperidin; 2-Undecyl-7,7,9,9-tetramethyl-l-oxa-3,8-diaza-4-oxospiro[4.5]decan; Oxopiperanzinyltriazinen; einem Reaktionsprodukt von 7,7,9,9-Tetramethyl-2-cycloundecyl-1-oxa-3,8-diaza-4-oxospiro[4.5]decan und Epichlorhydrin; Tetrakis(2,2,6,6-tetramethyl-4-piperidyl)butan-1,2,3,4-tetracarboxylat; 1,2,3,4-Butantetracarbonsäure, Tetrakis(1,2,2,6,6-pentamethyl-4-piperidinyl)ester; 1,2,3,4-Butantetracarbonsäure, 1,2,2,6,6-Pentamethyl-4-piperdinyltridecylester; 1,2,3,4-Butantetracarbonsäure, 2,2,6,6-Tetramethyl-4-piperidinyltridecylester; 1,2,3,4-Butantetracarbonsäure, Polymer mit 2,2,6,6-Tetramethyl-2,4,8,10-tetraoxaspiro[5.5]-undecan-3,9-diethanol, 1,2,2,6,6-Pentamethyl-4-piperdinylester; 1,2,3,4-Butantetracarbonsäure, Polymer mit 2,2,6,6-Tetramethyl-2,4,8,10-tetraoxaspiro[5.5]-undecan-3,9-diethanol, 2,2,6,6-Tetramethyl-4-piperdinylester; Bis(1-undecanoxy-2,2,6,6-tetramethylpiperidin-4-yl)carbonat; 1-(2-Hydroxy-2-methylpropoxy)-2,2,6,6-tetramethyl-4-piperdinol; 1-(2-Hydroxy-2-methylpropoxy)-4-octadecanoyloxy-2,2,6,6-tetramethylpiperidin; 1-(4-Octadecanoyloxy-2,2,6,6-tetramethylpiperidin-1-yloxy)-2-octadecanoyloxy-2-me-thylpropan; 1-(2-Hydroxyethyl)-2,2,6,6-tetramethyl-4-piperdinol; einem Reaktionsprodukt von 1-(2-Hydroxyethyl)-2,2,6,6-tetramethyl-4-piperdinol und Dimethylsuccinat; 2,2,4,4-Tetramethyl-7-oxa-3,20-diazadispiro[5.1.11,2]heneicosan-21-on; dem Ester von 2,2,6,6-Tetramethyl-4-piperidinol mit höheren Fettsäuren; 3-Dodecyl-1-(2,2,6,6-tetramethyl-4-piperidyl)pyrrolidin-2,5-dion; 1H-Pyrrol-2,5-dion, 1-Octadecyl-, Polymer mit (1-Methylethe-nyl)benzol und 1-(2,2,6,6-Tetramethyl-4-piperidinyl)-1H-pyrrol-2,5-dion; Piperazinon, 1,1',1"-[1,3,5-Triazin-2,4,6-triyltris[(cyclohexylimino)-2,1-ethandiyl]]t-ris[3,3,5,5-t-etramethyl-; Piperazinon, 1,1',1"-[1,3,5-Triazin-2,4,6-triyltris-[(cyclohexylimino)-2,1-ethandiyl]]tris[3,3,4,5,5-pentamethyl-; dem Reaktionsprodukt von 7,7,9,9-Tetramethyl-2-cycloundecyl-1-oxa-3,8-diaza-4-oxospi-ro[4.5]decan und Epichlorhydrin; dem Kondensat von N,N'-Bis(2,2,6,6-tetramethylpiperidin-4-yl)hexamethylendiamin und 4-Cyclohexylamino-2,6-dichlor-1,3,5-triazin; dem Kondensat von 1,2-Bis(3-amino-propylamino)ethan, 2,4,6-Trichlor-1,3,5-triazin und 4-Butylamino-2,2,6,6-tetramethylpiperidin; dem Kondensat von N,N'-Bis(2,2,6,6-tetramethylpiperidin-4-yl)hexamethylendiamin und 4-Morpholino-2,6-dichlor-1,3,5-triazin; dem Kondensat von 2-Chlor-4,6-bis(4-n-butylamino-2,2,6,6-tetramethylpiperidyl)-1,3,5-triazin und 1,2-Bis(3-aminopropylami-no)ethan; dem Kondensat von 2-Chlor-4,6-bis(4-n-butylamino-1,2,2,6,6-pentamethylpiperidyl)-1,3,5-triazin und 1,2-Bis-(3-aminopropylamino)ethan; 2-[(2-Hydroxyethyl)amino]-4,6-bis[N-(1-cyclohexyl-oxy-2,2,6,6-tetramethylpiperidin-4-yl)butylamino-1,3,5-triazin; Propandisäure, [(4-Methoxyphenyl)-methylen]-bis-(1,2,2,6,6-pentamethyl-4-piperidinyl)ester; Tetrakis(2,2,6,6-tetramethylpiperidin-4-yl)-1,2,3,4-butantetracarboxylat; Benzolpropansäure, 3,5-Bis(1,1-dimethylethyl)-4-hydroxy-, 1-[2-[3-[3,5-bis(1,1-dimethylethyl)-4-hydroxyphenyl]-1-oxopropoxy]ethyl]-2,2,6,6-tetramethyl-4-pi-peridinylester; N-(1-Octyloxy-2,2,6,6-tetramethylpiperidin-4-yl)-N'-dodecyloxalamid; Tris(2,2,6,6-tetramethylpiperidin-4-yl)nitrilotriacetat; 1,5-Dioxaspiro{5,5}undecan-3,3-dicarbonsäure, Bis-(1,2,2,6,6-pentamethyl-4-piperidinyl); 1,5-Dioxa-spiro{5,5}undecan-3,3-dicarbonsäure, Bis(2,2,6,6-tetramethyl-4-piperidinyl); dem Kondensat von 1-(2-Hydroxyethyl)-2,2,6,6-tetramethyl-4-hydroxypiperidin und Bernsteinsäure; dem Kondensat von N,N'-Bis(2,2,6,6-tetramethylpiperidin-4-yl)hexamethylendiamin und 4-tert-Octylamino-2,6-dichlor-1,3,5-triazin; 1,2,3,4-Butantetracarbonsäure, 1,2,2,6,6-Pentamethyl-4-piperidinyltridecylester; Tetrakis(2,2,6,6-tetramethylpiperidin-4-yl)-1,2,3,4-butantetracarboxylat; 1,2,3,4-Butantetracarbonsäure, 2,2,6,6-Tetramethyl-4-piperidinyltri-decylester; Tetrakis(1,2,2,6,6-pentamethylpiperidin-4-yl)-1,2,3,4-butantetracarboxylat; Mischung von 2,2,4,4-Tetramethyl-21-oxo-7-oxa-3.20-diaza-spiro(5.1.11,2)-heneicosan-20-propansäure-dodecylester und 2,2,4,4-Tetramethyl-21-oxo-7-oxa-3.20-diazaspiro(5.1.11.2)-heneicosan-20-propansäurete-tradecylester; 1H,4H,5H,8H-2,3a,4a,6,7a,8a-Hexa-azacyclopenta[def]fluoren-4,8-dion, Hexahydro-2,6-bis(2,2,6,6-tetramethyl-4-piperidinyl)-; Polymethyl[propyl-3-oxy(2',2',6',6'-tetramethyl-4,4'-piperidinyl)]siloxan; Polymethyl[propyl-3-oxy-(1',2',2',6',6'-pentamethyl-4,4'-piperidinyl) ]siloxan; Copolymer von Methylmethacrylat mit Ethylacrylat und 2,2,6,6-Tetramethylpiperidin-4-ylacry-lat; Copolymer von gemischten C₂₀- bis C₂₄-alpha-Olefinen und (2,2,6,6-Tetramethylpiperidin-4-yl)-succinimid; 1,2,3,4-Butantetracarbonsäure, Polymer mit β,β,β',β'-Tetramethyl-2,4,8,10-tetraoxaspiro-[5.5]undecan-3,9-diethanol, 1,2,2,6,6-Pentamethyl-4-piperidinylester; 1,2,3,4-Butantetracarbonsäure, Polymer mit β,β,β',β'-Tetramethyl-2,4,8,10-tetra-oxaspiro[5.5]undecan-3,9-diethanol, 2,2,6,6-Tetramethyl-4-piperidinylestercopolymer; 1,3-Benzoldicarboxamid, N,N'-Bis(2,2,6,6-tetramethyl-4-piperidinyl; 1,1'-(1,10-Dioxo-1,10-decandiyl)-bis(hexa-hydro-2,2,4,4,6-pentamethylpyrimidin; Ethandiamid, N-(1-Acetyl-2,2,6,6-tetramethylpiperidinyl)-N'-dodecyl; Formamid, N,N'-1,6-Hexandiylbis[N-(2,2,6,6-tetramethyl-4-piperidinyl); D-Glucitol, 1,3:2,4-Bis-O-(2,2,6,6-tetramethyl-4-piperidinyliden)-; 2,2,4,4-Tetramethyl-7-oxa-3,20-diaza-21-oxo-dispi-ro[5.1.11.2]heneicosan; Propanamid, 2-Methyl-N-(2,2,6,6-tetramethyl-4-piperidinyl)-2-[(2,2,6,6-tetramethyl-4-piperidinyl)amino]-; 7-Oxa-3,20-di-azadispiro[5.1.11,2]heneicosan-20-propansäure, 2,2,4,4-Tetramethyl-21-oxo-, Dodecylester; N-(2,2,6,6-Tetramethylpiperidin-4-yl)-ß-aminopropi-onsäuredodecylester; N-(2,2,6,6-Tetramethylpiperidin-4-yl)-N'-aminooxalamid; Propanamid, N-(2,2,6,6-Tetramethyl-4-piperidinyl)-3-[(2,2,6,6-tetramethyl-4-piperidinyl)amino]-; Mischung von 4-Hexadecyloxy- und 4-Stearyloxy-2,2,6,6-tetramethylpiperidin; 3-Dodecyl-1-(1,2,2,6,6-Pentamethylpiperidin-4-yl)pyrrolidin-2,5-dion; 3-Dodecyl-1-(1-ethanoyl-2,2,6,6-pentamethylpiperidin-4-yl)pyrrolidin-2,5-dion; Bis(2,2,6,6-tetramethylpiperidin-4-yl)succinat; Bis(1,2,2,6,6-pentamethylpiperidin-4-yl)-n-butyl-3,5-di-tert-butyl-4-hydroxy-benzylmalonat; Tris(2,2,6,6-tetramethylpiperidin-4-yl)nitrilotriacetat; 1,1'-(1,2-Ethandiyl)bis-(3,3,5,5-tetramethylpiperazinon); 4-Benzoyl-2,2,6,6-tetramethylpiperidin; 4-Stearyloxy-2,2,6,6-tetramethylpiperidin; Bis(1,2,2,6,6-penta-methylpiperidyl)-2-n-butyl-2-(2-hydroxy-3,5-di-tert.-butylbenzyl)malonat; 3-n-Octyl-7,7,9,9-tetramethyl-1,3,8-triazaspiro[4.5]decan-2,4-dion; Bis(1-octyloxy-2,2,6,6-tetramethylpiperidyl) sebacat; Bis(1-octyloxy-2,2,6,6-tetramethylpiperidyl)-succinat; 8-Acetyl-3-dodecyl-7,7,9,9-tetramethyl-1,3,8-triazaspiro[4.5]decan-2,4-dion; 3-Dodecyl-1-(2,2,6,6-tetramethylpiperidin-4-yl)pyrrolidin-2,5-dion; 3-Dodecyl-1-(1-ethanoyl-2,2,6,6-tetramethylpiperidin-4-yl)pyrrolidin-2,5-dion; 3-Dodecyl-1-(1,2,2,6,6-pentamethylpiperidin-4-yl)pyrrolidin-2,5-dion; einer Mischung aus 4-Hexadecyloxy- und 4-Stearyloxy-2,2,6,6-tetramethylpiperidin; 2-Undecyl-7,7,9,9-tetramethyl-1-oxa-3,8-diaza-4-oxospi-ro[4.5]decan; 1,5-Dioxaspiro{5,5}undecan-3,3-dicarbonsäure, Bis(2,2,6,6-tetramethyl-4-piperidinyl) und 1,5-Dioxaspiro{5,5}undecan-3,3-dicarbonsäure, Bis(1,2,2,6,6-pentamethyl-4-piperidinyl); N¹-(ß-Hydroxyethyl)3,3-pentamethylen-5,5-dimethyl-piperazin-2-on; N¹-tert-Octyl-3,3,5,5-tetramethyl-diazepin-2-on; N¹-tert-Octyl-3,3-pentamethylen-5,5-hexamethylendiazepin-2-on; N¹-tert-Octyl-3,3-pentamethylen-5,5-dimethylpiperazin-2-on; trans-1,2-Cyclohexan-bis-(N¹-5,5-dimethyl-3,3-pentamethylen-2-piperazinon; trans-1,2-Cyclohexan-bis-(N¹-3,3,5,5-dispiropentamethylen-2-piperazinon); N¹-Isopropyl-1,4-diazadispiro-(3,3,5,5)pentamethylen-2-piperazinon; N¹-Isopropyl-1,4-diazadispiro-3,3-pentamethylen-5,5-tetramethylen-2-piperazinon; N¹-Isopropyl-5,5-dimethyl-3,3-pentamethylen-2-piperazinon; trans-1,2-Cyclohexanbis-N¹-(dimethyl-3,3-pentamethylen-2-piperazinon); N¹-Octyl-5,5-dimethyl-3,3-pentamethylen-1,4-diazepin-2-on und N¹-Octyl-1,4-diazadispiro-(3,3,5,5)pentamethylen-1,5-diazepin-2-on.

12. Verwendung nach Anspruch 10 oder 11, wobei der Lichtstabilisator einen Ultraviolettlichtabsorber ausgewählt aus der Gruppe bestehend aus einer 2-Hydroxybenzophenonverbindung, einer 2-(2'-Hydroxyphenyl)benzotriazolverbindung, einer 2-(2'-Hydroxyphenyl)-1,3,5-triazinverbindung und Kombinationen davon umfasst;
wobei der Ultraviolettlichtabsorber beispielsweise eine 2-(2-Hydroxyphenyl)-1,3,5-triazinverbindung ist, die ausgewählt ist aus der Gruppe bestehend aus 4,6-Bis-(2,4-dimethylphenyl)-2-(2-hydroxy-4-octyloxyphenyl)-s-triazin; 4,6-Bis-(2,4-dimethylphenyl)-2-(2,4-dihydroxyphenyl)-s-triazin; 2,4-Bis(2,4-dihydroxyphenyl)-6-(4-chlorphenyl)-s-triazin; 2,4-Bis[2-hydroxy-4-(2-hydroxy-ethoxy)phenyl]-6-(4-chlorphenyl)-s-triazin; 2,4-Bis[2-hydro-xy-4-(2-hydroxy-4-(2-hydroxyethoxy)phenyl]-6-(2,4-dimethylphenyl)-s-triazin; 2,4-Bis[2-hydroxy-4-(2-hydroxyethoxy)phenyl]-6-(4-bromphenyl)-s-triazin; 2,4-Bis[2-hydroxy-4-(2-acetoxyethoxy)phenyl]-6-(4-chlorphenyl)-s-triazin; 2,4-Bis(2,4-dihydroxyphenyl)-6-(2,4-dimethylphenyl)-s-triazin; 2,4-Bis(4-biphenylyl)-6-[2-hydroxy-4-[(octyloxycarbonyl)-ethylidenoxy]phenyl]-s-triazin; 2,4-Bis(4-bipheny-lyl)-6-[2-hydroxy-4-(2-ethylhexyloxy)phenyl]-s-triazin; 2-Phenyl-4-[2-hydroxy-4-(3-sec-butyloxy-2-hydroxypropyloxy)phenyl]-6-[2-hydroxy-4-(3-sec-amyloxy-2-hydroxypropyloxy)phenyl]-s-triazin; 2,4-Bis(2,4-dimethylphenyl)-6-[2-hydroxy-4 (-3-benzyl-oxy-2-hydroxypropyloxy)phenyl]-s-triazin; 2,4-Bis-(2-hydroxy-4-n-butyloxyphenyl)-6-(2,4-di-n-butyl-oxyphenyl)-s-triazin; 2,4-Bis(2,4-dimethylphenyl)-6-[2-hydroxy-4-(3-nonyloxy-2-hydroxypropyloxy)-5-α-cumylphenyl]-s-triazin; Methylenbis-{2,4-bis-(2,4-dimethylphenyl)-6-[2-hydroxy-4-(3-butyloxy-2-hydroxypropoxy)phenyl]-s-triazin}; Methylen-verbrückter Dimermischung, die in den Positionen 3:5', 5:5' und 3:3' in einem Verhältnis von 5:4:1 verbrückt ist; 2,4,6-Tris(2-hydroxy-4-isooctyloxy-carbonylisopropylidenoxyphenyl)-s-triazin; 2,4-Bis(2,4-dimethylphenyl)-6-(2-hydroxy-4-hexyloxy-5-α-cumylphenyl)-s-triazin; 2-(2,4,6-Trimethylphenyl)-4,6-bis[2-hydroxy-4-(3-butyloxy-2-hydroxypro-pyloxy)phenyl]-s-triazin; 2,4,6-Tris[2-hydroxy-4-(3-sec-butyloxy-2-hydroxypropyloxy)-phenyl]-s-triazin; Mischung von 4,6-Bis-(2,4-dimethylphenyl)-2-(2-hydroxy-4-(3-dodecyloxy-2-hydroxypropoxy)phenyl)-s-triazin und 4,6-Bis-(2,4-dimethylphenyl)-2-(2-hydroxy-4-(3-tridecyloxy-2-hydroxypropoxy)phenyl)-s-triazin; 4,6-Bis-(2,4-dimethylphenyl)-2-(2-hydroxy-4(3-(2-ethylhexyloxy)-2-hydroxypropoxy)-phenyl)-s-triazin; 4,6-Diphenyl-2-(4-hexyloxy-2-hydroxyphenyl)-s-triazin und Kombinationen davon.

13. Verwendung nach einem der vorhergehenden Ansprüche, wobei die Stabilisatorverbindung des Weiteren mindestens eine Verbindung umfasst, die ausgewählt ist aus der Gruppe bestehend aus:
einer Hydroxylaminverbindung gemäß Formel (VIII): 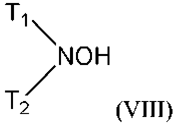 wobei
T₁ ausgewählt ist aus einem Element ausgewählt aus der Gruppe bestehend aus einem gegebenenfalls substituierten C₁- bis C₃₆-Kohlenwasserstoffrest, einem gegebenenfalls substituierten C₅- bis C₁₂-Cycloalkyl und einem gegebenenfalls substituierten C₇- bis Cg-Aralkyl; und
T₂ ausgewählt ist aus Wasserstoff oder T₁; und einer tertiären Aminoxidverbindung gemäß Formel (IX) : 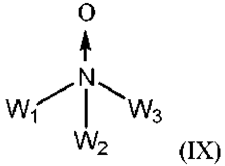 wobei
jeder von W₁ und W₂ unabhängig gewählt ist aus einem C₆- bis C₃₆-Kohlenwasserstoffrest ausgewählt aus einem Element ausgewählt aus der Gruppe bestehend aus geradkettigem oder verzweigtkettigem C₆-bis C₃₆-Alkyl, einem C₆- bis C₁₂-Aryl, einem C₇- bis C₃₆-Aralkyl, einem C₇- bis C₃₆-Alkaryl, einem C₅-bis C₃₆-Cycloalkyl, einem C₆- bis C₃₆-Alkcycloalkyl und einem C₆- bis C₃₆-Cycloalkylalkyl;
W₃ gewählt ist aus einem C₁- bis C₃₆-Kohlenwasserstoffrest gewählt aus einem Element ausgewählt aus der Gruppe bestehend aus geradkettigem oder verzweigtkettigem C₁- bis C₃₆-Alkyl, einem C₆- bis C₁₂-Aryl, einem C₇- bis C₃₆-Aralkyl, einem C₇- bis C₃₆-Alkaryl, einem C₅- bis C₃₆-Cycloalkyl, einem C₆- bis C₃₆-Alkcycloalkyl und einem C₆- bis C₃₆-Cycloalkylalkyl;
mit der Maßgabe, dass mindestens eines von W₁, W₂ und W₃ eine β-Kohlenstoff-Wasserstoff-Bindung enthält; und
wobei die Alkyl-, Aralkyl-, Alkaryl-, Cycloalkyl-, Alkcycloalkyl- und Cycloalkylalkylgruppen durch eine bis sechzehn Gruppen gewählt aus einem Element ausgewählt aus der Gruppe bestehend aus -O-, -S-, -SO-, -SO₂-, -COO-, -OCO-, -CO-, -NW₄-, -CONW₄- und -NW₄CO- unterbrochen sein können, oder wobei die Alkyl-, Aralkyl-, Alkaryl-, Cycloalkyl-, Alkcycloalkyl- und Cycloalkylallylgruppen mit einer bis sechzehn Gruppen substituiert sein können, die gewählt sind aus einem Element ausgewählt aus der Gruppe bestehend aus -OW₄, -SW₄, -COOW₄, -OCOW₄, -COW₄, -N(W₄)₂, -CON(W₄)₂, -NW₄COW₄ und 5-und 6-gliedrigen Ringen, welche die Gruppe -C (CH₃) (CH₂R_{X})NL (CH₂R_{X}) (CH₃) C- enthalten, oder wobei die Alkyl-, Aralkyl-, Alkaryl-, Cycloalkyl-, Alkcycloalkyl- und Cycloalkylalkylgruppen sowohl unterbrochen als auch durch die oben genannten Gruppen substituiert sind; und wobei
W₄ ausgewählt ist aus Wasserstoff oder C₁-C₈-Alkyl;
Rₓ ausgewählt ist aus Wasserstoff oder Methyl; und
L ausgewählt ist aus einem C₁- bis C₃₀-Alkyl; einem Anteil -C(O)R oder einem Anteil -OR, wobei R eine geradkettige oder verzweigte C₁- bis C₃₀-Alkylgruppe ist; und
wobei die Arylgruppen durch ein Element ausgewählt aus der Gruppe bestehend aus einer bis drei Halogengruppen, einer C₁- bis C₈-Alkylgruppe, einer C₁-bis C₈-Alkoxygruppe und Kombinationen davon substituiert sein können; wobei beispielsweise die Verbindung gemäß Formel (VIII) ein N,N-Dikoh-lenwasserstoffhydroxylamin gewählt aus einem Element ausgewählt aus der Gruppe bestehend aus N,N-Dibenzylhydroxylamin; N,N-Diethylhydroxylamin; N,N-Dioctylhydroxylamin; N,N-Dilaurylhydroxylamin; N,N-Didodecylhydroxylamin; N,N-Ditetradecylhydro-xylamin; N,N-Dihexadecylhydroxylamin; N,N-Diocta-decylhydroxylamin; N-Hexadecyl-N-tetradecylhydroxylamin; N-Hexadecyl-N-heptadecylhydroxylamin; N-Hexadecyl-N-octadecylhydroxylamin; N-Heptadecyl-N-octadecylhydroxylamin und N,N-Di(hydriertem Talg)-hydroxylamin ist.

14. Verwendung nach einem der vorhergehenden Ansprüche, wobei die Stabilisatorzusammensetzung in 0,001 Gew.% bis 65,0 Gew.% des Gesamtgewichts des organischen Materials vorhanden ist.

15. Verwendung nach Anspruch 14, wobei die Stabilisatorzusammensetzung in 0,01 Gew.% bis 25 Gew.% des Gesamtgewichts des organischen Materials vorhanden ist.

## Revendications

1. Utilisation d'une composition stabilisante comprenant une quantité stabilisante d'acétate de vitamine E dans un matériau polymère organique pour améliorer la propriété rhéologique dudit matériau polymère organique, dans laquelle la propriété rhéologique améliorée est mesurée par une plus faible variation des valeurs d'indice de fluidité dudit matériau polymère organique après des mesures répétées de celui-ci dans un essai d'extrusion à passages multiples.

2. Utilisation selon la revendication 1, dans laquelle ladite composition stabilisante est ajoutée avant ou pendant le traitement dudit matériau organique.

3. Utilisation selon la revendication 1 ou 2 dans laquelle ladite utilisation a pour objectif l'amélioration de la propriété rhéologique dudit matériau polymère organique dans un procédé de production d'un article moulé, dans laquelle ladite composition stabilisante est ajoutée avant ou pendant le traitement dudit matériau organique.

4. Utilisation selon la revendication 3 dans laquelle ledit procédé de production d'un article moulé est choisi parmi un moulage par injection, un rotomoulage, un moulage par extrusion soufflage, un moulage rouleau à rouleau, métal moulage par injection, compression moulage, un moulage par transfert, un moulage par immersion, un moulage assisté par gaz, un moulage par insertion-injection, un micromoulage, un moulage par réaction-injection, et un moulage par injection à double injection ;
par exemple dans laquelle ledit procédé de production d'un article moulé est un procédé de rotomoulage.

5. Utilisation selon l'une quelconque des revendications 1 à 4, dans laquelle le matériau organique est choisi dans le groupe constitué de polyoléfines, polyesters, polyéthers, polycétones, polyamides, caoutchoucs naturels et synthétiques, polyuréthanes, polystyrènes, polyacrylates, polyméthacrylates, polyacétals, polyacrylonitriles, polybutadiènes, acrylonitrile-butadiène-styrène, styrène-acrylonitrile, acrylate-styrène-acrylonitrile, acétate-butyrate cellulosique, polymères cellulosiques, polyimides, polyamideimides, polyétherimides, polyphénylsulfures, polyphényloxydepolysulfones, polyéthersulfones, polychlorures de vinyle, polycarbonates, polycétones aliphatiques, oléfines thermoplastiques, polyacrylates et polyesters réticulés par aminorésine, polyesters et polyacrylates réticulés par polyisocyanate, résines phénol/formaldéhyde, urée/formaldéhyde et melamine/formaldéhyde, résines alkyde, résines polyester, résines acrylate réticulées avec des résines mélamine, résines urée, isocyanates, isocyanurates, carbamates, résines époxy, résines époxy réticulées dérivées de composés glycidyle aliphatiques, cycloaliphatiques, hétérocycliques et aromatiques, qui sont réticulés avec des anhydrides ou des amines, polysiloxanes, polymères formés par addition de Michael, amines, amines bloques avec des composés insaturés et méthylène activés, cétimines avec des composés insaturés et méthylène activés, polycétimines en combinaison avec des résines acryliques-polyacétoacétate insaturées, polycétimines en combinaison avec des résines acryliques insaturées, compositions durcissables par rayonnement, résines époxymélamine, colorants organiques, produits cosmétiques, formulations de papier à base de cellulose, papier pour film photographique, fibres, cires et encres ;
par exemple dans laquelle le matériau organique est un polymère de polyoléfine choisi dans le groupe constitué de i) polymères de monooléfines choisis parmi les suivants : polypropylène, polyisobutylène, polybut-1-ène, poly-4-méthylpent-1-ène ; ii) dioléfines choisies parmi le polyisoprène ou le polybutadiène ; iii) polymères de cyclooléfines choisis parmi le cyclopentène et le norbornène ; iv) polyéthylène choisi parmi polyéthylène facultativement réticulé, polyéthylène haute densité (HDPE), polyéthylène haute densité et de poids moléculaire élevé (HDPE-HMW), polyéthylène haute densité et de poids moléculaire très élevé (HDPE-UHMW), polyéthylène moyenne densité (MDPE), polyéthylène basse densité (LDPE), polyéthylène basse densité linéaire (LLDPE), polyéthylène très basse densité (VLDPE), et polyéthylène ultrabasse densité (ULDPE) ; v) copolymères de ceux-ci ; et vi) mélanges de ceux-ci.

6. Utilisation selon l'une quelconque des revendications 1 à 5, dans laquelle la composition stabilisante comprend en outre au moins un phosphite ou une phosphonite organique choisis dans le groupe constitué d'un composé selon les formules 1 à 7 : 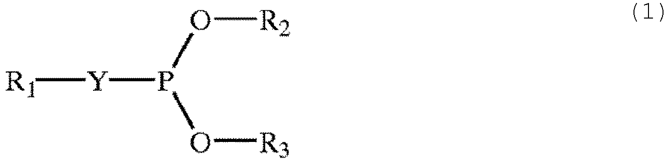 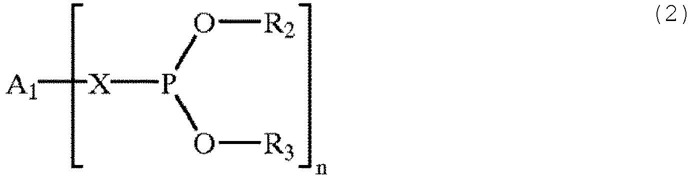 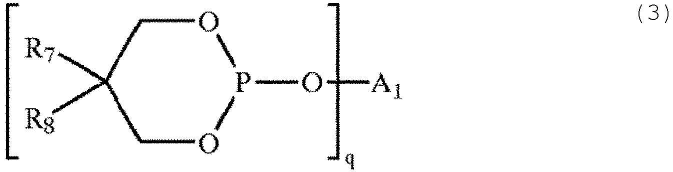 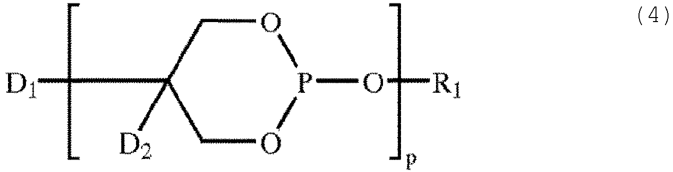 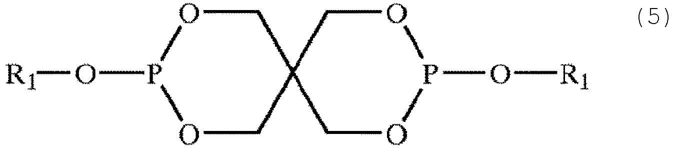 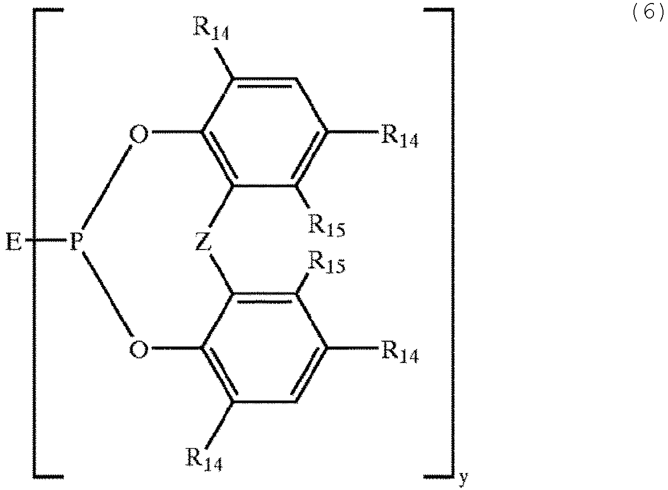 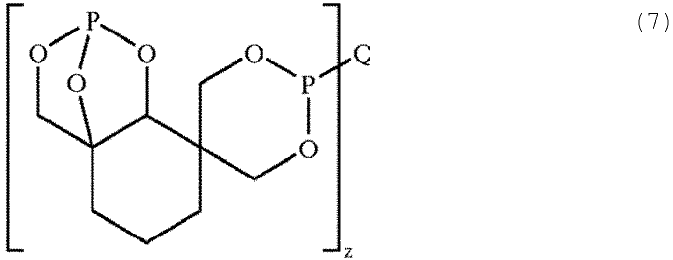 dans lesquelles les indices sont des entiers et
n est 2, 3 ou 4 ; p est 1 ou 2 ; q est 2 ou 3 ; r est 4 à 12 ; y est 1, 2 ou 3 ; et z est 1 à 6 ;
A₁, si n est 2, est alkylène en C₂-C₁₈ ; alkylène en C₂-C₁₂ interrompu par un atome d'oxygène, un atome de soufre ou -NR₄- ; un radical de formule 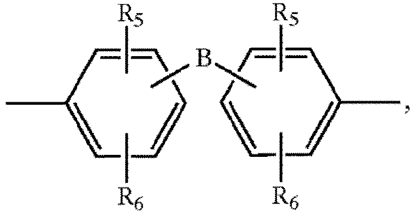 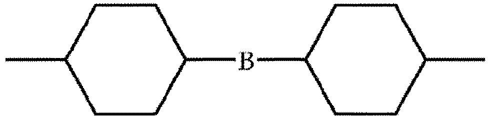
ou phénylène ;
A₁, si n est 3, est un radical de formule -CᵣH₂ᵣ₋₁- ;
A₁, si n est 4, est 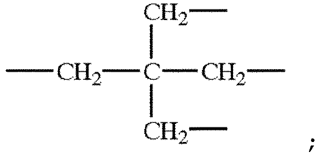
A₂ est tel que défini pour A₁ si n est 2 ;
B est une liaison directe, -CH₂-, -CHR₄-, -CR₁R₄-, un atome de soufre, cycloalkylidène en C₅-C₇, ou cyclohexylidène qui est substitué par de 1 à 4 radicaux alkyle en C₁-C₄ en position 3, 4 et/ou 5 ;
D₁, si p est 1, est alkyle en C₁-C₄ et, si p est 2, est - CH₂OCH₂- ;
D₂, si p est 1, est alkyle en C₁-C₄ ;
E, si y est 1, est alkyle en C₁-C₁₈, -OR₁ ou halogène ;
E, si y est 2, est -O-A₂-O-,
E, si y est 3, est un radical de formule R₄C(CH₂O-)₃ ou N(CH₂CH₂O-)₃ ;
Q est le radical d'un alcool au moins z-valent ou phénol, ce radical étant lié par l'intermédiaire de l'atome d'oxygène à l'atome de phosphore ;
R₁, R₂ et R₃, indépendamment les uns des autres, sont alkyle en C₁-C₁₈ qui est non substitué ou substitué par halogène, -COOR₄, -CN ou -CONR₄R₄ ; alkyle en C₂-C₁₈ interrompu par un atome d'oxygène, un atome de soufre ou -NR₄- ; phénylalkyle en C₇-C₉ ; cycloalkyle en C₅-C₁₂, phényle ou naphtyle ; naphtyle ou phényle substitué par halogène, 1 à 3 radicaux alkyle ou radicaux alcoxy ayant un total de 1 à 18 atomes de carbone ou par phénylalkyle en C₇-C₉ ; ou un radical de formule 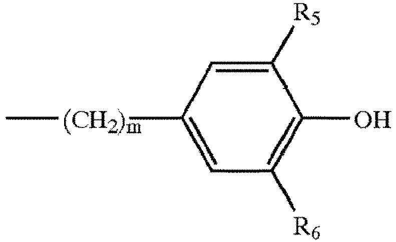
dans laquelle m est un entier dans la plage de 3 à 6 ;
R₄ est hydrogène, alkyle en C₁-C₈, cycloalkyle en C₅-C₁₂ ou phénylalkyle en C₇-C₉,
R₅ et R₆, indépendamment l'un de l'autre, sont hydrogène, alkyle en C₁-C₈ ou cycloalkyle en C₅-C₆,
R₇ et R₈, si q est 2, indépendamment l'un de l'autre, sont alkyle en C₁-C₄ ou sont conjointement un radical 2,3-déhydropentaméthylène ; et
R₇ et R₈, si q est 3, sont méthyle ;
R₁₄ est hydrogène, alkyle en C₁-C₉ ou cyclohexyle,
R₁₅ est hydrogène ou méthyle et, si deux ou plus de deux radicaux R₁₄ et R₁₅ sont présents, ces radicaux sont identiques ou différents,
X et Y sont chacun une liaison directe ou un atome d'oxygène,
Z est une liaison directe, méthylène, -C(R₁₆)₂- ou un atome de soufre, et
R₁₆ est alkyle en C₁-C₈ ;
un trisarylphosphite selon la formule 8 : 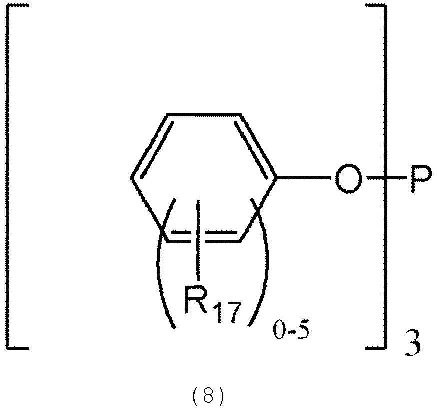
dans laquelle R₁₇ est un substituant qui est identique ou différent aux positions 0 à 5 de la partie aromatique de formule 8 et est indépendamment choisi parmi un membre choisi dans le groupe constitué d'alkyle en C₁-C₂₀, cycloalkyle en C₃-C₂₀, alkylcycloalkyle en C₄-C₂₀, aryle en C₆-C₁₀, et alkylaryle en C₇-C₂₀ ; et des combinaisons de ceux-ci ;
par exemple dans laquelle le phosphite ou la phosphonite organique sont choisis dans le groupe constitué des suivants : triphénylphosphite ; diphénylalkylphosphites ; phényldialkylphosphites ; trilaurylphosphite ; trioctadécylphosphite ; distéarylpentaérythritol-phosphite ; tris(2,4-di-tert-butylphényl)phosphite ; tris(nonylphényl)phosphite ; un composé des formules (A), (B), (C), (D), (E), (F), (G), (H), (J), (K) et (L) : 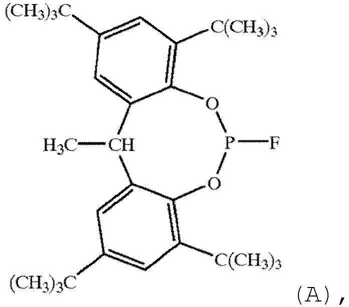 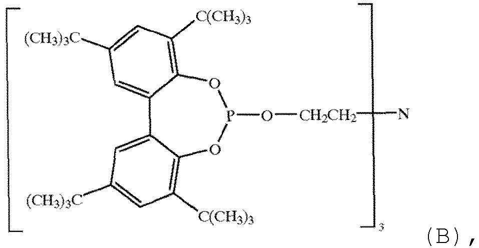 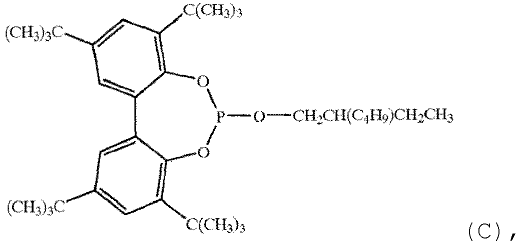 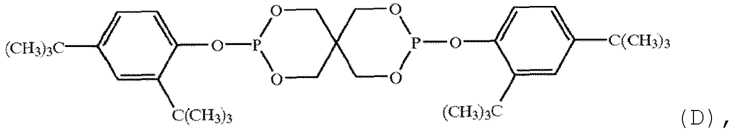 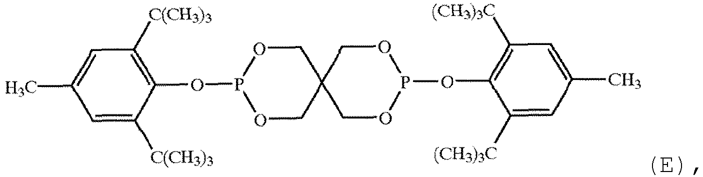 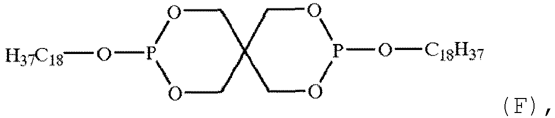 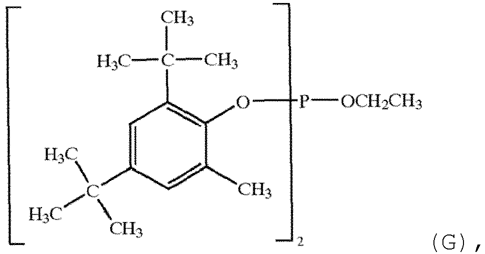 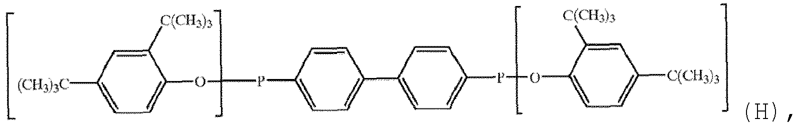 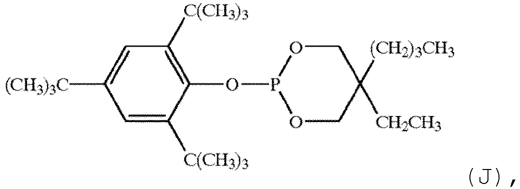 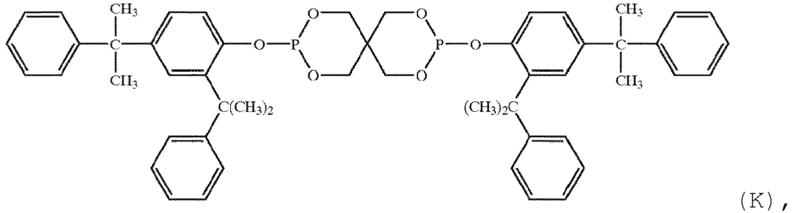 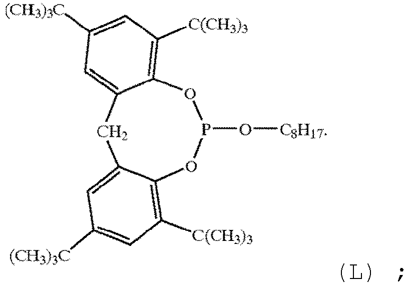 et 2-butyl-2-éthyl-1,3-propanediol 2,4,6-tri-t-butylphénol-phosphite, bis-(2,6-di-t-butyl-4-méthlphényl)pentaérythritol-diphosphite, 2-butyl-2-éthyl-1,3-propanediol-2,4-di-cumylphénol-phosphite, 2-butyl-2-éthyl-1,3-propanediol-4-méthyl-2,6-di-t-butylphénol-phosphite et bis-(2,4,6-tri-t-butyl-phényl)pentaérythritol-diphosphite.

7. Utilisation selon l'une quelconque des revendications précédentes, dans laquelle la composition stabilisante comprend en outre au moins un composé de phénol encombré comportant un fragment moléculaire selon une ou plusieurs des formules (IVa), (IVb) ou (IVc) : 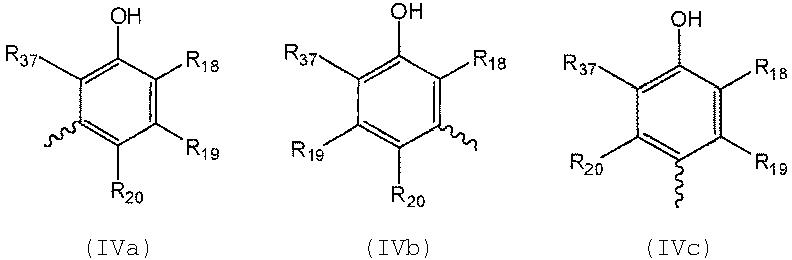
dans lesquelles
R₁₈ est choisi parmi hydrogène ou un hydrocarbyle en C₁₋₄ ; chacun de R₁₉ et R₂₀ est indépendamment choisi parmi hydrogène ou un hydrocarbyle en C₁-C₂₀ ; et
R₃₇ est choisi parmi un hydrocarbyle en C₁-C₁₂.

8. Utilisation selon la revendication 7, dans laquelle l'au moins un composé de phénol est choisi dans le groupe constitué de (1,3,5-tris(4-t-butyl-3-hydroxy-2,6-diméthylbenzyl)-1,3,5-triazine-2,4,6-(1H,3H, 5H)-trione ; 1,1,3-tris(2'-méthyl-4'-hydroxy-5'-*t-*butylphényl(butane ; bis[3-(3-t-butyl-4-hydroxy-5-méthylphényl)propionate] de triéthylène glycol ; 4,4'-thiobis(2-t-butyl-5-méthylphénol) ; bis[3-(3-t-butyl-4-hydroxyl-5-méthylphényl)propionate] de 2,2'-thiodiéthylène ; 3-(3'-t-butyl-4'-hydroxy-5'-méthylphényl)propionate d'octadécyle ; tétrakisméthylène(3-t-butyl-4-hydroxy-5-méthylhydrocinnamate)méthane ; *N,N*'-hexaméthylène bis[3-(3-t-butyl-4-hydroxy-5-méthylphényl)propionamide] ; thiodipropionate de di(4-tert-butyl-3-hydroxy-2,6-diméthylbenzyle) ; et 3,5-di-(tert)-butyl-4-hydroxyhydrocinnamate d'octadécyle.

9. Utilisation selon l'une quelconque des revendications précédentes,
dans laquelle la composition stabilisante comprend en outre au moins un composé choisi dans le groupe constitué des suivants : α-tocophérol, β-tocophérol, γ-tocophérol, δ-tocophérol, tocotriénols apparentés, et mélanges de ceux-ci ; et/ou
dans laquelle l'acétate de vitamine E est présent de 0,001 % à 5,0 % en poids du poids total de la composition stabilisante ; ou de 0,01 % à 1,0 % en poids du poids total de la composition stabilisante.

10. Utilisation selon l'une quelconque des revendications précédentes, dans laquelle la composition stabilisante comprend en outre une quantité efficace d'un photostabilisant choisi dans le groupe constitué de photostabilisants à amine encombrée, benzoates d'hydroxyle encombrés, phénolates de nickel, photostabilisants aux ultraviolets, et des combinaisons de ceux-ci.

11. Utilisation selon la revendication 10, dans laquelle le photostabilisant est un composé photostabilisant à amine encombrée comprenant un fragment moléculaire selon la formule (VI) : 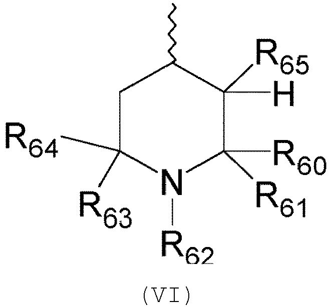 dans laquelle
R₆₂ est choisi parmi un membre choisi dans le groupe constitué d'hydrogène ; OH ; hydrocarbyle en C₁-C₂₀ ; - CH₂CN ; acyle en C₁-C₁₂ ; et alcoxy en C₁-C₁₈ ;
R₆₅ est choisi parmi un membre choisi dans le groupe constitué d'hydrogène ; et hydrocarbyle en C₁-C₈ ; et chacun de R₆₀, R₆₁, R₆₃ et R₆₄ est indépendamment choisi parmi un hydrocarbyle en C₁-C₂₀, ou R₆₀ et R₆₁ et/ou R₆₃ et R₆₄, conjointement avec l'atome de carbone auquel ils sont liés forment un cycloalkyle en C₅-C₁₀ ;
ou la formule (VIa) 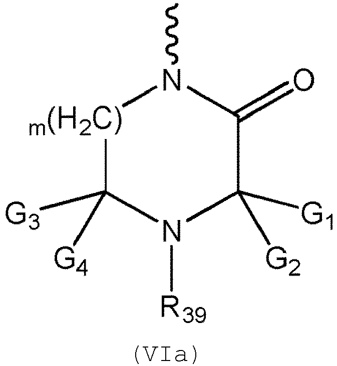 dans laquelle
m est un entier de 1 à 2 ;
R₃₉ est choisi parmi un membre choisi dans le groupe constitué d'hydrogène ; OH ; hydrocarbyle en C₁-C₂₀ ; - CH₂CN ; acyle en C₁-C₁₂ ; et alcoxy en C₁-C₁₈ ; et
chacun de G₁ à G₄ est indépendamment choisi parmi un hydrocarbyle en C₁-C₂₀ ; et/ou
dans laquelle le photostabilisant à amine encombrée est choisi dans le groupe constitué des suivants : sébacate de bis(2,2,6,6-tétraméthylpipéridin-4-yle) ; succinate de bis(2,2,6,6-tétraméthylpipéridin-4-yle) ; sébacate de bis(1,2,2,6,6-pentaméthylpipéridin-4-yle) ; sébacate de bis(1-octyloxy-2,2,6,6-tétraméthylpipéridin-4-yle) ; 3,5-di-tert-butyl-4-hydroxybenzylmalonate de bis(1,2,2,6,6-pentaméthylpipéridin-4-yl)n-butyle ; un condensat de 1-(2-hydroxyéthyl)-2,2,6,6-tétraméthyl-4-hydroxypipéridine et d'acide succinique ; stéarate de 2,2,6,6-tétraméthylpipéridin-4-yle ; dodécanate de 2,2,6,6-tétraméthylpipéridin-4-yle ; stéarate de 1,2,2,6,6-pentaméthylpipéridin-4-yle ; dodécanate de 1,2,2,6,6-pentaméthylpipéridin-4-yle ; un condensat de N,N'-bis(2,2,6,6-tétraméthylpipéridin-4-yl)hexaméthylènediamine et de 4-tert-octylamino-2,6-dichloro-1,3,5-triazine ; nitrilotriacétate de tris(2,2,6,6-tétraméthylpipéridin-4-yle) ; 1,2,3,4-butanetétracarboxylate de tétrakis(2,2,6,6-tétraméthylpipéridin-4-yle) ; 4-benzoyl-2,2,6,6-tétraméthylpipéridine ; 4-stéaryloxy-2,2,6,6-tétraméthylpipéridine ; bis(1,2,2,6,6-pentaméthylpipéridyl)-2-n-butyl-2-(2-hydroxy-3,5-di-tert-butylbenzyl)malonate ; 3-n-octyl-7,7,9,9-tétraméthyl-1,3,8-triazaspiro[4.5]décan-2,4-dione ; sébacate de bis(1-octyloxy-2,2,6,6-tétraméthylpipéridyle) ; succinate de bis(1-octyloxy-2,2,6,6-tétraméthylpipéridyle) ; un condensat de N,N'-bis(2,2,6,6-tétraméthylpipéridin-4-yl)hexaméthylènediamine et de 4-morpholino-2,6-dichloro-1,3,5-triazine ; un condensat de 2-chloro-4,6-bis(4-n-butylamino-2,2,6,6-tétraméthylpipéridyl)-1,3,5-triazine et de 1,2-bis(3-aminopropylamino)éthane ; un condensat de 2-chloro-4,6-bis(4-n-butylamino-1,2,2,6,6-pentaméthylpipéridyl)-1,3,5-triazine et de 1,2-bis-(3-aminopropylamino)éthane; 8-acétyl-3-dodécyl-7,7,9,9-tétraméthyl-1,3,8-triazaspiro[4.5]décane-2,4-dione ; 3-dodécyl-1-(2,2,6,6-tétraméthylpipéridin-4-yl)pyrrolidin-2,5-dione ; 3-dodécyl-1-(1-éthanoyl-2,2,6,6-tétraméthylpipéridin-4-yl)pyrrolidin-2,5-dione ; 3-dodécyl-1-(1,2,2,6,6-pentaméthylpipéridin-4-yl)pyrrolidine-2,5-dione ; un mélange de 4-hexadécyloxy-et 4-stéaryloxy-2,2,6,6-tétraméthylpipéridine ; un condensat de N,N'-bis(2,2,6,6-tétraméthylpipéridin-4-yl)hexaméthylènediamine et de 4-cyclohexylamino-2,6-dichloro-1,3,5-triazine ; un condensat de 1,2-bis(3-aminopropylamino)éthane, 2,4,6-trichloro-1,3,5-triazine et de 4-butylamino-2,2,6,6-tétraméthylpipéridine ; 2-undécyl-7,7,9,9-tétraméthyl-1-oxa-3,8-diaza-4-oxospiro[4.5]décane ; oxo-pipéranezinyl-triazines ; un produit de réaction de 7,7,9,9-tétraméthyl-2-cycloundécyl-1-oxa-3,8-diaza-4-oxospiro[4.5]décane et d'épichlorohydrine ; tétrakis(2,2,6,6-tétraméthyl-4-pipéridyle) butane-1,2,3,4-tétracarboxylate ; acide 1,2,3,4-butanetétracarboxylique, ester de tétrakis(1,2,2,6,6-pentaméthyl-4-pipéridinyle) ; acide 1,2,3,4-butanetétracarboxylique, ester de 1,2,2,6,6-pentaméthyl-4-pipéridinyle tridécyle ; acide 1,2,3,4-butanetétracarboxylique, ester de 2,2,6,6-tétraméthyl-4-pipéridinyle tridécyle ; acide 1,2,3,4-butanetétracarboxylique, polymère avec l'ester de 1,2,2,6,6-pentaméthyl-4-pipéridinyle de 2,2,6,6-tétraméthyl-2,4,8,10-tétraoxaspiro[5.5]-undécane-3,9-diéthanol ; acide 1,2,3,4-butanetétracarboxylique, polymère avec l'ester de 2,2,6,6-tétraméthyl-4-pipéridinyle de 2,2,6,6-tétraméthyl-2,4,8,10-tétraoxaspiro[5.5]-undécane-3,9-diéthanol ; carbonate de bis(1-undécanoxy-2,2,6,6-tétraméthylpipéridin-4-yle) ; 1-(2-hydroxy-2-méthylpropoxy)-2,2,6,6-tétraméthyl-4-pipéridinol ; 1-(2-hydroxy-2-méthylpropoxy)-4-octadécanoyloxy-2,2,6,6-tétraméthylpipéridine ; 1-(4-octadécanoyloxy-2,2,6,6-tétraméthylpipéridin-1-yloxy)-2-octadécanoyloxy-2-méthylpropane ; 1-(2-hydroxyéthyl)-2,2,6,6-tétraméthyl-4-pipéridinol ; un produit de réaction de 1-(2-hydroxyéthyl)-2,2,6,6-tétraméthyl-4-pipéridinol et de diméthylsuccinate ; 2,2,4,4-tétraméthyl-7-oxa-3,20-diazadispiro[5,1.11,2]hénéicosan-21-one; l'ester de 2,2,6,6-tétraméthyl-4-pipéridinol avec des acides gras supérieurs ; 3-dodécyl-1-(2,2,6,6-tétraméthyl-4-pipéridyl)pyrrolidine-2,5-dione ; 1H-pyrrole-2,5-dione, 1-octadécyl-, polymère avec le (1-méthyléthényl)benzène et la 1-(2,2,6,6-tétraméthyl-4-pipéridinyl)-1H-pyrrole-2,5-dione ; pipérazinone, 1,1',1"-[1,3,5-triazine-2,4,6-triyltris[(cyclohexylimino)-2,1-éthanediyl]]tris[3,3,5,5-tétraméthyl-pipérazinone, 1,1',1"-[1,3,5-triazine-2,4,6-triyltris[(cyclohexylimino)-2,1-éthanediyl]]tris[3,3,4,5,5-pentaméthyl- ; le produit de réaction de 7,7,9,9-tétraméthyl-2-cycloundécyl-1-oxa-3,8-diaza-4-oxospiro[4.5]décane et d'épichlorohydrine ; le condensat de N,N'-bis(2,2,6,6-tétraméthylpipéridin-4-yl)hexaméthylènediamine et de 4-cyclohexylamino-2,6-dichloro-1,3,5-triazine; le condensat de 1,2-bis(3-aminopropylamino)éthane, 2,4,6-trichloro-1,3,5-triazine et de 4-butylamino-2,2,6,6-tétraméthylpipéridine ; le condensat de N,N'-bis(2,2,6,6-tétraméthylpipéridin-4-yl)hexaméthylènediamine et de 4-morpholino-2,6-dichloro-1,3,5-triazine ; le condensat de 2-chloro-4,6-bis(4-n-butylamino-2,2,6,6-tétraméthylpipéridyl)-1,3,5-triazine et de 1,2-bis(3-aminopropylamino)éthane ; le condensat de 2-chloro-4,6-bis(4-n-butylamino-1,2,2,6,6-pentaméthylpipéridyl)-1,3,5-triazine et de 1,2-bis-(3-aminopropylamino)éthane ; 2-[(2-hydroxyéthyl)amino]-4,6-bis[N-(1-cyclohexyloxy-2,2,6,6-tétraméthylpipéridin-4-yl)butylamino-1,3,5-triazine ; acide propanedioïque, ester de [(4-méthoxyphényl)-méthylène]-bis-(1,2,2,6,6-pentaméthyl-4-pipéridinyle) ; tétrakis(2,2,6,6-tétraméthylpipéridin-4-yl)-1,2,3,4-butanetétracarboxylate ; acide benzènepropanoïque, 3,5-bis(1,1-diméthyléthyl)-4-hydroxy-, ester de 1-[2-[3-[3,5-bis(1,1-diméthyléthyl)-4-hydroxyphényl]-1-oxopropoxy]éthyl]-2,2,6,6-tétraméthyl-4-pipéridinyle ; N-(1-octyloxy-2,2,6,6-tétraméthylpipéridin-4-yl)-N'-dodécyloxalamide ; nitrilotriacétate de tris(2,2,6,6-tétraméthylpipéridin-4-yle) ; acide 1,5-dioxaspiro{5,5}undécane-3,3-dicarboxylique, bis(1,2,2,6,6-pentaméthyl-4-pipéridinyle) ; acide 1,5-dioxaspiro{5,5}undécane-3,3-dicarboxylique, bis(2,2,6,6-tétraméthyl-4-pipéridinyle); le condensat de 1-(2-hydroxyéthyl)-2,2,6,6-tétraméthyl-4-hydroxypipéridine et d'acide succinique ; le condensat de N,N'-bis(2,2,6,6-tétraméthylpipéridin-4-yl)hexaméthylènediamine et de 4-tert-octylamino-2,6-dichloro-1,3,5-triazine ; acide 1,2,3,4-butanetétracarboxylique, ester tridécylique de 1,2,2,6,6-pentaméthyl-4-pipéridinyle ; tétrakis(2,2,6,6-tétraméthylpipéridin-4-yl)-1,2,3,4-butanetétracarboxylate ; acide 1,2,3,4-butanetétracarboxylique, ester tridécylique de 2,2,6,6-tétraméthyl-4-pipéridinyle ; tétrakis(1,2,2,6,6-pentaméthylpipéridin-4-yl)-1,2,3,4-butanetétracarboxylate ; mélange d'ester dodécylique d'acide 2,2,4,4-tétraméthyl-21-oxo-7-oxa-3,20-diazaspiro(5,1.11,2)-hénéicosane-20-propanoïque et d'ester tétradécylique d'acide 2,2,4,4-tétraméthyl-21-oxo-7-oxa-3,20-diazaspiro(5,1.11,2)-hénéicosane-20-propanoïque ; 1H,4H,5H,8H-2,3a,4a,6,7a,8a-hexaazacyclopenta[def]fluorène-4,8-dione, hexahydro-2,6-bis(2,2,6,6-tétraméthyl-4-pipéridinyl)- ; polyméthyl[propyl-3-oxy(2',2',6',6'-tétraméthyl-4,4'-pipéridinyl)]siloxane ; polyméthyl[propyl-3-oxy(1',2',2',6',6'-pentaméthyl-4,4'-pipéridinyl)]siloxane; copolymère de méthacrylate de méthyle avec l'acrylate d'éthyle et l'acrylate de 2,2,6,6-tétraméthylpipéridin-4-yle ; copolymère d'alpha-oléfines en C20 à C24 mixtes et de (2,2,6,6-tétraméthylpipéridin-4-yl)succinimide ; acide 1,2,3,4-butanetétracarboxylique, polymère avec le β,β,β',β'-tétraméthyl-2,4,8,10-tétraoxaspiro[5.5]undécane-3,9-diéthanol, ester de 1,2,2,6,6-pentaméthyl-4-pipéridinyle ; acide 1,2,3,4-butanetétracarboxylique, polymère avec le β,β,β',β'-tétraméthyl-2,4,8,10-tétraoxaspiro[5.5]undécane-3,9-diéthanol, copolymère d'ester de 2,2,6,6-tétraméthyl-4-pipéridinyle ; 1,3-benzènedicarboxamide, N,N'-bis(2,2,6,6-tétraméthyl-4-pipéridinyle ; 1,1'-(1,10-dioxo-1,10-décanediyl)-bis(hexahydro-2,2,4,4,6-pentaméthylpyrimidine ; éthanediamide, N-(1-acétyl-2,2,6,6-tétraméthylpipéridinyl)-N'-dodécyle ; formamide, N,N'-1,6-hexanediylbis[N-(2,2,6,6-tétraméthyl-4-pipéridinyle) ; D-glucitol, 1,3:2,4-bis-O-(2,2,6,6-tétraméthyl-4-pipéridinylidène)- ; 2,2,4,4-tétraméthyl-7-oxa-3,20-diaza-21-oxo-dispiro[5,1.11,2]hénéicosane ; propanamide, 2-méthyl-N-(2,2,6,6-tétraméthyl-4-pipéridinyl)-2-[(2,2,6,6-tétraméthyl-4-pipéridinyl)amino]- ; 7-oxa-3,20-diazadispiro[5,1.11,2]hénéicosane-20-propanoïque acide, 2,2,4,4-tétraméthyl-21-oxo-, dodécyle ester ; N-(2,2,6,6-tétraméthylpipéridin-4-yl)-β-aminopropionique acide dodécyle ester ; N-(2,2,6,6-tétraméthylpipéridin-4-yl)-N'-aminooxalamide ; propanamide, N-(2,2,6,6-tétraméthyl-4-pipéridinyl)-3-[(2,2,6,6-tétraméthyl-4-pipéridinyl)amino]- ; mélange de 4-hexadécyloxy- et 4-stéaryloxy-2,2,6,6-tétraméthylpipéridine ; 3-dodécyl-1-(1,2,2,6,6-pentaméthylpipéridin-4-yl)pyrrolidine-2,5-dione ; 3-dodécyl-1-(1-éthanoyl-2,2,6,6-pentaméthylpipéridin-4-yl)pyrrolidine-2,5-dione ; succinate de bis(2,2,6,6-tétraméthylpipéridin-4-yle) ; malonate de bis(1,2,2,6,6-pentaméthylpipéridin-4-yl)-n-butyl-3,5-di-tert-butyl-4-hydroxybenzyle ; nitrilotriacétate de tris(2,2,6,6-tétraméthylpipéridin-4-yle) ; 1,1'-(1,2-éthanediyl)bis(3,3,5,5-tétraméthylpipérazinone) ; 4-benzoyl-2,2,6,6-tétraméthylpipéridine ; 4-stéaryloxy-2,2,6,6-tétraméthylpipéridine ; malonate de bis(1,2,2,6,6-pentaméthylpipéridyl)-2-n-butyl-2-(2-hydroxy-3,5-di-tert-butylbenzyle) ; 3-n-octyl-7,7,9,9-tétraméthyl-1,3,8-triazaspiro[4.5]décan-2,4-dione ; sébacate de bis(1-octyloxy-2,2,6,6-tétraméthylpipéridyle) ; succinate de bis(1-octyloxy-2,2,6,6-tétraméthylpipéridyle) ; 8-acétyl-3-dodécyl-7,7,9,9-tétraméthyl-1,3,8-triazaspiro[4.5]décane-2,4-dione ; 3-dodécyl-1-(2,2,6,6-tétraméthylpipéridin-4-yl)pyrrolidin-2,5-dione ; 3-dodécyl-1-(1-éthanoyl-2,2,6,6-tétraméthylpipéridin-4-yl)pyrrolidin-2,5-dione ; 3-dodécyl-1-(1,2,2,6,6-pentaméthylpipéridin-4-yl)pyrrolidine-2,5-dione ; un mélange de 4-hexadécyloxy- et de 4-stéaryloxy-2,2,6,6-tétraméthylpipéridine ; 2-undécyl-7,7,9,9-tétraméthyl-1-oxa-3,8-diaza-4-oxospiro[4.5]décane ; acide 1,5-dioxaspiro{5,5}undécane-3,3-dicarboxylique, bis(2,2,6,6-tétraméthyl-4-pipéridinyle) et acide 1,5-dioxaspiro{5,5}undécane-3,3-dicarboxylique, bis(1,2,2,6,6-pentaméthyl-4-pipéridinyle) ; N¹-(β-hydroxyéthyl)-3,3-pentaméthylène-5,5-diméthylpipérazin-2-one ; N¹-tert-octyl-3,3,5,5-tétraméthyl-diazépin-2-one; N¹-tert-octyl-3,3-pentaméthylène-5,5-hexaméthylène-diazépin-2-one ; N¹-tert-octyl-3,3-pentaméthylène-5,5-diméthylpipérazin-2-one; trans-1,2-cyclohexane-bis-(N¹-5,5-diméthyl-3,3-pentaméthylène-2-pipérazinone ; trans-1,2-cyclohexane-bis-(N¹-3,3,5,5-dispiropentaméthylène-2-pipérazinone) ; N¹-isopropyl-1,4-diazadispiro-(3,3,5,5)pentaméthylène-2-pipérazinone ; N¹-isopropyl-1,4-diazadispiro-3,3-pentaméthylène-5,5-tétraméthylène-2-pipérazinone ; N¹-isopropyl-5,5-diméthyl-3,3-pentaméthylène-2-pipérazinone ; trans-1,2-cyclohexane-bis-N¹-(diméthyl-3,3-pentaméthylène-2-pipérazinone) ; N¹-octyl-5,5-diméthyl-3,3-pentaméthylène-1,4-diazépin-2-one ; et N¹-octyl-1,4-diazadispiro-(3,3,5,5)pentaméthylène-1,5-diazépin-2-one.

12. Utilisation selon la revendication 10 ou 11, dans laquelle le photostabilisant comprend un absorbeur de lumière ultraviolette choisi dans le groupe constitué d'un composé de 2-hydroxybenzophénone, un composé de 2-(2'-hydroxyphényl)benzotriazole, un composé de 2-(2'-hydroxyphényl)-1,3,5-triazine, et des combinaisons de ceux-ci ;
par exemple dans laquelle l'absorbeur de lumière ultraviolette est un composé de 2-(2'-hydroxyphényl)-1,3,5-triazine choisi dans le groupe constitué des suivants : 4,6-bis-(2,4-diméthylphényl)-2-(2-hydroxy-4-octyloxyphényl)-s-triazine ; 4,6-bis-(2,4-diméthylphényl)-2-(2,4-dihydroxyphényl)-s-triazine ; 2,4-bis(2,4-dihydroxyphényl)-6-(4-chlorophényl)-s-triazine ; 2,4-bis[2-hydroxy-4-(2-hydroxy-éthoxy)phényl]-6-(4-chlorophényl)-s-triazine ; 2,4-bis[2-hydroxy-4-(2-hydroxy-4-(2-hydroxy-éthoxy)phényl]-6-(2,4-diméthylphényl)-s-triazine ; 2,4-bis[2-hydroxy-4-(2-hydroxyéthoxy)phényl]-6-(4-bromophényl)-s-triazine ; 2,4-bis[2-hydroxy-4-(2-acétoxyéthoxy)phényl]-6-(4-chlorophényl)-s-triazine ; 2,4-bis(2,4-dihydroxyphényl)-6-(2,4-diméthylphényl)-s-triazine ; 2,4-bis(4-biphénylyl)-6-[2-hydroxy-4-[(octyloxycarbonyl)éthylidèneoxy]phényl]-s-triazine ; 2,4-bis(4-biphénylyl)-6-[2-hydroxy-4-(2-éthylhexyloxy)phényl]-s-triazine ; 2-phényl-4-[2-hydroxy-4-(3-sec-butyloxy-2-hydroxypropyloxy)phényl]-6-[2-hydroxy-4-(3-sec-amyloxy-2-hydroxypropyloxy)phényl]-s-triazine ; 2,4-bis(2,4-diméthylphényl)-6-[2-hydroxy-4(-3-benzyloxy-2-hydroxypropyloxy)phényl]-s-triazine ; 2,4-bis(2-hydroxy-4-n-butyloxyphényl)-6-(2,4-di-n-butyloxyphényl)-s-triazine ; 2,4-bis(2,4-diméthylphényl)-6-[2-hydroxy-4-(3-nonyloxy-2-hydroxypropylox-y)-5-α-cumylphényl]-s-triazine; méthylènebis-{2,4-bis(2,4-diméthylphényl)-6-[2-hydroxy-4-(3-butyloxy-2-hydroxypropoxy)phényl]-s-triazine} ; mélange de dimères pontés par méthylène pontés aux positions 3:5', 5:5' et 3:3' dans un rapport 5:4:1 ; 2,4,6-tris(2-hydroxy-4-isooctyloxycarbonyliso-propylidèneoxy-phényl)-s-triazine ; 2,4-bis(2,4-diméthylphényl)-6-(2-hydroxy-4-hexyloxy-5-α-cumylphényl)-s-triazine ; 2-(2,4,6-triméthylphényl)-4,6-bis[2-hydroxy-4-(3-butyloxy-2-hydroxypropyloxy)phényl]-s-triazine ; 2,4,6-tris[2-hydroxy-4-(3-sec-butyloxy-2-hydroxypropyloxy)-phényl]-s-triazine ; mélange de 4,6-bis-(2,4-diméthylphényl)-2-(2-hydroxy-4-(3-dodécyloxy-2-hydroxypropoxy)phényl)-s-triazine et 4,6-bis-(2,4-diméthylphényl)-2-(2-hydroxy-4-(3-tridécyloxy-2-hydroxypropoxy)phényl)-s-triazine ; 4,6-bis-(2,4-diméthylphényl)-2-(2-hydroxy-4(3-(2-éthylhexyloxy)-2-hydroxypropoxy)-phényl)-s-triazine ; 4,6-diphényl-2-(4-hexyloxy-2-hydroxyphényl)-s-triazine ; et des combinaisons de ceux-ci.

13. Utilisation selon l'une quelconque des revendications précédentes, dans laquelle la composition stabilisante comprend en outre au moins un composé choisi dans le groupe constitué de :
un composé d'hydroxylamine selon la formule (VIII) : 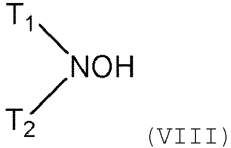 dans laquelle
T₁ est choisi parmi un membre choisi dans le groupe constitué d'un hydrocarbyle en C₁-C₃₆ facultativement substitué, un cycloalkyle en C₅-C₁₂ facultativement substitué et un aralkyle en C₇-C₉ facultativement substitué ; et
T₂ est choisi parmi hydrogène ou T₁ ; et
un composé d'oxyde d'aminé tertiaire selon la formule (IX) : 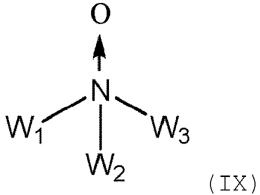 dans laquelle
chacun de W₁ et W₂ est indépendamment choisi parmi un hydrocarbyle en C₆-C₃₆ choisi parmi un membre choisi dans le groupe constitué d'un alkyle en C₆-C₃₆ à chaîne linéaire ou ramifiée, un aryle en C₆-C₁₂, un aralkyle en C₇-C₃₆, un alkaryle en C₇-C₃₆, un cycloalkyle en C₅-C₃₆, un alkylcloalkyle en C₆-C₃₆ ; et un cycloalkylalkyle en C₆-C₃₆ ;
W₃ est choisi parmi un hydrocarbyle en C₁-C₃₆ choisi parmi un membre choisi dans le groupe constitué d'un alkyle en C₆-C₃₆ à chaîne linéaire ou ramifiée, un aryle en C₆-C₁₂,
un aralkyle en C₇-C₃₆, un alkaryle en C₇-C₃₆, un cycloalkyle en C₅-C₃₆, un alkylcloalkyle en C₆-C₃₆ ; et un cycloalkylalkyle en C₆-C₃₆ ;
à condition qu'au moins l'un de W₁, W₂ et W₃ contient une liaison β carbone-hydrogène ; et
dans laquelle lesdits groupes alkyle, aralkyle, alkaryle, cycloalkyle, alkcycloalkyle et cycloalkylalkyle peuvent être interrompus par un à seize groupes choisis parmi un membre choisi dans le groupe constitué de -O-, -S-, -SO-, -SO₂-, -COO-, -OCO-, -CO-, -NW₄-, -CONW₄- et -NW₄CO-, ou dans laquelle lesdits groupes alkyle, aralkyle, alkaryle, cycloalkyle, alkcycloalkyle et cycloalkylallyle peuvent être substitués par un à seize groupes choisis parmi un membre choisi dans le groupe constitué de -OW₄, -SW₄, - COOW₄, -OCOW₄, -COW₄, -N(W₄)₂, -CON(W₄)₂, -NW₄COW₄ et des cycles de 5 et 6 chaînons contenant le groupe - C (CH₃) (CH₂Rₓ)NL(CH₂Rₓ)(CH₃) C-, ou dans laquelle lesdits groupes alkyle, aralkyle, alkaryle, cycloalkyle, alkcycloalkyle et cycloalkylalkyle sont à la fois interrompus et substitués par les groupes mentionnés cidessus ; et
dans laquelle
W₄ est choisi parmi hydrogène ou un alkyle en C₁-C₈ ;
Rₓ est choisi parmi hydrogène ou méthyle ; et
L est choisi parmi un alkyle en C₁-C₃₀ ; un fragment - C(O)R, ou un fragment -OR, où R est un groupe alkyle en C₁-C₃₀ à chaîne linéaire ou ramifiée ; et
dans laquelle lesdits groupes aryle peuvent être substitués par un membre choisi dans le groupe constitué d'un à trois groupes halogène, un groupe alkyle en C₁-C₈, un groupe alcoxy en C₁-C₈, et des combinaisons de ceux-ci ; par exemple
dans laquelle le composé selon la formule (VIII) est une N,N-dihydrocarbylhydroxylamine choisie parmi un membre choisi dans le groupe constitué des suivantes : N,N-dibenzylhydroxylamine ; N,N-diéthylhydroxylamine ; N,N-dioctylhydroxylamine ; N,N-dilaurylhydroxylamine ; N,N-didodécylhydroxylamine ; N,N-ditétradécylhydroxylamine ; N,N-dihexadécylhydroxylamine ; N,N-dioctadécylhydroxylamine ; N-hexadécyl-N-tétradécylhydroxylamine ; N-hexadécyl-N-heptadécylhydroxylamine ; N-hexadécyl-N-octadécylhydroxylamine ; N-heptadécyl-N-octadécylhydroxylamine ; et N,N-di(suif hydrogéné)hydroxylamine.

14. Utilisation selon l'une quelconque des revendications précédentes, dans laquelle la composition stabilisante est présente de 0,001 % à 65,0 % en poids du poids total du matériau organique.

15. Utilisation selon la revendication 14, dans laquelle la composition stabilisante est présente de 0,01 % à 25 % en poids du poids total du matériau organique.
